(19) Europäisches Patentamt / European Patent Office / Office européen des brevets

(11) EP 1 436 425 B1

(12) **EUROPEAN PATENT SPECIFICATION**

(45) Date of publication and mention of the grant of the patent: **02.12.2009 Bulletin 2009/49**

(21) Application number: **02777275.5**

(22) Date of filing: **02.10.2002**

(51) Int Cl.:
***C12Q 1/68*** *(2006.01)* ***C07K 14/47*** *(2006.01)*
***C12N 5/12*** *(2006.01)* ***C12N 15/11*** *(2006.01)*
***A61K 48/00*** *(2006.01)*

(86) International application number: **PCT/EP2002/011034**

(87) International publication number: **WO 2003/031650 (17.04.2003 Gazette 2003/16)**

# (54) GENES AND PROTEINS FOR PREVENTION, PREDICTION, PROGNOSIS AND THERAPY OF CARDIOVASCULAR DISEASE

GENE UND PROTEINE FÜR VERHINDERUNG, VORHERSAGE, PROGNOSE UND THERAPIE DER HERZGEFÄSS-KRANKHEIT

GENES ET PROTEINES UTILISES DANS LA PREVENTION, LA PREDICTION, LE PRONOSTIC ET LA THERAPIE DE LA MALADIE CARDIOVASCULAIRE

(84) Designated Contracting States:
**AT BE BG CH CY CZ DE DK EE ES FI FR GB GR IE IT LI LU MC NL PT SE SK TR**

(30) Priority: **08.10.2001 GB 0124145**

(43) Date of publication of application:
**14.07.2004 Bulletin 2004/29**

(73) Proprietor: **Siemens Healthcare Diagnostics GmbH**
**65760 Eschborn (DE)**

(72) Inventors:
- **MUNNES, Marc**
  **40699 Erkrath (DE)**
- **GEHRMANN, Mathias**
  **51373 Leverkusen (DE)**
- **WICK, Maresa**
  **10179 Berlin (DE)**
- **SCHMITZ, Gerd**
  **93161 Sinzing (DE)**

(74) Representative: **Maier, Daniel Oliver et al**
**Siemens AG**
**CT IP**
**Postfach 22 16 34**
**80506 München (DE)**

(56) References cited:
**WO-A-01/72774 US-A- 6 087 117**

- **BARRANS ET AL.: "Construction of a human cardiovascular cDNA microarray: portrait of the failing heart" BIOCHEMICAL AND BIOPHYSICAL RESEARCH COMMUNICATIONS, vol. 280, January 2001 (2001-01), pages 964-969, XP002248512**
- **MCCAFFREY ET AL.: "High-level of expression of Egr-1 and Egr-1-inducible genes in mouse and human atherosclerosis" THE JOURNAL OF CLINICAL INVESTIGATION, vol. 105, no. 5, March 2000 (2000-03), pages 653-662, XP002248513**
- **LAWN ET AL.: "The Tangier disease gene product ABC1 controls the cellular apolipoprotein-mediated lipid removal pathway" THE JOURNAL OF CLINICAL INVESTIGATION, vol. 104, no. 8, October 1999 (1999-10), pages R25R-R31, XP002248514**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

SEQUENCE LISTING

<110> Bayer AG

<120> Genes and proteins for prevention, prediction, prognosis and therapy of cardiovascular disease

<130> Lea 35643 WO

<150> GB0124145
<151> 2001-10-08

<160> 2

<170> PatentIn version 3.1

<210> 1

<211> 670
<212> DNA
<213> Homo sapiens

<400> 1

```
cggccacgag gcggaatccc ttctgctctc ccagcgcagc gccgccgccc ggcccctcca      60
gcttcccgga ccatggccaa cctggagcgc accttcatcg ccatcaagcc ggacggcgtg     120
cagcgcggcc tggtgggcga gatcatcaag cgcttcgagc agaagggatt ccgcctcgtg     180
gccatgaagt tcctccgggc ctctgaagaa cacctgaagc agcactacat tgacctgaaa     240
gaccgaccat tcttccctgg gctggtgaag tacatgaact cagggccggt tgtggccatg     300
gtctgggagg ggctgaacgt ggtgaagaca ggccgagtga tgcttgggga gaccaatcca     360
gcagattcaa agccaggcac cattcgtggg gacttctgca ttcaggttgg caggaacatc     420
attcatggca gtgattcagt aaaaagtgct gaaaaagaaa tcagcctatg gtttaagcct     480

gaagaactgg ttgactacaa gtcttgtgct catgactggg tctatgaata agaggtggac     540
acaacagcag tctccttcag cacggcgtgg tgtgtccctg gacacagctc ttcattccat     600
tgacttagag gcaacaggat tgatcattct tttatagagc atatttgcca ataaagcttt     660
tggaagccgg                                                            670
```

<210> 75
<211> 152
<212> PRT
<213> Homo sapiens

<400> 75

```
Met Ala Asn Leu Glu Arg Thr Phe Ile Ala Ile Lys Pro Asp Gly Val
1               5                   10                  15

Gln Arg Gly Leu Val Gly Glu Ile Ile Lys Arg Phe Glu Gln Lys Gly
            20                  25                  30

Phe Arg Leu Val Ala Met Lys Phe Leu Arg Ala Ser Glu Glu His Leu
        35                  40                  45

Lys Gln His Tyr Ile Asp Leu Lys Asp Arg Pro Phe Phe Pro Gly Leu
    50                  55                  60

Val Lys Tyr Met Asn Ser Gly Pro Val Val Ala Met Val Trp Glu Gly
65                  70                  75                  80

Leu Asn Val Val Lys Thr Gly Arg Val Met Leu Gly Glu Thr Asn Pro
                85                  90                  95

Ala Asp Ser Lys Pro Gly Thr Ile Arg Gly Asp Phe Cys Ile Gln Val
            100                 105                 110

Gly Arg Asn Ile Ile His Gly Ser Asp Ser Val Lys Ser Ala Glu Lys
        115                 120                 125

Glu Ile Ser Leu Trp Phe Lys Pro Glu Glu Leu Val Asp Tyr Lys Ser
    130                 135                 140

Cys Ala His Asp Trp Val Tyr Glu
145                 150
```

## Description

### TECHNICAL FIELD OF THE INVENTION

The present invention relates to a polynucleotide sequence for the diagnosis and treatment of cardiovascular disease, including, but not limited to, arteriosclerosis, angina pectoris, myocardial infarction, ischemia, restenosis, and arterial inflammation. Specifically, the present invention identifies and describes a gene which is differentially expressed in a cardiovascular disease state, relative to its expression in normal, and/or in response to manipulations relevant to cardiovascular disease (e.g. incubation of isolated macrophages in the presence of enzymatic modified LDL). In particular the gene is down-regulated in macrophages of patients with inherited predisposition for arteriosclerosis. Also disclosed are methods for utilizing this gene, polynucleotides derived from this gene as diagnostic markers for cardiovascular disease, particularly arteriosclerosis.

Moreover, the present invention provides methods for the diagnostic monitoring of patients undergoing clinical evaluation for the treatment of cardiovascular disease, and for monitoring the efficacy of compounds in clinical trials. Additionally, the present invention describes methods for the diagnostic evaluation and prognosis of various cardiovascular diseases, and for the identification of subjects exhibiting a predisposition to such conditions

### BACKGROUND OF THE INVENTION

Cardiovascular diseases such as arteriosclerosis, ischemia, myocardial infarction, and angina pectoris are a major health risk throughout the industrialized world.

#### *Arteriosclerosis*

The principal cell types of the artery wall, the endothelial cell, the smooth muscle cell and the monocyte/macrophage, are major players in the events involved in initiation and evolution of the arteriosclerotic plaque. The process, in normal circumstances a protective response to insults to the endothelium and smooth muscle cells (SMCs) of the wall of the artery, consists of the formation of fibrofatty and fibrous lesions (fatty streaks) or plaques, preceded and accompanied by inflammation.

The first observable event in the formation of an arteriosclerotic plaque occurs when blood-borne monocytes adhere to the vascular endothelial layer and transmigrate through to the sub-endothelial space. Within the vessel wall monocytes differentiate into macrophages due to the extracellular stimuli. Adjacent endothelial cells at the same time produce oxidized low density lipoprotein (LDL). These oxidized LDL's are then taken up in large amounts by the macrophages through scavenger receptors expressed on their surfaces. In contrast to the tightly regulated pathway by which native LDL (nLDL) is taken up by nLDL specific receptors. But not only genes of the LDL uptake machinery are of great diagnostic and therapeutic interest since the cellular cholesterol content is normally under strict homeostatic control, and mechanisms of *de novo* synthesis and efflux are also highly regulated. Cholesterol efflux pathways have been a focus of much recent attention, as studies on protein and cholesterol transport converged, pointing at cholesterol-rich membrane micro-domains or proteolipid complexes, or both, as carriers of newly synthesised free cholesterol to the plasma membrane. Cellular cholesterol is accrued by:

(i) intemalisation of intact low-density lipoprotein (LDL) carrying cholesterylester by endocytosis via high-affinity LDL receptors;

(ii) selective uptake of free cholesterol by monomer exchange, mainly from LDL;

(iii) selective uptake of cholesteryl ester by exchange, mainly from HDL; and

(iv) *de novo* synthesis of cholesterol by the mevalonate pathway in the endoplasmic reticulum (ER).

Several lines of evidence suggest that the pathways involved in transport of protein and cholesterol from the ER to the plasma membrane are different. In arteriosclerosis either of these pathways is disturbed and as a consequence lipid-filled macrophages, so called foam cells, and their accumulation lead to the development of fatty streaks. Some fatty streaks subsequently accumulate smooth muscle cells, which migrate from the medial layer. With the secretion of extracellular matrix molecules by the smooth muscle cells, fibrous plaques develop and increase in size. Progression of the disease is characterised by the accumulation of lipids and fibrous elements in the large arteries. The advanced lesions of arteriosclerosis may occlude the artery concerned, and result from an excessive inflammatory-fibroproliferative response to numerous different forms of insult, resulting in restriction of the flow of blood, leading to ischemia. For example, shear stresses are thought to be responsible for the frequent occurrence of arteriosclerotic plaques in regions of the circulatory system where turbulent blood flow occurs, such as branch points and irregular structures [for review see Lusis et al., (2)].

Especially the anterior descending branch of the left coronary artery is susceptible to arteriosclerosis. With time, these plaques can lead to a partial reduction or a sudden total block of the blood's flow. In rare cases coronary artery spasm of unknown origin can provoke that situation as well. The major complications are angina pectoris, myocardial infarction, and sudden cardiac death.

***Ischemia***

Ischemia is a sequela of arteriosclerosis characterised by a lack of oxygen supply in tissues of organs due to inadequate perfusion. Such inadequate perfusion can have number of natural causes, including arteriooslerotic or restenotic lesions, anaemia, or stroke, to name a few. Many medical interventions, such as the interruption of the flow of blood during bypass surgery, for example, also lead to ischemia. In addition to sometimes being caused by diseased cardiovascular tissue, ischemia may sometimes affect cardiovascular tissue, such as in ischemic heart disease. Ischemia may occur in any organ, however, that is suffering a lack of oxygen supply. Not infrequently, two or more causes of ischemia will coexist, such as an increase in oxygen demand due to left ventricular hypertrophy and a reduction in oxygen supply secondary to coronary arteriosclerosis.

***Angina pectoris***

Angina pectoris, another sequela of arteriosclerosis, is characterised by episodes of chest discomfort and pressure due to insufficient blood supply, typically precipitated by exertion and relieved by rest. Angina pectoris is usually triggered by activity, emotional stress, or temperatures and persists only a few minutes. The blood circulation and oxygen supply of the cardiac muscle is reduced for a short period of time due to constriction of coronary arteries.

With progressive arteriosclerosis sensations of pain can be experienced even during periods of rest. Angina pectoris certainly is a sign that a person is at increased risk of heart attack.

***Myocardial infarction***

A heart attack or myocardial infarction occurs when the supply of oxygen and nutrient-rich blood to the heart muscle is severely reduced or cut off completely, resulting in sharp pain. In most patients an acute thrombus, often associated with plaque rupture, occludes the artery. If the blood supply is shut down for a long time cardiac muscle cells die from lack of oxygen. If only a small part of the heart muscle is deprived of oxygen the victim might recover. However, disability or death can result, depending on how much the heart muscle is damaged. Therefore, people with a genetic predisposition or risk factors like diabetes, hypertension, high cholesterol, and obesity should be extremely careful.

Early diagnosis of patients at risk to develop arteriosclerosis will allow to initiate early preventative steps. Prevention, optimal treatment, and rehabilitation measures are necessary to avoid the sequela of arteriosclerosis· such as stroke, angina pectoris, ischemia, or myocardial infarction, to improve the quality of life and to extend overall survival in these patients.

Arteriosclerosis, the most prevalent cardiovascular disease, is the principal cause of heart attack, stroke, and gangrene of the extremities, and thereby the principle cause of death in the United States. Arteriosclerosis is now recognized as a multifactorial disease process associated with several important environmental and genetic risk factors [for a detailed review, see Ross et al. (1)]. Such risk factors include hypertension, elevated levels of homocysteine or LDL/VLDL, smoking, diabetes mellitus, and obesity. Because of the presumed role of the excessive inflammatory-fibroproliferative response in arteriosclerosis and ischemia, a number of researchers have investigated, in the context of arterial injury, the expression of certain factors involved in inflammation, cell recruitment and proliferation. These factors include growth factors, cytokines, and other chemicals, including lipids involved in cell recruitment and migration, cell proliferation and the control of lipid and protein synthesis. These results so far have not lead to satisfactory improvements for the patients and subsequently there is an ongoing need for novel preventive, predictive, diagnostic, prognostic and therapeutic compositions, uses and methods. The foregoing studies are aimed at defining the role of particular gene products in the excessive inflammatory-fibroproliferative response leading to arteriosclerotic plaque formation.

WO0172774 describes a number of genes implicated in the processes of cell cycle progression, including mitosis and meiosis. USPS 6,087,117 deals with the production and use of human nm23 protein and antibodies therefore BARRANS ET AL. is related to "Construction of a human cardiovascular cDNA microarray: portrait of the failing heart" (BIOCHEMICAL AND BIOPHYSICAL RESEARCH COMMUNICATIONS, vol. 280, January 2001 (2001-01), pages 964-969). MCCAFFREY ET AL. deals with "High-level of expression of Egr-1 and Egr-1-inducible genes in mouse and human atherosclerosis" (THE JOURNAL OF CLINICAL INVESTIGATION, vol. 105, no. 5, March 2000 (2000-03), pages 653-662). LAWN ET AL. describe "The Tangier disease gene product ABC1 controls the cellular apolipoprotein-mediated lipid removal pathway" (THE JOURNAL OF CLINICAL INVESTIGATION, vol. 104, no. 8, October 1999 (1999-10), pages R25R-R31)

## SUMMARY OF THE INVENTION

The present invention relates to the method of claim 1. The present invention relates to novel, predictive, diagnostic, and prognostic methods for cardiovascular diseases and arteriosclerosis in particular. Specifically, a gene is identified and described which is differentially expressed in cardiovascular disease states, relative to their expression in normal, or non-cardiovascular disease states, as well as derivatives, fragments, analogues and homologues thereof.

The invention is based, in part, on systematic search strategies involving *in vivo and in vitro* cardiovascular disease experiments coupled with sensitive and high throughput gene expression assays, based on DNA chip technology. In contrast to approaches that merely evaluate the expression of a given gene product presumed to play a role in a disease process, the search strategies and assays used herein permit the identification of all genes, whether known or novel, that are expressed or repressed in the disease condition, as well as the evaluation of their temporal regulation and function during disease progression. This comprehensive approach and evaluation permits the discovery of novel genes and gene products, as well as the identification of an array of genes and gene products (whether novel or known) involved in novel pathways that play a major role in the disease pathology. Thus based on the identification of genes relevant for the pathophysiology of cardiovascular diseases such as arteriosclerosis and it's sequela, the invention provides novel targets useful for prevention, prediction, diagnosis, prognosis monitoring, rational drug screening and design, and/or other therapeutic intervention of cardiovascular diseases and arteriosclerosis in particular.

"Differential expression", as used herein, refers to both quantitative as well as qualitative differences in the genes' expression patterns depending on differential development and/or reaction to lipid environment of macrophages. Differentially expressed genes may represent "marker genes," and/or "target genes" which are named "CVD genes" or "CVD gene" hereinafter. "CVD genes" or "CVD gene" refers to polynucleotides but also to the polypeptides encoded thereby. The expression pattern of a differentially expressed "CVD gene" may be utilized as part of a prognostic or diagnostic cardiovascular disease evaluation., Alternatively, a "CVD gene" may be used in methods for identifying reagents and compounds and uses of these reagents and compounds for the treatment of cardiovascular disease as well as methods of treatment. Also "CVD gene" refers to a differentially expressed gene involved in cardiovascular diseases such that modulation of the level of target gene expression or of target gene product activity may act to ameliorate a cardiovascular disease condition. Compounds that modulate target gene expression or activity of the target gene product can be used in the treatment of cardiovascular disease.

It is an objective of the invention to provide methods for the prediction, diagnosis, prognosis of cardiovascular disease and in particular arteriosclerosis.

In one embodiment, the invention pertains to a method of determining the phenotype of a cell or tissue, comprising detecting the differential expression, relative to a normal or untreated cell, of at least one nucleic acid comprising SEQ ID No. 1

wherein the nucleic acid is differentially expressed by at least about 1.5 fold, at least about 2 fold, at least about 3 fold.

In a further aspect the invention pertains to a method of determining the phenotype of a cell or tissue, comprising detecting the differential expression, relative to a normal or untreated cell, of at least one nucleic acid which hybridises under stringent conditions to SEQ ID No. 1, wherein the nucleic acid is differentially expressed by at least at least about 1.5 fold , at least about 2 fold or at least about 3 fold.

In another embodiment of the invention a "CVD gene" or a gene product of a "CVD gene" can be used to identify cells or tissue in individuals which exhibit a phenotype predisposed to cardiovascular disease or a diseased phenotype, thereby (a) predicting whether an individual is at risk for the development, or (b) diagnosing whether an individual is having, or (c) prognosing the progression or the outcome of the treatment cardiovascular disease and arteriosclerosis in particular.

## DETAILED DESCRIPTION OF THE INVENTION

### *Definitions*

"Biological activity" or "bioactivity" or "activity" or "biological function", which are used interchangeably, herein mean an effector or antigenic function that is directly or indirectly performed by a polypeptide (whether in its native or denatured conformation), or by any fragment thereof *in vivo* or *in vitro.* Biological activities include but are not limited to binding to polypeptides, binding to other proteins or molecules, enzymatic activity, signal transduction, activity as a DNA binding protein, as a transcription regulator, ability to bind damaged DNA, etc. A bioactivity can be modulated by directly affecting the subject polypeptide. Alternatively, a bioactivity can be altered by modulating the level of the polypeptide, such as by modulating expression of the corresponding gene.

The term "biomarker" refers a biological molecule, e.g., a nucleic acid, peptide, hormone, etc., whose presence or concentration can be detected and correlated with a known condition, such as a disease state.

The term "biological sample", as used herein, refers to a sample obtained from an organism or from components (e.g., cells) of an organism. The sample may be of any biological tissue or fluid. Frequently the sample will be a "clinical sample" which is a sample derived from a patient. Such samples include, but are not limited to, sputum, blood, blood cells (e.g., white cells), tissue or fine needle biopsy samples, urine, peritoneal fluid, and pleural fluid, or cells therefrom. Biological samples may also include sections of tissues such as frozen sections taken for histological purposes.

By "array" or "matrix" is meant an arrangement of addressable locations or "addresses" on a device. The locations can be arranged in two dimensional arrays, three dimensional arrays, or other matrix formats. The number of locations can range from several to at least hundreds of thousands. Most importantly, each location represents a totally independent reaction site. Arrays include but are not limited to nucleic acid arrays, protein arrays and antibody arrays. A "nucleic acid array" refers to an array containing nucleic acid probes, such as oligonucleotides or larger portions of genes. The nucleic acid on the array is preferably single stranded. Arrays wherein the probes are oligonucleotides are referred to as "oligonucleotide arrays" or "oligonucleotide chips." A "microarray," also referred to herein as a "biochip" or "biological chip" is an array of regions having a density of discrete regions of at least about $100/cm^2$, and preferably at least about $1000/cm^2$. The regions in a microarray have typical dimensions, e.g., diameters, in the range of between about 10-250 $\mu$m, and are separated from other regions in the array by about the same distance. A "protein array" refers to an array containing polypeptide probes or protein probes which can be in native form or denatured. An "antibody array" refers to an array containing antibodies which include but are not limited to monoclonal antibodies (e.g. from a mouse), chimeric antibodies, humanized antibodies or phage antibodies and single chain antibodies as well as fragments from antibodies.

"Small molecule" as used herein, is meant to refer to a composition, which has a molecular weight of less than about 5 kD and most preferably less than about 4 kD. Small molecules can be nucleic acids, peptides, polypeptides, peptidomimetics, carbohydrates, lipids or other organic (carbon-containing) or inorganic molecules. Many pharmaceutical companies have extensive libraries of chemical and/or biological mixtures, often fungal, bacterial, or algal extracts, which can be screened with any of the assays of the invention to identify compounds that modulate a bioactivity.

"Marker gene," as used herein, refers to a differentially expressed gene whose expression pattern may be utilized as part of a prognostic or diagnostic cardiovascular disease evaluation, or which, alternatively, may be used in methods for identifying compounds useful for the treatment of cardiovascular disease. A marker gene may also have the characteristics of a target gene.

"Target gene", as used herein, refers to a differentially expressed gene involved in cardiovascular disease in a manner by which modulation of the level of target gene expression or of target gene product activity may act to ameliorate symptoms of cardiovascular disease. A target gene may also have the characteristics of a marker gene.

The terms "modulated" or "modulation" and "differentially regulated" as used herein refer to both upregulation (i.e., activation or stimulation (e.g., by agonizing or potentiating) and down regulation [i.e., inhibition or suppression (e.g., by antagonizing, decreasing or inhibiting)].

"Transcriptional regulatory unit" refers to DNA sequences, such as initiation signals, enhancers, and promoters, which induce or control transcription of protein coding sequences with which they are operably linked. In preferred embodiments, transcription of one of the genes is under the control of a promoter sequence (or other transcriptional regulatory sequence) which controls the expression of the recombinant gene in a cell-type in which expression is intended. It will also be understood that the recombinant gene can be under the control of transcriptional regulatory sequences which are the same or which are different from those sequences which control transcription of the naturally occurring forms of the polypeptide.

The term "derivative" refers to the chemical modification of a polypeptide sequence, or a polynucleotide sequence. Chemical modifications of a polynucleotide sequence can include, for example, replacement of hydrogen by an alkyl, acyl, or amino group. A derivative polynucleotide encodes a polypeptide which retains at least one biological or immunological function of the natural molecule. A derivative polypeptide is one modified by glycosylation, pegylation, or any similar process that retains at least one biological or immunological function of the polypeptide from which it was derived.

The present invention provides nucleic acid sequences and proteins encoded thereby, as well as probes derived from the nucleic acid sequences, antibodies directed to the encoded proteins, and predictive, preventive, diagnostic, prognostic and therapeutic methods for individuals which are at risk for or which have cardiovascular disease and arteriosclerosis in particular. The sequences disclosure herein have been found to be differentially expressed in samples relevant for cardiovascular diseases.

The present invention is based on the identification of 74 genes that are differentially regulated (up- or down-regulated) in macrophages with/without incubation with eLDL of patients with clinical evidence of CVD. The identification of 74 human genes which were not known to be differentially regulated in cardiovascular disease states and their significance for the disease is described in the working examples herein.
The characterisation of the expression of these genes in particular disease states provides newly identified roles in cardiovascular diseases. The gene names, the database accession numbers (GenBank and UniGene) and the fold-regulation values are given in the Tables 1 and 2. The primer sequences used for the gene amplification are shown in Table 3. Table 4 provides information about the gene function the functional class of the proteins which are encoded by the 74 differentially regulated genes.

In either situation, detecting expression of these genes in excess of normal expression provides for the diagnosis of cardiovascular disease. Furthermore, in testing the efficacy of compounds during clinical trials, a decrease in the level of the expression of these genes corresponds to a return from a disease condition to a normal state, and thereby indicates a positive effect of the compound. The cardiovascular diseases that may be so diagnosed, monitored in clinical trials, and treated include but are not limited to arteriosclerosis, ischemia, restenosis, and arterial inflammation.

The examples presented below, demonstrate the use of the cardiovascular disease experiments of the invention to identify cardiovascular disease target genes, and demonstrates the use of marker genes in diagnostics and as surrogate markers for testing the efficacy of candidate drugs in basic research and in clinical trials.

"Gene or Genes" as used herein refers to the polynucleotides of SEQ ID NO. 1 to 74, as well as derivatives, fragments, analogs and homologues thereof, the polypeptides encoded thereby, the polypeptides of SEQ ID NO. 75 to 147 and the corresponding genomic transcription units which can be derived or identified with standard techniques well known in the art using the information disclosed in Tables 1 to 3. The GenBank and the UniGene accession numbers of the polynucleotide sequences of the SEQ IDs NO. 1 to 74 are shown in the Tables 1 and 2.

### *Polynucleotides*

A "CVD gene" polynucleotide can be single- or double-stranded and comprises a coding sequence or the complement of a coding sequence for a "CVD gene" polypeptide. Degenerate nucleotide sequences encoding human "CVD gene" polypeptides, as well as homologous nucleotide sequences which are at least about 50, 55, 60, 65, 70, preferably about 75, 90, 96, or 98% identical to the nucleotide sequences of SEQ ID NO.1 to 74 also are "CVD gene" polynucleotides. Percent sequence identity between the sequences of two polynucleotides is determined using computer programs such as ALIGN which employ the FASTA algorithm, using an affine gap search with a gap open penalty of -12 and a gap extension penalty of -2. Complementary DNA (cDNA) molecules, species homologues, and variants of "CVD gene" polynucleotides which encode biologically active "CVD gene" polypeptides also are "CVD gene" polynucleotides.

### *Preparation of Polynucleotides*

A naturally occurring "CVD gene" polynucleotide can be isolated free of other cellular components such as membrane components, proteins, and lipids. Polynucleotides can be made by a cell and isolated using standard nucleic acid purification techniques, or synthesized using an amplification technique, such as the polymerase chain reaction (PCR), or by using an automatic synthesizer. Methods for isolating polynucleotides are routine and are known in the art. Any such technique for obtaining a polynucleotide can be used to obtain isolated "CVD gene" polynucleotides. For example, restriction enzymes and probes can be used to isolate polynucleotide fragments which comprises "CVD gene" nucleotide sequences. Isolated polynucleotides are in preparations which are free or at least 70, 80, or 90% free of other molecules.

"CVD gene" cDNA molecules can be made with standard molecular biology techniques, using "CVD gene" mRNA as a template. Any RNA isolation technique which does not select against the isolation of mRNA may be utilized for the purification of such RNA samples. See, for example, Sambrook et al., (3).; and Ausubel, F. M. et al.,(4). Additionally, large numbers of tissue samples may readily be processed using techniques well known to those of skill in the art, such as, for example, the single-step RNA isolation process of Chomczynski, P. (1989, U.S. Pat. No. 4,843,155).

"CVD gene" cDNA molecules can thereafter be replicated using molecular biology techniques known in the art and disclosed in manuals such as Sambrook et al., (3) . An amplification technique, such as PCR, can be used to obtain additional copies of polynucleotides of the invention, using either human genomic DNA or cDNA as a template.

Alternatively, synthetic chemistry techniques can be used to synthesizes "CVD gene" polynucleotides. The degeneracy of the genetic code allows alternate nucleotide sequences to be synthesized which will encode a "CVD gene" polypeptide or a biologically active variant thereof.

### *Identification of differential expression*

Transcripts within the collected RNA samples which represent RNA produced by differentially expressed genes may be identified by utilizing a variety of methods which are ell known to those of skill in the art. For example, differential screening [Tedder, T. F. et al., (5)], subtractive hybridization [Hedrick, S. M. et al., (6); Lee, S. W. et al., (7)], and, preferably, differential display (Liang, P., and Pardee, A. B., 1993, U.S. Pat. No. 5,262,311), may be utilized to identify nucleic acid sequences derived from genes that are differentially expressed.

Differential screening involves the duplicate screening of a cDNA library in which one copy of the library is screened with a total cell cDNA probe corresponding to the mRNA population of one cell type while a duplicate copy of the cDNA library is screened with a total cDNA probe corresponding to the mRNA population of a second cell type. For example, one cDNA probe may correspond to a total cell cDNA probe of a cell type derived from a control subject, while the second cDNA probe may correspond to a total cell cDNA probe of the same cell type derived from an experimental subject. Those clones which hybridise to one probe but not to the other potentially represent clones derived from genes differentially expressed in the cell type of interest in control versus experimental subjects.

Subtractive hybridization techniques generally involve the isolation of mRNA taken from two different sources, e.g., control and experimental tissue, the hybridization of the mRNA or single-stranded cDNA reverse-transcribed from the isolated mRNA, and the removal of all hybridized, and therefore double-stranded, sequences. The remaining non-hybridized, single-stranded cDNAs, potentially represent clones derived from genes that are differentially expressed in the two mRNA sources. Such single-stranded cDNAs are then used as the starting material for the construction of a library comprising clones derived from differentially expressed genes.

The differential display technique describes a procedure, utilizing the well known polymerase chain reaction (PCR; the experimental embodiment set forth in Mullis, K. B., 1987, U.S. Pat. No. 4,683,202) which allows for the identification of sequences derived from genes which are differentially expressed. First, isolated RNA is reverse-transcribed into single-stranded cDNA, utilizing standard techniques which are well known to those of skill in the art. Primers for the reverse transcriptase reaction may include, but are not limited to, oligo dT-containing primers, preferably of the reverse primer type of oligonucleotide described below. Next, this technique uses pairs of PCR primers, as described below, which allow for the amplification of clones representing a random subset of the RNA transcripts present within any given cell. Utilizing different pairs of primers allows each of the mRNA transcripts present in a cell to be amplified. Among such amplified transcripts may be identified those which have been produced from differentially expressed genes.

The reverse oligonucleotide primer of the primer pairs may contain an oligo dT stretch of nucleotides, preferably eleven nucleotides long, at its 5' end, which hybridises to the poly(A) tail of mRNA or to the complement of a cDNA reverse transcribed from an mRNA poly(A) tail. Second, in order to increase the specificity of the reverse primer, the primer may contain one or more, preferably two, additional nucleotides at its 3' end. Because, statistically, only a subset of the mRNA derived sequences present in the sample of interest will hybridise to such primers, the additional nucleotides allow the primers to amplify only a subset of the mRNA derived sequences present in the sample of interest. This is preferred in that it allows more accurate and complete visualization and characterization of each of the bands representing amplified sequences.

The forward primer may contain a nucleotide sequence expected, statistically, to have the ability to hybridise to cDNA sequences derived from the tissues of interest. The nucleotide sequence may be an arbitrary one, and the length of the forward oligonucleotide primer may range from about 9 to about 13 nucleotides, with about 10 nucleotides being preferred. Arbitrary primer sequences cause the lengths of the amplified partial cDNAs produced to be variable, thus allowing different clones to be separated by using standard denaturing sequencing gel electrophoresis. PCR reaction conditions should be chosen which optimise amplified product yield and specificity, and, additionally, produce amplified products of lengths which may be resolved utilizing standard gel electrophoresis techniques. Such reaction conditions are well known to those of skill in the art, and important reaction parameters include, for example, length and nucleotide sequence of oligonucleotide primers as discussed above, and annealing and elongation step temperatures and reaction times. The pattern of clones resulting from the reverse transcription and amplification of the mRNA of two different cell types is displayed via sequencing gel electrophoresis and compared. Differences in the two banding patterns indicate potentially differentially expressed genes.

When screening for full-length cDNAs, it is preferable to use libraries that have been size-selected to include larger cDNAs. Randomly-primed libraries are preferable, in that they will contain more sequences which contain the 5' regions of genes. Use of a randomly primed library may be especially preferable for situations in which an oligo d(T) library does not yield a full-length cDNA. Genomic libraries can be useful for extension of sequence into 5' nontranscribed regulatory regions.

Commercially available capillary electrophoresis systems can be used to analyse the size or confirm the nucleotide sequence of PCR or sequencing products. For example, capillary sequencing can employ flowable polymers for electrophoretic separation, four different fluorescent dyes (one for each nucleotide) which are laser activated, and detection of the emitted wavelengths by a charge coupled device camera. Output/light intensity can be converted to electrical signal using appropriate software (e.g. GENOTYPER and Sequence NAVIGATOR, Perkin Elmer; ABI), and the entire process from loading of samples to computer analysis and electronic data display can be computer controlled. Capillary electrophoresis is especially preferable for the sequencing of small pieces of DNA which might be present in limited amounts in a particular sample.

Once potentially differentially expressed gene sequences have been identified via bulk techniques such as, for example, those described above, the differential expression of such putatively differentially expressed genes should be corroborated. Corroboration may be accomplished via, for example, such well known techniques as Northern analysis and/or RT-PCR. Upon corroboration, the differentially expressed genes may be further characterized, and may be identified as target and/or marker genes, as discussed, below.

Also, amplified sequences of differentially expressed genes obtained through, for example, differential display may be used to isolate full length clones of the corresponding gene. The full length coding portion of the gene may readily be isolated, without undue experimentation, by molecular biological techniques well known in the art. For example, the isolated differentially expressed amplified fragment may be labeled and used to screen a cDNA library. Alternatively, the labeled fragment may be used to screen a genomic library.

An analysis of the tissue distribution of the mRNA produced by the identified genes may be conducted, utilizing standard techniques well known to those of skill in the art. Such techniques may include, for example, Northern analyses and RT-PCR. Such analyses provide information as to whether the identified genes are expressed in tissues expected to contribute to cardiovascular disease. Such analyses may also provide quantitative information regarding steady state mRNA regulation, yielding data concerning which of the identified genes exhibits a high level of regulation in, preferably, tissues which may be expected to contribute to cardiovascular disease.

Such analyses may also be performed on an isolated cell population of a particular cell type derived from a given tissue. Additionally, standard in situ hybridization techniques may be utilized to provide information regarding which cells within a given tissue express the identified gene. Such analyses may provide information regarding the biological function of an identified gene relative to cardiovascular disease in instances wherein only a subset of the cells within the tissue is thought to be relevant to cardiovascular disease.

*Extending Polynucleotides*

In one embodiment of such a procedure for the identification and cloning of full length gene sequences, RNA may be isolated, following standard procedures, from an appropriate tissue or cellular source. A reverse transcription reaction may then be performed on the RNA using an oligonucleotide primer complimentary to the mRNA that corresponds to the amplified fragment, for the priming of first strand synthesis. Because the primer is anti-parallel to the mRNA, extension will proceed toward the 5' end of the mRNA. The resulting RNA hybrid may then be "tailed" with guanines using a standard terminal transferase reaction, the hybrid may be digested with RNAase H, and second strand synthesis may then be primed with a poly-C primer. Using the two primers, the 5' portion of the gene is amplified using PCR. Sequences obtained may then be isolated and recombined with previously isolated sequences to generate a full-length cDNA of the differentially expressed genes of the invention. For a review of cloning strategies and recombinant DNA techniques, see e.g., Sambrook et al., (3); and Ausubel et al., (4).

Various PCR-based methods can be used to extend the nucleic acid sequences disclosed herein to detect upstream sequences such as promoters and regulatory elements. For example, restriction site PCR uses universal primers to retrieve unknown sequence adjacent to a known locus [Sarkar,(8)]. Genomic DNA is first amplified in the presence of a primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR also can be used to amplify or extend sequences using divergent primers based on a known region [Triglia et al., (9)]. Primers can be designed using commercially available software, such as OLIGO 4.06 Primer Analysis software (National Biosciences Inc., Plymouth, Minn.), to be 2230 nucleotides in length, to have a GC content of 50% or more, and to anneal to the target sequence at temperatures about 68-72 °C. The method uses several restriction enzymes to generate a suitable fragment in the known region of a gene. The fragment is then circularized by intramolecular ligation and used as a PCR template.

Another method which can be used is capture PCR, which involves PCR amplification of DNA fragments adjacent to a known sequence in human and yeast artificial chromosome DNA [Lagerstrom et al.,(10)]. In this method, multiple restriction enzyme digestions and ligations also can be used to place an engineered double-stranded sequence into an unknown fragment of the DNA molecule before performing PCR.

Another method which can be used to retrieve unknown sequences is that of Parker et al., (11). Additionally, PCR, nested primers, and PROMOTERFINDER libraries (CLONTECH, Palo Alto, Calif.) can be used to walk genomic DNA (CLONTECH, Palo Alto, Calif.). This process avoids the need to screen libraries and is useful in finding intron/exon junctions.

The sequences of the identified genes may be used, utilizing standard techniques, to place the genes onto genetic maps, e.g., mouse [Copeland & Jenkins, (12)] and human genetic maps [Cohen, et al., (13)]. Such mapping information may yield information regarding the genes' importance to human disease by, for example, identifying genes which map near genetic regions to which known genetic cardiovascular disease tendencies map.

*Identification of Polynucleotide Variants and Homologues*

Variants and homologues of the "CVD gene" polynucleotides described above also are "CVD gene" polynucleotides. Typically, homologous "CVD gene" polynucleotide sequences can be identified by hybridization of candidate polynucleotides to known "CVD gene" polynucleotides under stringent conditions, as is known in the art. For example, using the following wash conditions: 2X SSC (0.3 M NaCl, 0.03 M sodium citrate, pH 7.0), 0.1 % SDS, room temperature twice, 30 minutes each; then 2X SSC, 0.1% SDS, 50 EC once, 30 minutes; then 2X SSC, room temperature twice, 10 minutes each homologous sequences can be identified which contain at most about 25-30% basepair mismatches. More preferably, homologous nucleic acid strands contain 15-25% basepair mismatches, even more preferably 5-15% basepair mismatches.

Species homologues of the "CVD gene" polynucleotides disclosed herein also can be identified by making suitable probes or primers and screening cDNA expression libraries from other species, such as mice, monkeys, or yeast. Human variants of "CVD gene" polynucleotides can be identified, for example, by screening human cDNA expression libraries. It is well known that the $T_m$ of a double-stranded DNA decreases by 1-1.5 °C with every 1% decrease in homology [Bonner et al., (14)]. Variants of human "CVD gene" polynucleotides or "CVD gene" polynucleotides of other species can therefore be identified by hybridizing a putative homologous "CVD gene" polynucleotide with a polynucleotide having a nucleotide sequence of one of the sequences of the SEQ ID Nos:1 to 74 or the complement thereof to form a test hybrid. The melting temperature of the test hybrid is compared with the melting temperature of a hybrid comprising polynucleotides having perfectly complementary nucleotide sequences, and the number or percent of basepair mismatches within the test hybrid is calculated.

Nucleotide sequences which hybridize to "CVD gene" polynucleotides or their complements following stringent hybridization and/or wash conditions also are "CVD gene" polynucleotides. Stringent wash conditions are well known and understood in the art and are disclosed, for example, in Sambrook et al., (3). Typically, for stringent hybridization conditions a combination of temperature and salt concentration should be chosen that is approximately 12-20°C below the calculated $T_m$ of the hybrid under study. The $T_m$ of a hybrid between a "CVD gene" polynucleotide having a nucleotide sequence of one of the sequences of the SEQ ID NOS: 1 to 74 or the complement thereof and a polynucleotide sequence which is at least about 50, preferably about 75, 90, 96, or 98% identical to one of those nucleotide sequences can be calculated, for example, using the equation of Bolton and McCarthy, (15):

$$T_m = 81.5\ ^{\circ}C - 16.6(\log_{10}[Na^+]) + 0.41(\%G + C) - 0.63(\%formamide) - 600/l),$$

where 1 = the length of the hybrid in basepairs.

Stringent wash conditions include, for example, 4X SSC at 65°C, or 50% formamide, 4X SSC at 28 °C, or 0.5X SSC, 0.1% SDS at 65°C. Highly stringent wash conditions include, for example, 0.2X SSC at 65°C.

The biological function of the identified genes may be more directly assessed by utilizing relevant in vivo and in vitro systems. In vivo systems may include, but are not limited to, animal systems which naturally exhibit cardiovascular disease predisposition, or ones which have been engineered to exhibit such symptoms, including but not limited to the apoE-deficient arteriosclerosis mouse model [Plump et al., (16)].

*Polypeptides*

"CVD gene" polypeptides according to the invention comprise an amino acid selected from the amino acid sequence which are encoded by any of the polynucleotide sequences of the SEQ ID NOS: 1 to 74 or derivatives, fragments, analogues and homologues thereof. A CVD gene" polypeptide of the invention therefore can be a portion, a full-length, or a fusion protein comprising all or a portion of a "CVD gene" polypeptide.

*Protein Purification*

"CVD gene" polypeptides can be purified from any cell which expresses the enzyme, including host cells which have been transfected with "CVD gene" expression constructs. Blood vessels are an especially useful source of "CVD gene" polypeptides. A purified "CVD gene" polypeptide is separated from other compounds which normally associate with the "CVD gene" polypeptide in the cell, such as certain proteins, carbohydrates, or lipids, using methods well-known in the art. Such methods include, but are not limited to, size exclusion chromatography, ammonium sulfate fractionation, ion exchange chromatography, affinity chromato-graphy, and preparative gel electrophoresis. A preparation of purified "CVD gene" polypeptides is at least 80% pure; preferably, the preparations are 90%, 95%, or 99% pure. Purity of the preparations can be assessed by any means known in the art, such as SDS-polyacrylamide gel electrophoresis.

*Expression of Polynucleotides*

To express a "CVD gene" polynucleotide, the polynucleotide can be inserted into an expression vector which contains the necessary elements for the transcription and translation of the inserted coding sequence. Methods which are well known to those skilled in the art can be used to construct expression vectors containing sequences encoding "CVD gene" polypeptides and appropriate transcriptional and translational control elements. These methods include in vitro recombinant DNA techniques, synthetic techniques, and in vivo genetic recombination. Such techniques are described, for example, in Sambrook et al., (3) and in Ausubel et al., (4).

A variety of expression vector/host systems can be utilized to contain and express sequences encoding a "CVD gene" polypeptide. These include, but are not limited to, microorganisms, such as bacteria transformed with recombinant bacteriophage, plasmid, or cosmid DNA expression vectors; yeast transformed with yeast expression vectors, insect cell systems infected with virus expression vectors (e.g., baculovirus), plant cell systems transformed with virus expression vectors (e.g., cauliflower mosaic virus, CaMV; tobacco mosaic virus, TMV) or with bacterial expression vectors (e.g., Ti or pBR322 plasmids), or animal cell systems.

The control elements or regulatory sequences are those regions of the vector enhancers, promoters, 5' and 3' untranslated regions which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells (e.g., heat shock, RUBISCO, and storage protein genes) or from plant viruses (e.g., viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding a "CVD gene" polypeptide, vectors based on SV40 or EBV can be used with an appropriate selectable marker.

*Obtaining Polypeptides*

"CVD gene" polypeptides can be obtained, for example, by purification from human cells, by expression of "CVD gene" polynucleotides, or by direct chemical synthesis.

*Biologically Active Variants*

"CVD gene" polypeptide variants which are biologically active, i.e., retain an "CVD gene" activity, also are "CVD gene" polypeptides. Preferably, naturally or non-naturally occurring "CVD gene" polypeptide variants have amino acid sequences which are at least about 60, 65, or 70, preferably about 75, 80, 85, 90, 92, 94, 96, or 98% identical to the amino acid sequence of any of the sequences of the SEQ ID NOS: 75 to 147 or a fragment thereof. Percent identity between a putative "CVD gene" polypeptide variant and an amino acid sequence encoded by any of the polynucleotide sequences of the SEQ ID NOS: 75 to 147 is determined using the Needleman/Wunsch algorithm (108) with the substitutions-matrix BLOSUM62 (109) and a gap creation penalty of 8 and a gap extension penalty of 2.

Variations in percent identity can be due, for example, to amino acid substitutions, insertions, or deletions. Amino acid substitutions are defined as one for one amino acid replacements. They are conservative in nature when the substituted amino acid has similar structural and/or chemical properties. Examples of conservative replacements are substitution of a leucine with an isoleucine or valine, an aspartate with a glutamate, or a threonine with a serine.

Amino acid insertions or deletions are changes to or within an amino acid sequence. They typically fall in the range of about 1 to 5 amino acids. Guidance in determining which amino acid residues can be substituted, inserted, or deleted without abolishing biological or immunological activity of a "CVD gene" polypeptide can be found using computer programs well known in the art, such as DNASTAR software. Whether an amino acid change results in a biologically active "CVD gene" polypeptide can readily be determined by assaying for "CVD gene" activity, as described for example, in the specific Examples, below. Larger insertions or deletions can also be caused by alternative splicing. Protein domains can be inserted or deleted without altering the main activity of the protein.

*Fusion Proteins*

Fusion proteins are useful for generating antibodies against "CVD gene" polypeptide amino acid sequences and for use in various assay systems. For example, fusion proteins can be used to identify proteins which interact with portions of a "CVD gene" polypeptide. Protein affinity chromatography or library-based assays for protein-protein interactions, such as the yeast two-hybrid or phage display systems, can be used for this purpose. Such methods are well known in the art and also can be used as drug screens.

A "CVD gene" polypeptide fusion protein comprises two polypeptide segments fused together by means of a peptide bond. The first polypeptide segment comprises at least 25, 50, 75, 100, 150, 200, 300, 400, 500, 600, 700 or 750 contiguous amino acids of an amino acid sequence encoded by any polynucleotide sequences of the SEQ ID NOS: 1 to 74 or of a biologically active variant, such as those described above. The first polypeptide segment also can comprise full-length "CVD gene".

The second polypeptide segment can be a full-length protein or a protein fragment. Proteins commonly used in fusion protein construction include β-galactosidase, - glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S-tag, Lex a DNA binding domain (DBD) fusions, GAL4 DNA binding domain fusions, and herpes simplex virus (HSV) BP16 protein fusions.

A fusion protein also can be engineered to contain a cleavage site located between the "CVD gene" polypeptide-encoding sequence and the heterologous protein sequence, so that the "CVD gene" polypeptide can be cleaved and purified away from the heterologous moiety.

A fusion protein can be synthesized chemically, as is known in the art. Preferably, a fusion protein is produced by covalently linking two polypeptide segments or by standard procedures in the art of molecular biology. Recombinant DNA methods can be used to prepare fusion proteins, for example, by making a DNA construct which comprises coding sequences selected from any of the polynucleotide sequences of the SEQ ID NOS:1 to in proper reading frame with nucleotides encoding the second polypeptide segment and expressing the DNA construct in a host cell, as is known in the art. Many kits for constructing fusion proteins are available from companies such as Promega Corporation (Madison, WI), Stratagene (La Jolla, CA), CLONTECH (Mountain View, CA), Santa Cruz Biotechnology (Santa Cruz, CA), MBL International Corporation (MIC; Watertown, MA), and Quantum Biotechnologies (Montreal, Canada; 1-888-DNA-KITS).

*Identification_of Species Homologs*

Species homologues of human a "CVD gene" polypeptide can be obtained using "CVD gene" polypeptide polynucleotides (described below) to make suitable probes or primers for screening cDNA expression libraries from other species, such as mice, monkeys, or yeast, identifying cDNAs which encode homologs of a "CVD gene" polypeptide, and expressing the cDNAs as is known in the art.

*Bacterial and Yeast Expression Systems*

In bacterial systems, a number of expression vectors can be selected depending upon the use intended for the "CVD gene" polypeptide. For example, when a large quantity of the "CVD gene" polypeptide is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding the "CVD gene" polypeptide can be ligated into the vector in frame with sequences for the amino terminal Met and the subsequent 7 residues of β-galactosidase so that a hybrid protein is produced. pIN vectors [Van Heeke & Schuster, (17)] or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione agarose beads followed by elution in the presence of free glutathione. Proteins made in such systems can be designed to include heparin, thrombin, or factor Xa protease cleavage sites so that the cloned polypeptide of interest can be released from the GST moiety at will.

In the yeast Saccharomyces cerevisiae, a number of vectors containing constitutive or inducible promoters such as alpha factor, alcohol oxidase, and PGH can be used. For reviews, see Ausubel et al., (4) and Grant et al., (18).

*Plant and Insect Expression Systems*

If plant expression vectors are used, the expression of sequences encoding "CVD gene" polypeptides can be driven by any of a number of promoters. For example, viral promoters such as the 35S and 19S promoters of CaMV can be used alone or in combination with the omega leader sequence from TMV [Takamatsu, (19)]. Alternatively, plant promoters such as the small subunit of RUBISCO or heat shock promoters can be used [Coruzzi et al., (19); Broglie et al., (21); Winter et al., (22)]. These constructs can be introduced into plant cells by direct DNA transformation or by pathogen-mediated transfection. Such techniques are described in a number of generally available reviews (e.g., Hobbs or Murray, in MCGRAW HILL YEARBOOK OF SCIENCE AND TECHNOLOGY, (23)].

An insect system also can be used to express a "CVD gene" polypeptide. For example, in one such system Autographa californica nuclear polyhedrosis virus (AcNPV) is used as a vector to express foreign genes in Spodoptera frugiperda cells or in Trichoplusia larvae. Sequences encoding "CVD gene" polypeptides can be cloned into a nonessential region of the virus, such as the polyhedrin gene, and placed under control of the polyhedrin promoter. Successful insertion of "CVD gene" polypeptides will render the polyhedrin gene inactive and produce recombinant virus lacking coat protein. The recombinant viruses can then be used to infect S. frugiperda cells or Trichoplusia larvae in which "CVD gene" polypeptides can be expressed [Engelhard et al., (24)].

*Mammalian Expression Systems*

A number of viral-based expression systems can be used to express "CVD gene" polypeptides in mammalian host cells. For example, if an adenovirus is used as an expression vector, sequences encoding "CVD gene" polypeptides can be ligated into an adenovirus transcription/translation complex comprising the late promoter and tripartite leader sequence. Insertion in a nonessential E1 or E3 region of the viral genome can be used to obtain a viable virus which is capable of expressing a "CVD gene" polypeptide in infected host cells [Logan & Shenk, (25)]. If desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (e.g., liposomes, polycationic amino polymers, or vesicles).

Specific initiation signals also can be used to achieve more efficient translation of sequences encoding "CVD gene" polypeptides. Such signals include the ATG initiation codon and adjacent sequences. In cases where sequences encoding a "CVD gene" polypeptide, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic. The efficiency of expression can be enhanced by the inclusion of enhancers which are appropriate for the particular cell system which is used [Scharf et al., (26)].

*Host Cells*

A host cell strain can be chosen for its ability to modulate the expression of the inserted sequences or to process the expressed "CVD gene" polypeptide in the desired fashion. Such modifications of the polypeptide include, but are not limited to, acetylation, carboxylation, glycosylation, phosphorylation, lipidation, and acylation. Posttranslational processing which cleaves a "prepro" form of the polypeptide also can be used to facilitate correct insertion, folding and/or function. Different host cells which have specific cellular machinery and characteristic mechanisms for Post-translational activities (e.g., CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express "CVD gene" polypeptides can be transformed using expression vectors which can contain viral origins of replication and/or endogenous expression elements and a selectable marker gene on the same or on a separate vector. Following the introduction of the vector, cells can be allowed to grow for 12 days in an enriched medium before they are switched to a selective medium. The purpose of the selectable marker is to confer resistance to selection, and its presence allows growth and recovery of cells which successfully express the introduced "CVD gene" sequences. Resistant clones of stably transformed cells can be proliferated using tissue culture techniques appropriate to the cell type. See, for example, R.I. Freshney, (27).

Any number of selection systems can be used to recover transformed cell lines. These include, but are not limited to, the herpes simplex virus thymidine kinase (Wigler et al., (28)] and adenine phosphoribosyltransferase [Lowy et al., (29)] genes which can be employed in $tk^-$ or $aprt^-$ cells, respectively. Also, antimetabolite, antibiotic, or herbicide resistance can be used as the basis for selection. For example, dhfr confers resistance to methotrexate [Wigler et al., (30)], npt confers resistance to the aminoglycosides, neomycin and G418 [Colbere-Garapin et al., (31)], and als and pat confer resistance to chlorsulfuron and phosphinotricin acetyltransferase, respectively. Additional selectable genes have been described. For example, trpB allows cells to utilize indole in place of tryptophan, or hisD, which allows cells to utilize histinol in place of histidine [Hartman & Mulligan, (32)]. Visible markers such as anthocyanins, β-glucuronidase and its substrate GUS, and luciferase and its substrate luciferin, can be used to identify transformants and to quantify the amount of transient or stable protein expression attributable to a specific vector system [Rhodes et al., (33)].

*Detecting Expression and gene product*

Although the presence of marker gene expression suggests that the "CVD gene" polynucleotide is also present, its presence and expression may need to be confirmed. For example, if a sequence encoding a "CVD gene" polypeptide is inserted within a marker gene sequence, transformed cells containing sequences which encode a "CVD gene" polypeptide can be identified by the absence of marker gene function. Alternatively, a marker gene can be placed in tandem with a sequence encoding a "CVD gene" polypeptide under the control of a single promoter. Expression of the marker gene in response to induction or selection usually indicates expression of the "CVD gene" polynucleotide.

Alternatively, host cells which contain a "CVD gene" polynucleotide and which express a "CVD gene" polypeptide can be identified by a variety of procedures known to those of skill in the art. These procedures include, but are not limited to, DNA-DNA or DNA-RNA hybridization and protein bioassay or immunoassay techniques which include membrane, solution, or chip-based technologies for the detection and/or quantification of nucleic acid or protein. For example, the presence of a polynucleotide sequence encoding a "CVD gene" polypeptide can be detected by DNA-DNA or DNA-RNA hybridization or amplification using probes or fragments or fragments of polynucleotides encoding a "CVD gene" polypeptide. Nucleic acid amplification-based assays involve the use of oligonucleotides selected from sequences encoding a "CVD gene" polypeptide to detect transformants which contain a "CVD gene" polynucleotide.

A variety of protocols for detecting and measuring the expression of a "CVD gene" polypeptide, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on a "CVD gene" polypeptide can be used, or a competitive binding assay can be employed. These and other assays are described in Hampton et al., (34) and Maddox et al., (35).

A wide variety of labels and conjugation techniques are known by those skilled in the art and can be used in various nucleic acid and amino acid assays. Means for producing labeled hybridization or PCR probes for detecting sequences related to polynucleotides encoding "CVD gene" polypeptides include oligo labeling, nick translation, end-labeling, or PCR amplification using a labeled nucleotide. Alternatively, sequences encoding a "CVD gene" polypeptide can be cloned into a vector for the production of an mRNA probe. Such vectors are known in the art, are commercially available, and can be used to synthesise RNA probes in vitro by addition of labelled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

*Expression and Purification of Polypeptides*

Host cells transformed with nucleotide sequences encoding a "CVD gene" polypeptide can be cultured under conditions suitable for the expression and recovery of the protein from cell culture. The polypeptide produced by a transformed cell can be secreted or stored intracellular depending on the sequence and/or the vector used. As will be understood by those of skill in the art, expression vectors containing polynucleotides which encode "CVD gene" polypeptides can be designed to contain signal sequences which direct secretion of soluble "CVD gene" polypeptides through a prokaryotic or eukaryotic cell membrane or which direct the membrane insertion of membrane-bound "CVD gene" polypeptide.

As discussed above, other constructions can be used to join a sequence encoding a "CVD gene" polypeptide to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor Xa or enterokinase (Invitrogen, San Diego, CA) between the purification domain and the "CVD gene" polypeptide also can be used to facilitate purification.

One such expression vector provides for expression of a fusion protein containing a "CVD gene" polypeptide and 6 histidine residues preceding a thioredoxin or an enterokinase cleavage site. The histidine residues facilitate purification by IMAC (immobilized metal ion affinity chromatography, as described in Porath et al., (36), while the enterokinase cleavage site provides a means for purifying the "CVD gene" polypeptide from the fusion protein. Vectors which contain fusion proteins are disclosed in Kroll et al., (37).

*Chemical Synthesis*

Sequences encoding a "CVD gene" polypeptide can be synthesised, in whole or in part, using chemical methods well known in the art (see Caruthers et al., (38) and Horn et al., (39). Alternatively, a "CVD gene" polypeptide itself can be produced using chemical methods to synthesise its amino acid sequence, such as by direct peptide synthesis using solid-phase techniques [Merrifield, (40) and Roberge et al., (41)]. Protein synthesis can be performed using manual techniques or by automation. Automated synthesis can be achieved, for example, using Applied Biosystems 431A Peptide Synthesizer (Perkin Elmer). Optionally, fragments of "CVD gene" polypeptides can be separately synthesized and combined using chemical methods to produce a full-length molecule.

The newly synthesized peptide can be substantially purified by preparative high performance liquid chromatography [Creighton, (42)]. The composition of a synthetic "CVD gene" polypeptide can be confirmed by amino acid analysis or sequencing (e.g., the Edman degradation procedure; see Creighton, (42). Additionally, any portion of the amino acid sequence of the "CVD gene" polypeptide can be altered during direct synthesis and/or combined using chemical methods with sequences from other proteins to produce a variant polypeptide or a fusion protein.

*Production of Altered Polypeptides*

As will be understood by those of skill in the art, it may be advantageous to produce "CVD gene" polypeptide-encoding nucleotide sequences possessing non-natural occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence.

The nucleotide sequences disclosed herein can be engineered using methods generally known in the art to alter "CVD gene" polypeptide-encoding sequences for a variety of reasons, including but not limited to, alterations which modify the cloning, processing, and/or expression of the polypeptide or mRNA product. DNA shuffling by random fragmentation and PCR re-assembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

*Diagnostic and Prognostic Assays*

The present invention provides method for determining whether a subject is at risk for developing cardiovascular disease and arteriosclerosis in particular by detecting the disclosed biomarkers, i.e., the disclosed polynucleotide markers comprising any of the polynucleotides sequences of the SEQ ID NO:1 and/or the polypeptide markers encoded thereby or comprising any of the polypeptide sequences of the SEQ ID NO: 75 for cardiovascular disease and arteriosclerosis in particular in particular encoded thereby.

In clinical applications, biological samples can be screened for the presence and/or absence of the biomarkers identified herein. Such samples are for example needle biopsy cores, surgical resection samples, or body fluids like serum and urine. For example, these methods include obtaining a biopsy, which is optionally fractionated by cryostat sectioning to enrich diseases cells to about 80% of the total cell population. In certain embodiments, nucleic acids extracted from these samples may be amplified using techniques well known in the art. The expression levels of selected markers detected would be compared with statistically valid groups of diseased and healthy samples.

In one embodiment the diagnostic method comprises determining whether a subject has an abnormal mRNA and/or protein level of the disclosed markers, such as by Northern blot analysis, reverse transcription-polymerase chain reaction (RT-PCR), in situ hybridization, immunoprecipitation, Western blot hybridization, or immunohistochemistry. According to the method, cells are obtained from a subj ect and the levels of the disclosed biomarkers, protein or mRNA level, is determined and compared to the level of these markers in a healthy subject. An abnormal level of the biomarker polypeptide or mRNA levels is likely to be indicative of cardiovascular disease such as arteriosclerosis.

*1. Polynucleotide detection*

In one embodiment, the method for the diagnosis or prognosis of cardiovascular disease is done by the detection of:

(a) polynucleotide selected from the polynucleotide of the SEQ ID NO: 1;
(b) a polynucleotide which hybridises under stringent conditions to a polynucleotide specified in (a) encoding a polypeptide exhibiting the same biological function as specified for the respective sequence in SEQ ID NO.75;
(c) a polynucleotide the sequence of which deviates from the polynucleotide specified in (a) and (b) due to the generation of the genetic code and encodes a polypeptide exhibiting the same biological function as specified for the respective sequence in SEQ ID NO 1;

(d) a polynucleotide which represents a specific fragment, derivative or allelic variation of a polynucleotide sequence specified in (a) to (c); in a biological sample comprising the following steps: hybridizing any polynucleotide specified in (a) to (d) to a nucleic acid material of a biological sample, thereby forming a hybridization complex; and detecting said hybridization complex.

In another embodiment the method for the diagnosis or prognosis of cardiovascular disease is done as just described but, wherein before hybridization, the nucleic acid material of the biological sample is amplified.

In another embodiment the method for the diagnosis or prognosis of cardiovascular disease is done by the detection of:

(a) a polynucleotide selected from the polynucleotides of the SEQ ID NO: 1;

(b) a polynucleotide which hybridises under stringent conditions to a polynucleotide specified in (a) encoding a polypeptide exhibiting the same biological function as specified for the respective sequence in SEQ ID NO 75;

(c) a polynucleotide the sequence of which deviates from the polynucleotide specified in (a) and (b) due to the generation of the genetic code and encodes a polypeptide exhibiting the same biological function as specified for the respective sequence in SEQ ID NO 75;

(d) a polynucleotide which represents a specific fragment, derivative or allelic variation of a polynucleotide sequence specified in (a) to (c);

(e) a polypeptide encoded by a polynucleotide sequence specified in (a) to (d) comprising the steps of contacting a biological sample with a reagent which specifically interacts with the polynucleotide specified in (a) to (d) or the polypeptide specified in (e).

<u>*2. DNA array technology*</u>

In one embodiment, the present Invention also provides a method wherein polynucleotide probes are immobilized an a DNA chip in an organised array. Oligonucleotides can be bound to a solid Support by a variety of processes, including lithography. For example a chip can hold up to 4100,00 oligonucleotides (GeneChip, Affymetrix). These polynucleotide probes comprise a nucleotide sequence at least about 12 nucleotides in length, preferably at least about 15 nucleotides, more preferably at least about 25 nucleotides, and most preferably at least about 40 nucleotides, and up to all or nearly all of a sequence which is complementary to a portion of the coding sequence of a marker polynucleotide sequence of the SEQ ID NO:1 and is differentially expressed in cardiovascular tissue. The present invention provides significant advantages over the available tests for cardiovascular disease, such as arteriosclerosis, because it increases the reliability of the test by providing an array of polynucleotide markers an a single chip.

The method includes obtaining a biopsy of an affected artery, which is optionally fractionated by cryostat sectioning to enrich diseased cells to about 80% of the total cell population and the use of body fluids such as serum or urine. The DNA or RNA is then extracted, amplified, and analysed with a DNA chip to determine the presence of absence of the marker polynucleotide sequences. In one embodiment, the polynucleotide probes are spotted onto a substrate in a two-dimensional matrix or array, samples of polynucleotides can be labeled and then hybridised to the probes. Double-stranded polynucleotides, comprising the labeled sample polynucleotides bound to probe polynucleotides, can be detected once the unbound portion of the sample is washed away.

The probe polynucleotides can be spotted an substrates including glass, nitrocellulose, etc. The probes can be bound to the Substrate by either covalent bonds or by non-specific interactions, such as hydrophobic interactions. The sample polynucleotides can be labelled using radioactive labels, fluorophores, chromophores, etc. Techniques for constructing arrays and methods of using these arrays are described in EP No. 0 799 897; PCT No. WO 97/29212; PCT No. WO 97/27317; EP No. 0 785 280; PCT No. WO 97/02357; U.S. Pat. No. 5,593,839; U.S. Pat. No. 5,578,832; EP No. 0 728 520; U.S. Pat. No. 5,599,695; EP No. 0 721 016; U.S. Pat. No. 5,556,752; PCT No. WO 95/22058; and U.S. Pat. No. 5,631,734. Further, arrays can be used to examine differential expression of genes and can be used to determine gene function. For example, arrays of the instant polynucleotide sequences can be used to determine if any of the polynucleotide sequences are differentially expressed between normal cells and diseased cells, for example. High expression of a particular message in a diseased sample, which is not observed in a corresponding normal sample, can indicate a cardiovascular disease specific protein.

Accordingly, in one aspect, the invention provides probes and primers that are specific to the unique polynucleotide markers disclosed herein.

In one embodiment, the method comprises using a polynucleotide probe to determine the presence of cardiovascular disease cells in a tissue from a patient. Specifically, the method comprises:

1) providing a polynucleotide probe comprising a nucleotide sequence at least 12 nucleotides in length, preferably at least 15 nucleotides, more preferably, 25 nucleotides, and most preferably at least 40 nucleotides, and up to all or nearly all of the coding sequence which is complementary to a portion of the coding sequence of a polynucleotide selected from the polynucleotides or the SEQ ID NOS:1 or a sequence complementary thereto and is
2) differentially expressed in cardiovascular disease, such as arteriosclerosis ;
3) obtaining a tissue sample from a patient with cardiovascular disease and arteriosclerosis in particular;
4) providing a second tissue sample from a patient with no cardiovascular disease;
5) contacting the polynucleotide probe under stringent conditions with RNA of each of said first and second tissue samples (e.g., in a Northern blot or in situ hybridization assay); and
6) comparing (a) the amount of hybridization of the probe with RNA of the first tissue sample, with (b) the amount of hybridization of the probe with RNA of the second tissue sample;

wherein a statistically significant difference in the amount of hybridization with the RNA of the first tissue sample as compared to the amount of hybridization with the RNA of the second tissue sample is indicative of cardiovascular disease and arteriosclerosis in particular in the first tissue sample.

To illustrate, such genetic lesions can be detected by ascertaining the existence of at least one of these:

I. a deletion of one or more nucleotides from the polynucleotide sequence
II. an addition of one or more nucleotides to the polynucleotide sequence
III. a Substitution of one or more nucleotides of the polynucleotide sequence
IV. a gross chromosomal rearrangement of the polynucleotide sequence
V. a gross alteration in the level of a messenger RNA transcript of the polynucleotide sequence
VI. aberrant modification of the polynucleotide sequence, such as of the methylation Pattern of the genomic DNA
VII. the presence of a non-wild type splicing Pattern of a messenger RNA transcript of the gene
VIII. a non-wild type level of the marker polypeptide
IX. allelic loss of the gene
X. inappropriate post-translational modification of the marker polypeptide

Herein are described assay techniques for detecting mutations in the encoding polynucleotide sequence. These methods include, but are not limited to, methods involving sequence analysis, Southern blot hybridization, restriction enzyme site mapping, and methods involving detection of absence of nucleotide pairing between the polynucleotide to be analyzed and a probe.

Specific diseases or disorders, e.g., genetic diseases or disorders, are associated with specific allelic variants of polymorphic regions of certain genes, which do not necessarily encode a mutated Protein. Thus, the presence of a specific allelic variant of a polymorphic region of a gene in a subject can render the subject susceptible to developing a specific disease or disorder. Polymorphic regions in genes, can be identified, by determining the nucleotide sequence of genes in populations of individuals. If a polymorphic region is identified, then the link with a specific disease can be determined by studying specific populations of individuals, e.g. individuals which developed a specific disease, such as cardiovascular disease. A polymorphic region can be located in any region of a gene, e.g., exons, in coding or non coding regions of exons, introns, and promoter region.

Herein is described a polynucleotide composition comprising a polynucleotide probe including a region of nucleotide sequence which is capable of hybridising to a sense or antisense sequence of a gene or naturally occurring mutants thereof, or 5' or 3' flanking sequences or intronic sequences naturally associated with the subject genes or naturally occurring mutants thereof. The polynucleotide of a cell is rendered accessible for hybridization, the probe is contacted with the polynucleotide of the sample, and the hybridization of the probe to the sample polynucleotide is detected. Such techniques can be used to detect lesions or allelic variants at either the genomic or mRNA level, including deletions, substitutions, etc., as well as to determine mRNA transcript levels.

A preferred detection method is allele specific hybridization using probes overlapping the mutation or polymorphic site and having about 5, 10, 20, 25, or 30 nucleotides around the mutation or polymorphic region. In a preferred embodiment of the invention, several probes capable of hybridising specifically to allelic variants are attached to a solid phase support, e.g., a "chip". Mutation detection analysis using these chips comprising oligonucleotides, also termed "DNA probe arrays" is described e.g., in Cronin et al. (43). In one embodiment, a chip comprises all the allelic variants of at least one polymorphic region of a gene. The solid phase support is then contacted with a test polynucleotide and hybridization to the specific probes is detected. Accordingly, the identity of numerous allelic variants of one or more genes can be identified in a simple hybridization experiment.

In certain embodiments, detection of the lesion comprises utilizing the probe/primer in a polymerase chain reaction (PCR) (see, e.g. U.S. Patent Nos. 4,683,195 and 4,683,202), such as anchor PCR or RACE PCR, or, alternatively, in a ligase chain reaction (LCR) (see, e.g., Landegran et al., (44) and Nakazawa et al., (45)], the latter of which can be particularly useful for detecting point mutations in the gene (see Abravaya et al., (46)]. In a merely illustrative embodiment, the method includes the steps of (i) collecting a sample of cells from a patient, (ii) isolating polynucleotide (e.g., genomic, mRNA or both) from the cells of the sample, (iii) contacting the polynucleotide sample with one or more primers which specifically hybridise to a polynucleotide sequence under conditions such that hybridization and amplification of the polynucleotide (if present) occurs, and (iv) detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication [Guatelli, J.C. et al., (47)], transcriptional amplification system [Kwoh, D.Y. et al., (48)], Q-Beta replicase [Lizardi, P.M. et al., (49)], or any other polynucleotide amplification method, followed by the detection of the amplified molecules using techniques well known to those of skill in the art. These detection schemes are especially useful for the detection of polynucleotide molecules if such molecules are present in very low numbers.

In a preferred embodiment of the subject assay, mutations in, or allelic variants, of a gene from a sample cell are identified by alterations in restriction enzyme cleavage patterns. For example, sample and control DNA is isolated, amplified (optionally), digested with one or more restriction endonucleases, and fragment length sizes are determined by gel electrophoresis. Moreover; the use of sequence specific ribozymes (see, for example, U.S. Patent No. 5,498,531) can be used to score for the presence of specific mutations by development or loss of a ribozyme cleavage site.

*4. In situ hybridization*

In one aspect, the method comprises *in situ* hybridization with a probe derived from a given marker polynucleotide, which sequence is selected from the SEQ ID NO:1 or a sequence complementary thereto. The method comprises contacting the labeled hybridization probe with a sample of a given type of tissue from a patient potentially having cardiovascular disease and arteriosclerosis in particular as well as normal tissue from a person with no cardiovascular disease, and determining whether the probe labels tissue of the patient to a degree significantly different (e.g., by at least a factor of two, or at least a factor of five, or at least a factor of twenty, or at least a factor of fifty) than the degree to which normal tissue is labelled.

*5. Immunohistochemistry*

Where tissue samples are employed, immunohistochemical staining may be used to determine the number of cells having the marker polypeptide phenotype. For such staining, a multiblock of tissue is taken from the biopsy or other tissue sample and subjected to proteolytic hydrolysis, employing such agents as protease K or pepsin. In certain embodiments, it may be desirable to isolate a nuclear fraction from the sample cells and detect the level of the marker polypeptide in the nuclear fraction.

The tissues samples are fixed by treatment with a reagent such as formalin, glutaraldehyde, methanol, or the like. The samples are then incubated with an antibody, preferably a monoclonal antibody, with binding specificity for the marker polypeptides. This antibody may be conjugated to a Label for subsequent detection of binding, samples are incubated for a time Sufficient for formation of the immuno-complexes. Binding of the antibody is then detected by virtue of a Label conjugated to this antibody. Where the antibody is unlabelled, a second labelled antibody may be employed, e.g., which is specific for the isotype of the anti-marker polypeptide anti-body. Examples of Labels which may be employed include radionuclides, fluorescens, chemiluminescens, enzymes and such.

Where enzymes are employed, the Substrate for the enzyme may be added to the samples to provide a coloured or fluorescent product. Examples of suitable enzymes for use in conjugates include horseradish peroxidase, alkaline phosphatase, malate dehydrogenase and the like. Where not commercially available, such antibody-enzyme conjugates are readily produced by techniques known to those skilled in the art.

In one embodiment, the assay is performed as a dot blot assay. The dot blot assay finds particular application where tissue samples are employed as it allows determination of the average amount of the marker polypeptide associated with a Single cell by correlating the amount of marker polypeptide in a cell-free extract produced from a predetermined number of cells.

The diagnostic methods of the subject invention may also be employed as follow-up to treatment, e.g., quantification of the level of marker polypeptides may be indicative of the effectiveness of current or previously employed therapies for cardiovascular diseases and arteriosclerosis in particular as well as the effect of these therapies upon patient prognosis.

The diagnostic assays described above can be adapted to be used as prognostic assays, as well. Such an application takes advantage of the sensitivity of the assays of the Invention to events which take place at characteristic stages in the progression of plaque generation in case of arteriosclerosis. For example, a given marker gene may be up- or down-regulated at a very early stage, perhaps before the cell is developing into a foam cell, while another marker gene may be characteristically up or down regulated only at a much later stage. Such a method could involve the steps of contacting the mRNA of a test cell with a polynucleotide probe derived from a given marker polynucleotide which is expressed at different characteristic levels in cardiovascular disease tissue cells at different stages of arteriosclerosis progression, and determining the approximate amount of hybridization of the probe to the mRNA of the cell, such amount being an indication of the level of expression of the gene in the cell, and thus an indication of the stage of disease progression of the cell; alternatively, the assay can be carried out with an antibody specific for the gene product of the given marker polynucleotide, contacted with the proteins of the test cell. A battery of such tests will disclose not only the existence of a certain arteriosclerotic plaque, but also will allow the clinician to select the mode of treatment most appropriate for the disease, and to predict the likelihood of success of that treatment.

The methods of the invention can also be used to follow the clinical course of a given cardiovascular disease predisposition. For example, the assay of the Invention can be applied to a blood sample from a patient; following treatment of the patient for CVD, another blood sample is taken and the test repeated. Successful treatment will result in removal of demonstrate differential expression, characteristic of the cardiovascular disease tissue cells, perhaps approaching or even surpassing normal levels.

*6. Data analysis methods*

Comparison of the expression levels of one or more "CVD genes" with reference expression levels, e.g., expression levels in diseased cells of cardiovascular disease or in normal counterpart cells, is preferably conducted using computer systems. In one embodiment, expression levels are obtained in two cells and these two sets of expression levels are introduced into a computer system for comparison. In a preferred embodiment, one set of expression levels is entered into a computer system for comparison with values that are already present in the computer system, or in computer-readable form that is then entered into the computer system.

Herein is described a computer readable form of the gene expression profile data of the invention, or of values corresponding to the level of expression of at least one "CVD gene" in a diseased cell. The values can be mRNA expression levels obtained from experiments, e.g., microarray analysis. The values can also be mRNA levels normalised relative to a reference gene whose expression is constant in numerous cells under numerous conditions, e.g., GAPDH. In other embodiments, the values in the computer are ratios of, or differences between, normalised or non-normalized mRNA levels in different samples.

The gene expression profile data can be in the form of a table, such as an Excel table. The data can be alone, or it can be part of a larger database, e.g., comprising other expression profiles. For example, the expression profile data of the invention can be part of a public database. The computer readable form can be in a computer. In another embodiment, the invention provides a computer displaying the gene expression profile data.

In one embodiment, the invention provides a method for determining the similarity between the level of expression of one or more "CVD genes" in a first cell, e.g., a cell of a subject, and that in a second cell, comprising obtaining the level of expression of one or more "CVD genes" in a first cell and entering these values into a computer comprising a database including records comprising values corresponding to levels of expression of one or more "CVD genes" in a second cell, and processor instructions, e.g., a user interface, capable of receiving a selection of one or more values for comparison purposes with data that is stored in the computer. The computer may further comprise a means for converting the comparison data into a diagram or chart or other type of output.

Values representing expression levels of "CVD genes" are entered into a computer system, comprising one or more databases with reference expression levels obtained from more than one cell. For example, the computer comprises expression data of diseased and normal cells. Instructions are provided to the computer, and the computer is capable of comparing the data entered with the data in the computer to determine whether the data entered is more similar to that of a normal cell or of a diseased cell.

The computer comprises values of expression levels in cells of subjects at different stages of cardiovascular disease, and the computer is capable of comparing expression data entered into the computer with the data stored, and produce results indicating to which of the expression profiles in the computer, the one entered is most similar, such as to determine the stage of cardiovascular disease in the subject.

The reference expression profiles in the computer are expression profiles from cells of cardiovascular disease of one or more subjects, which cells are treated *in vivo* or in *vitro* with a drug used for therapy of cardiovascular disease. Upon entering of expression data of a cell of a subject treated *in vitro* or *in vivo* with the drug, the computer is instructed to compare the data entered to the data in the computer, and to provide results indicating whether the expression data input into the computer are more similar to those of a cell of a subject that is responsive to the drug or more similar to those of a cell of a subject that is not responsive to the drug. Thus, the results indicate whether the subject is likely to respond to the treatment with the drug or unlikely to respond to it.

Herein is described a system that comprises a means for receiving gene expression data for one or a plurality of genes; a means for comparing the gene expression data from each of said one or plurality of genes to a common reference frame; and a means for presenting the results of the comparison. This system may further comprise a means for clustering the data.

Herein is described a computer program for analysing gene expression data comprising (i) a computer code that receives as input gene expression data for a plurality of genes and (ii) a computer code that compares said gene expression data from each of said plurality of genes to a common reference frame.

Herein is described a machine-readable or computer-readable medium including program instructions for performing the following steps: (i) comparing a plurality of values corresponding to expression levels of one or more genes characteristic of cardiovascular disease in a query cell with a database including records comprising reference expression or expression profile data of one or more reference cells and an annotation of the type of cell; and (ii) indicating to which cell the query cell is most similar based on similarities of expression profiles. The reference cells can be cells from subjects at different stages of cardiovascular disease. The reference cells can also be cells from subjects responding or not responding to a particular drug treatment and optionally incubated in *vitro* or *in vivo* with the drug.

The reference cells may also be cells from subjects responding or not responding to several different treatments, and the computer system indicates a preferred treatment for the subject. Accordingly, the invention provides a method for selecting a therapy for a patient having cardiovascular disease, the method comprising: (i) providing the level of expression of one or more genes characteristic of cardiovascular disease in a diseased cell of the patient; (ii) providing a plurality of reference profiles, each associated with a therapy, wherein the subject expression profile and each reference profile has a plurality of values, each value representing the level of expression of a gene characteristic of cardiovascular disease; and (iii) selecting the reference profile most similar to the subject expression profile, to thereby select a therapy for said patient. In a preferred embodiment step (iii) is performed by a computer. The most similar reference profile may be selected by weighing a comparison value of the plurality using a weight value associated with the corresponding expression data.

The relative abundance of an mRNA in two biological samples can be scored as a perturbation and its magnitude determined (i.e., the abundance is different in the two sources of mRNA tested), or as not perturbed (i.e., the relative abundance is the same). In various embodiments, a difference between the two sources of RNA of at least a factor of about 25% (RNA from one source is 25% more abundant in one source than the other source), more usually about 50%, even more often by a factor of about 2 (twice as abundant), 3 (three times as abundant) or 5 (five times as abundant) is scored as a perturbation. Perturbations can be used by a computer for calculating and expression comparisons.

Preferably, in addition to identifying a perturbation as positive or negative, it is advantageous to determine the magnitude of the perturbation. This can be carried out, as noted above, by calculating the ratio of the emission of the two fluorophores used for differential labeling, or by analogous methods that will be readily apparent to those of skill in the art.

The computer readable medium may further comprise a pointer to a descriptor of a stage of cardiovascular disease or to a treatment for cardiovascular disease.

In operation, the means for receiving gene expression data, the means for comparing the gene expression data, the means for presenting, the means for normalising, and the means for clustering within the context of the systems of the present invention can involve a programmed computer with the respective functionalities described herein, implemented in hardware or hardware and software; a logic circuit or other component of a programmed computer that performs the operations specifically identified herein, dictated by a computer program; or a computer memory encoded with executable instructions representing a computer program that can cause a computer to function in the particular fashion described herein.

Those skilled in the art will understand that the systems and methods of the present invention may be applied to a variety of systems, including IBM-compatible personal computers running MS-DOS or Microsoft Windows.

The computer may have internal components linked to external components. The internal components may include a processor element interconnected with a main memory. The computer system can be an Intel Pentium®-based processor of 200 MHz or greater clock rate and with 32 MB or more of main memory. The external component may comprise a mass storage, which can be one or more hard disks (which are typically packaged together with the processor and memory). Such hard disks are typically of 1 GB or greater storage capacity. Other external components include a user interface device, which can be a monitor, together with an inputing device, which can be a "mouse", or other graphic input devices, and/or a keyboard. A printing device can also be attached to the computer.

Typically, the computer system is also linked to a network link, which can be part of an Ethernet link to other local computer systems, remote computer systems, or wide area communication networks, such as the Internet. This network link allows the computer system to share data and processing tasks with other computer systems.

Loaded into memory during operation of this system are several software components, which are both standard in the art and special to the instant invention. These software components collectively cause the computer system to function according to the methods of this invention. These software components are typically stored on a mass storage. A software component represents the operating system, which is responsible for managing the computer system and its network interconnections. This operating system can be, for example, of the Microsoft Windows' family, such as Windows 95, Windows 98, or Windows NT. A software component represents common languages and functions conveniently present on this system to assist programs implementing the methods specific to this invention. Many high or low level computer languages can be used to program the analytic methods of this invention. Instructions can be interpreted during run-time or compiled. Preferred languages include C/C++, and JAVA®. Most preferably, the methods of this invention are programmed in mathematical software packages which allow symbolic entry of equations and high-level specification of processing, including algorithms to be used, thereby freeing a user of the need to procedurally program individual equations or algorithms. Such packages include Matlab from Mathworks (Natick, Mass.), Mathematica from Wolfram Research (Champaign, Ill.), or S-Plus from Math Soft (Cambridge, Mass.). Accordingly, a software component represents the analytic methods of this invention as programmed in a procedural language or symbolic package. In a preferred embodiment, the computer system also contains a database comprising values representing levels of expression of one or more genes characteristic of cardiovascular disease. The database may contain one or more expression profiles of genes characteristic of cardiovascular disease in different cells.

In an exemplary implementation, to practice the methods of the present invention, a user first loads expression profile data into the computer system. These data can be directly entered by the user from a monitor and keyboard, or from other computer systems linked by a network connection, or on removable storage media such as a CD-ROM or floppy disk or through the network. Next the user causes execution of expression profile analysis software which performs the steps of comparing and, e.g., clustering co-varying genes into groups of genes.

In another exemplary implementation, expression profiles are compared using a method described in U.S. Patent No. 6,203,987. A user first loads expression profile data into the computer system. Geneset profile definitions are loaded into the memory from the storage media or from a remote computer, preferably from a dynamic geneset database system, through the network. Next the user causes execution of projection software which performs the steps of converting expression profile to projected expression profiles. The projected expression profiles are then displayed.

In yet another exemplary implementation, a user first leads a projected profile into the memory. The user then causes the loading of a reference profile into the memory. Next, the user causes the execution of comparison software which performs the steps of objectively comparing the profiles.

*Antisense oligonucleotides*

Antisense oligonucleotides are nucleotide sequences which are complementary to a specific DNA or RNA sequence. Once introduced into a cell, the complementary nucleotides combine with natural sequences produced by the cell to form complexes and block either transcription or translation. Preferably, an antisense oligonucleotide is at least 6 nucleotides in length, but can be at least 7, 8, 10, 12, 15, 20, 25, 30, 35, 40, 45, or 50 or more nucleotides long. Longer sequences also can be used. Antisense oligonucleotide molecules can be provided in a DNA construct and introduced into a cell as described above to decrease the level of "CVD gene" gene products in the cell.

Antisense oligonucleotides can be deoxyribonucleotides, ribonucleotides, peptide nucleic acids (PNAs; described in U.S. Pat. No. 5,714,331), locked nucleic acids (LNAs; described in WO 99/12826), or a combination of them. Oligonucleotides can be synthesised manually or by an automated synthesiser, by covalently linking the 5' end of one nucleotide with the 3' end of another nucleotide with non-phosphodiester internucleotide linkages such alkylphosphonates, phosphorothioates, phosphorodithioates, alkylphosphonothioates, alkylphosphonates, phosphoramidates, phosphate esters, carbamates, acetamidate, carboxymethyl esters, carbonates, and phosphate triesters. See Brown, (50); Sonveaux, (S1) and Uhlmann et al., (52).

Modifications of "CVD gene" expression can be obtained by designing antisense oligonucleotides which will form duplexes to the control, 5', or regulatory regions of the "CVD gene". Oligonucleotides derived from the transcription initiation site, e.g., between positions 10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or chaperons. Therapeutic advances using triplex DNA have been described in the literature [e.g., Gee et al., (53)]. An antisense oligonucleotide also can be designed to block translation of mRNA by preventing the transcript from binding to ribosomes.

Precise complementarity is not required for successful complex formation between an antisense oligonucleotide and the complementary sequence of a "CVD gene" polynucleotide. Antisense oligonucleotides which comprise, for example, 2, 3, 4, or 5 or more stretches of contiguous nucleotides which are precisely complementary to a "CVD gene" polynucleotide, each separated by a stretch of contiguous nucleotides which are not complementary to adjacent "CVD gene" nucleotides, can provide sufficient targeting specificity for "CVD gene" mRNA. Preferably, each stretch of complementary contiguous nucleotides is at least 4, 5, 6, 7, or 8 or more nucleotides in length. Non-complementary intervening sequences are preferably 1, 2, 3, or 4 nucleotides in length. One skilled in the art can easily use the calculated melting point of an antisense-sense pair to determine the degree of mismatching which will be tolerated between a particular antisense oligonucleotide and a particular "CVD gene" polynucleotide sequence.

Antisense oligonucleotides can be modified without affecting their ability to hybridise to a "CVD gene" polynucleotide. These modifications can be internal or at one or both ends of the antisense molecule. For example, internucleoside phosphate linkages can be modified by adding cholesteryl or diamine moieties with varying numbers of carbon residues between the amino groups and terminal ribose. Modified bases and/or sugars, such as arabinose instead of ribose, or a 3', 5' substituted oligonucleotide in which the 3' hydroxyl group or the 5' phosphate group are substituted, also can be employed in a modified antisense oligonucleotide. These modified oligonucleotides can be prepared by methods well known in the art. See, e.g., Agrawal et al., (54); Uhlmann et al., (52) and Uhlmann et al., (55).

*Ribozymes*

Ribozymes are RNA molecules with catalytic activity. See, e.g., Cech, (56); 1987; Cech, (57) and Couture & Stinchcomb, (58). Ribozymes can be used to inhibit gene function by cleaving an RNA sequence, as is known in the art (e.g., Haseloff et al., U.S. Patent 5,641,673). The mechanism of ribozyme action involves sequence-specific hybridization of the ribozyme molecule to complementary target RNA, followed by endonucleolytic cleavage. Examples include engineered hammerhead motif ribozyme molecules that can specifically and efficiently catalyze endonucleolytic cleavage of specific nucleotide sequences.

The transcribed sequence of a "CVD gene" can be used to generate ribozymes which will specifically bind to mRNA transcribed from a "CVD gene" genomic locus. Methods of designing and constructing ribozymes which can cleave other RNA molecules in trans in a highly sequence specific manner have been developed and described in the art [see Haseloff et al., (59)]. For example, the cleavage activity of ribozymes can be targeted to specific RNAs by engineering a discrete "hybridization" region into the ribozyme. The hybridization region contains a sequence complementary to the target RNA and thus specifically hybridises with the target (see, for example, Gerlach et al., EP No. 0 321201).

Specific ribozyme cleavage sites within a "CVD gene" RNA target can be identified by scanning the target molecule for ribozyme cleavage sites which include the following sequences: GUA, GUU, and GUC. Once identified, short RNA sequences of between 15 and 20 ribonucleotides corresponding to the region of the target RNA containing the cleavage site can be evaluated for secondary structural features which may render the target inoperable. Suitability of candidate "CVD gene" RNA targets also can be evaluated by testing accessibility to hybridization with complementary oligonucleotides using ribonuclease protection assays. Longer complementary sequences can be used to increase the affinity of the hybridization sequence for the target. The hybridising and cleavage regions of the ribozyme can be integrally related such that upon hybridising to the target RNA through the complementary regions, the catalytic region of the ribozyme can cleave the target.

Ribozymes can be introduced into cells as part of a DNA construct. Mechanical methods, such as microinjection, liposome-mediated transfection, electroporation, or calcium phosphate precipitation, can be used to introduce a ribozyme-containing DNA construct into cells in which it is desired to decrease "CVD gene" expression. Alternatively, if it is desired that the cells stably retain the DNA construct, the construct can be supplied on a plasmid and maintained as a separate element or integrated into the genome of the cells, as is known in the art. A ribozyme-encoding DNA construct can include transcriptional regulatory elements, such as a promoter element, an enhancer or UAS element, and a transcriptional terminator signal, for controlling transcription of ribozymes in the cells.

As taught in Haseloff et al., U.S. Pat. No. 5,641,673, ribozymes can be engineered so that ribozyme expression will occur in response to factors which induce expression of a target gene. Ribozymes also can be engineered to provide an additional level of regulation, so that destruction of mRNA occurs only when both a ribozyme and a target gene are induced in the cells.

*Polypeptide detection*

Herein described is a method of determining whether a cell sample obtained from a subject possesses an abnormal amount of marker polypeptide which comprises (a) obtaining a cell sample from the subject, (b) quantitatively determining the amount of the marker polypeptide in the sample so obtained, and (c) comparing the amount of the marker polypeptide so determined with a known standard, so as to thereby determine whether the cell sample obtained from the subject possesses an abnormal amount of the marker polypeptide. Such marker polypeptides may be detected by immunohistochemical assays, dot-blot assays, ELISA and the like.

*Antibodies*

Any type of antibody known in the art can be generated to bind specifically to an epitope of a "CVD gene" polypeptide. An antibody as used herein includes intact immunoglobulin molecules, as well as fragments thereof, such as Fab, $F(ab)_2$, and Fv, which are capable of binding an epitope of a "CVD gene" polypeptide. Typically, at least 6, 8, 10, or 12 contiguous amino acids are required to form an epitope. However, epitopes which involve non-contiguous amino acids may require more, e.g., at least 15, 25, or 50 amino acids.

An antibody which specifically binds to an epitope of a "CVD gene" polypeptide can be used therapeutically, as well as in immunochemical assays, such as Western blots, ELISAs, radioimmunoassays, immunohistochemical assays, immunoprecipitations, or other immunochemical assays known in the art. Various immunoassays can be used to identify antibodies having the desired specificity. Numerous protocols for competitive binding or immunoradiometric assays are well known in the art. Such immunoassays typically involve the measurement of complex formation between an immunogen and an antibody which specifically binds to the immunogen.

Typically, an antibody which specifically binds to a "CVD gene" polypeptide provides a detection signal at least 5-, 10-, or 20-fold higher than a detection signal provided with other proteins when used in an immunochemical assay. Preferably, antibodies which specifically bind to "CVD gene" polypeptides do not detect other proteins in immunochemical assays and can immunoprecipitate a "CVD gene" polypeptide from solution. "CVD gene" polypeptides can be used to immunize a mammal, such as a mouse, rat, rabbit, guinea pig, monkey, or human, to produce polyclonal antibodies. If desired, a "CVD gene" polypeptide can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels (e.g., aluminum hydroxide), and surface active substances (e.g. lysolecithin, pluronic polyols, polyanions, peptides, oil emulsions, keyhole limpet hemocyanin, and dinitrophenol). Among adjuvants used in humans, BCG (bacilli Calmette-Guerin) and Corynebacterium parvum are especially useful.

Monoclonal antibodies which specifically bind to a "CVD gene" polypeptide can be prepared using any technique which provides for the production of antibody molecules by continuous cell lines in culture. These techniques include, but are not limited to, the hybridoma technique, the human B cell hybridoma technique, and the EBV hybridoma technique [Kohler et al., (60); Kozbor et al., (61); Cote et al., (62) and Cole et al., (63)].

In addition, techniques developed for the production of chimeric antibodies, the splicing of mouse antibody genes to human antibody genes to obtain a molecule with appropriate antigen specificity and biological activity, can be used [Morrison et al., (64); Neuberger et al., (65); Takeda et al., (66)]. Monoclonal and other antibodies also can be humanized to prevent a patient from mounting an immune response against the antibody when it is used therapeutically. Such antibodies may be sufficiently similar in sequence to human antibodies to be used directly in therapy or may require alteration of a few key residues. Sequence differences between rodent antibodies and human sequences can be minimized by replacing residues which differ from those in the human sequences by site directed mutagenesis of individual residues or by grating of entire complementarity determining regions. Alternatively, humanized antibodies can be produced using recombinant methods, as described in GB2188638B. Antibodies which specifically bind to a "CVD gene" polypeptide can contain antigen binding sites which are either partially or fully humanized, as disclosed in U.S. Patent 5,565,332.

Alternatively, techniques described for the production of single chain antibodies can be adapted using methods known in the art to produce single chain antibodies which specifically bind to "CVD gene" polypeptides. Antibodies with related specificity, but of distinct idiotypic composition, can be generated by chain shuffling from random combinatorial immunoglobin libraries [Burton, (67)].

Single-chain antibodies also can be constructed using a DNA amplification method, such as PCR, using hybridoma cDNA as a template [Thirion et al., (68)]. Single-chain antibodies can be mono- or bispecific, and can be bivalent or tetravalent. Construction of tetravalent, bispecific single-chain antibodies is taught, for example, in Coloma & Morrison, (69). Construction of bivalent, bispecific single-chain antibodies is taught in Mallender & Voss, (70).

A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology [Verhaar et al., (71); Nicholls et al., (72)].

Antibodies which specifically bind to "CVD gene" polypeptides also can be produced by inducing in vivo production in the lymphocyte population or by screening immunoglobulin libraries or panels of highly specific binding reagents as disclosed in the literature [Orlandi et al., (73) and Winter et al., (74)].

Other types of antibodies can be constructed and used therapeutically. For example, chimeric antibodies can be constructed as disclosed in WO 93/03151. Binding proteins which are derived from immunoglobulins and which are multivalent and multispecific, such as the antibodies described in WO 94/13804, also can be prepared.

Antibodies according to the disclosure can be purified by methods well known in the art. For example, antibodies can be affinity purified by passage over a column to which a "CVD gene" polypeptide is bound. The bound antibodies can then be eluted from the column using a buffer with a high salt concentration.

Immunoassays are commonly used to quantify the levels of proteins in cell samples, and many other immunoassay techniques are known in the art. The disclosure is not limited to a particular assay procedure, and therefore is intended to include both homogeneous and heterogeneous procedures. Exemplary immunoassays which can be conducted according to the disclosure include fluorescence polarisation immunoassay (FPIA), fluorescence immunoassay (FIA), enzyme immunoassay (EIA), nephelometric inhibition immunoassay (NIA), enzyme linked immunosorbent assay (ELISA), and radioimmunoassay (RIA). An indicator moiety, or label group, can be attached to the subject antibodies and is selected so as to meet the needs of various uses of the method which are often dictated by the availability of assay equipment and compatible immunoassay procedures. General techniques to be used in performing the various immunoassays noted above are known to those of ordinary skill in the art.

In another embodiment, the level of the encoded product, i.e., the product encoded by any of the polynucleotide sequences of the SEQ ID NOS:1 to 74 or a sequence complementary thereto, in a biological fluid (e.g., blood or urine) of a patient may be determined as a way of monitoring the level of expression of the marker polynucleotide sequence in cells of that patient. Such a method would include the steps of obtaining a sample of a biological fluid from the patient, contacting the sample (or proteins from the sample) with an antibody specific for a encoded marker polypeptide, and determining the amount of immune complex formation by the antibody, with the amount of immune complex formation being indicative of the level of the marker encoded product in the sample. This determination is particularly instructive when compared to the amount of immune complex formation by the same antibody in a control sample taken from a normal individual or in one or more samples previously or subsequently obtained from the same person.

In another embodiment, the method can be used to determine the amount of marker polypeptide present in a cell, which in turn can be correlated with progression of the disorder, e.g., plaque formation. The level of the marker polypeptide can be used predictively to evaluate whether a sample of cells contains cells which are, or are predisposed towards becoming, plaque associated cells. The observation of marker polypeptide level can be utilized in decisions regarding, e.g., the use of more stringent therapies.

As set out above, one aspect of the present disclosure relates to diagnostic assays for determining, in the context of cells isolated from a patient, if the level of a marker polypeptide is significantly reduced in the sample cells. The term "significantly reduced" refers to a cell phenotype wherein the cell possesses a reduced cellular amount of the marker polypeptide relative to a normal cell of similar tissue origin.
For example, a cell may have less than about 50%, 25%, 10%, or 5% of the marker polypeptide that a normal control cell. In particular, the assay evaluates the level of marker polypeptide in the test cells, and, preferably, compares the measured level with marker polypeptide detected in at least one control cell, e.g., a normal cell and/or a transformed cell of known phenotype.

Of particular importance to the subject disclosure is the ability to quantify the level of marker polypeptide as determined by the number of cells associated with a normal or abnormal marker polypeptide level. The number of cells with a particular marker polypeptide phenotype may then be correlated with patient prognosis. In one embodiment of the disclosure, the marker polypeptide phenotype of the lesion is determined as a percentage of cells in a biopsy which are found to have abnormally high/low levels of the marker polypeptide. Such expression may be detected by immunohistochemical assays, dot-blot assays, ELISA and the like.

*Polypeptide activity*

In one embodiment the present disclosure provides a method for screening potentially therapeutic agents which modulate the activity of one or more "CVD gene" polypeptides, such that if the activity of the polypeptide is increased as a result of the upregulation of the "CVD gene" in a subject having or at risk for cardiovascular disease and arteriosclerosis in particular, the therapeutic substance will decrease the activity of the polypeptide relative to the activity of the some polypeptide in a subject not having or not at risk for cardiovascular diseases or arteriosclerosis in particular but not treated with the therapeutic agent. Likewise, if the activity of the polypeptide as a result of the downregulation of the "CVD gene" is decreased in a subject having or at risk for cardiovascular disease or arteriosclerosis in particular, the therapeutic agent will increase the activity of the polypeptide relative to the activity of the same polypeptide in a subject not having or not at risk for cardiovascular disease or arteriosclerosis in particular, but not treated with the therapeutic agent.

The acitivity of the "CVD gene" polypeptides indicated in Table 4 may be measured by any means known to those of skill in the art, and which are particular for the type of activity performed by the particular polypeptide. Examples of specific assays which may be used to measure the activity of particular polynucleotides are shown below.

a) G protein coupled receptors

In one embodiment, the "CVD gene" polynucleotide may encode a G protein coupled receptor. In one embodiment, the present disclosure provides a method of screening potential modulators (inhibitors or acitivators) of the G protein coupled receptor by measuring changes in the activity of the receptor in the presence of a candidate modulator.

*1. $G_i$-coupled receptors*

Cells (such as CHO cells or primary cells) are stably transfected with the relevant receptor and with an inducible CRE-luciferase construct. Cells are grown in 50% Dulbecco's modified Eagle medium / 50% F12 (DMEM/F12) supplemented with 10% FBS, at 37°C in a humidified atmosphere with 10% $CO_2$ and are routinely split at a ratio of 1:10 every 2 or 3 days. Test cultures are seeded into 384 - well plates at an appropriate density (e.g. 2000 cells / well in 35 μl cell culture medium) in DMEM/F12 with FBS, and are grown for 48 hours (range: ~ 24 - 60 hours, depending on cell line). Growth medium is then exchanged against serum free medium (SFM; e.g. Ultra-CHO), containing 0,1% BSA. Test compounds dissolved in DMSO are diluted in SFM and transferred to the test cultures (maximal final concentration 10 μmolar), followed by addition of forskolin (~ 1 μmolar, final conc.) in SFM + 0,1% BSA 10 minutes later. In case of antagonist screening both, an appropriate concentration of agonist, and forskolin are added. The plates are incubated at 37°C in 10% $CO_2$ for 3 hours. Then the supernatant is removed, cells are lysed with lysis reagent (25 mmolar phosphate-buffer, pH 7,8 , containing 2 mmolar DDT, 10% glycerol and 3% Triton X100). The luciferase reaction is started by addition of substrate-buffer (e.g. luciferase assay reagent, Promega) and luminescence is immediately determined (e.g. Berthold luminometer or Hamamatzu camera system).

*2. $G_s$ -coupled receptors*

Cells (such as CHO cells or primary cells) are stably transfected with the relevant receptor and with an inducible CRE-luciferase construct. Cells are grown in 50% Dulbecco's modified Eagle medium / 50% F12 (DMEM/F12) supplemented with 10% FBS, at 37°C in a humidified atmosphere with 10% $CO_2$ and are routinely split at a ratio of 1:10 every 2 or 3 days. Test cultures are seeded into 384 - well plates at an appropriate density (e.g. 1000 or 2000 cells / well in 35 μl cell culture medium) in DMEM/F12 with FBS, and are grown for 48 hours (range: ~ 24 - 60 hours, depending on cell line). The assay is started by addition of test-compounds in serum free medium (SFM; e.g. Ultra-CHO) containing 0,1% BSA: Test compounds are dissolved in DMSO, diluted in SFM and transferred to the test cultures (maximal final concentration 10 μmolar, DMSO conc. < 0,6 %). In case of antagonist screening an appropriate concentration of agonist is added 5 - 10 minutes later. The plates are incubated at 37°C in 10% $CO_2$ for 3 hours. Then the cells are lysed with 10 μl lysis reagent per well (25 mmolar phosphate-buffer, pH 7,8 , containing 2 mmolar DDT, 10% glycerol and 3% Triton X100) and the luciferase reaction is started by addition of 20 μl substrate-buffer per well (e.g. luciferase assay reagent, Promega). Measurement of luminescence is started immediately (e.g. Berthold luminometer or Hamamatzu camera system).

3. $G_q$ -coupled receptors

Cells (such as CHO cells or primary cells) are stably transfected with the relevant receptor. Cells expressing functional receptor protein are grown in 50% Dulbecco's modified Eagle medium / 50% F12 (DMEM/F12) supplemented with 10% FBS, at 37°C in a humidified atmosphere with 5% $CO_2$ and are routinely split at a cell line dependent ratio every 3 or 4 days. Test cultures are seeded into 384 - well plates at an appropriate density (e.g. 2000 cells / well in 35 μl cell culture medium) in DMEM/F12 with FBS, and are grown for 48 hours (range: ~ 24 - 60 hours, depending on cell line). Growth medium is then exchanged against physiological salt solution (e.g. Tyrode solution). Test compounds dissolved in DMSO are diluted in Tyrode solution containing 0.1% BSA and transferred to the test cultures (maximal final concentration 10 μmolar). After addition of the receptor specific agonist the resulting Gq-mediated intracellular calcium increase is measured using appropriate read-out systems (e.g. calcium-sensitive dyes).

b) Ion channels

Ion channels are integral membrane proteins involved in electrical signaling, transmembrane signal transduction, and electrolyte and solute transport. By forming macromolecular pores through the membrane lipid bilayer, ion channels account for the flow of specific ion species driven by the electrochemical potential gradient for the permeating ion. At the single molecule level, individual channels undergo conformational transitions ("gating") between the 'open' (ion conducting) and 'closed' (non conducting) state. Typical single channel openings last for a few milliseconds and result in elementary transmembrane currents in the range of $10^{-9}$ - $10^{-12}$ Ampere. Channel gating is controlled by various chemical and/or biophysical parameters, such as neurotransmitters and intracellular second messengers ('ligand-gated' channels) or membrane potential ('voltage-gated' channels). Ion channels are functionally characterised by their ion selectivity, gating properties, and regulation by hormones and pharmacological agents. Because of their central role in signaling and transport processes, ion channels present ideal targets for pharmacological therapeutics in various pathophysiological settings.

Screening for compounds interacting with ion channels to either inhibit or promote their activity can be based on (1.) binding and (2.) functional assays in living cells (110).

In one embodiment, the "CVD gene" may encode an ion channel. In one embodiment, the present disclosure provides a method of screening potential activators or inhibitors of channels activity of the "CVD gene" polypeptide. Screening for compounds interaction with ion channels to either inhibit or promote their acitivity can be based on (1.) binding and (2.) functional assays in living cells. See e.g. Hille (110).

1. For ligand-gated channels, e.g. ionotropic neurotransmitter/hormone receptors, assays can be designed detecting binding to the target by competition between the compound and a labeled ligand.
2. Ion channel function can be tested functionally in living cells. Target proteins are either expressed endogenously in appropriate reporter cells or are introduced recombinantly. Channel activity can be monitored by (2.1) concentration changes of the permeating ion (most prominently $Ca^{2+}$ ions), (2.2) by changes in the transmembrane electrical potential gradient, and (2.3) by measuring a cellular response (e.g. expression of a reporter gene, secretion of a neurotransmitter) triggered or modulated by the target activity.

    2.1 Channel activity results in transmembrane ion fluxes. Thus activation of ionic channels can be monitored by the resulting changes in intracellular ion concentrations using luminescent or fluorescent indicators. Because of its wide dynamic range and availability of suitable indicators this applies particularly to changes in intracellular $Ca^{2+}$ ion concentration ($[Ca^{2+}]_i$). $[Ca^{2+}]_i$ can be measured, for example, by aequorin luminescence or fluorescence dye technology (e.g. using Fluo-3, Indo-1, Fura-2). Cellular assays can be designed where either the $Ca^{2+}$ flux through the target channel itself is measured directly or where modulation of the target channel affects membrane potential and thereby the activity of co-expressed voltage-gated $Ca^{2+}$ channels.
    2.2 Ion channel currents result in changes of electrical membrane potential ($V_m$) which can be monitored directly using potentiometric fluorescent probes. These electrically charged indicators (e.g. the anionic oxonol dye $DiBAC_4(3)$) redistribute between extra- and intracellular compartment in response to voltage changes. The equilibrium distribution is governed by the Nernst-equation. Thus changes in membrane potential results in concomitant changes in cellular fluorescence. Again, changes in $V_m$ might be caused directly by the activity of the target ion channel or through amplification and/or prolongation of the signal by channels co-expressed in the same cell.
    2.3 Target channel activity can cause cellular $Ca^{2+}$ entry either directly or through activation of additional $Ca^{2+}$ channel (see 2.1). The resulting intracellular $Ca^{2+}$ signals regulate a variety of cellular responses, e.g. secretion or gene transcription. Therefore modulation of the target channel can be detected by monitoring secretion of a known hormone/transmitter from the target-expressing cell or through expression of a reporter gene (e.g. luciferase) controlled by an $Ca^{2+}$-responsive promoter element (e.g. cyclic AMP/ $Ca^{2+}$-responsive elements; CRE).

c) <u>DNA-binding proteins and transcription factors</u>

In one embodiment, the "CVD gene" may encode a DNA-binding protein or a transcription factor. The activity of such a DNA-binding protein or a transcription factor may be measured, for example, by a promoter assay which measures the ability of the DNA-binding protein or the transcription factor to initiate transcription of a test sequence linked to a particular promoter. In one embodiment, the present disclosure provides a method of screening test compounds for its ability to modulate the acitivity of such a DNA-binding protein or a transcription factor by measuring the changes in the expression of a test gene which is regulated by a promoter which is responsive to the transcription factor.

<u>Promotor assays</u>

A promoter assay was set up with a human hepatocellular carcinoma cell HepG2 that was stably transfected with a luciferase gene under the control of a gene of interest (e.g. thyroid hormone) regulated promoter. The vector 2xIROluc, which was used for transfection, carries a thyroid hormone responsive element (TRE) of two 12 bp inverted palindromes separated by an 8 bp spacer in front of a tk minimal promoter and the luciferase gene. Test cultures were seeded in 96 well plates in serum - free Eagle's Minimal Essential Medium supplemented with glutamine, tricine, sodium pyruvate, non - essential amino acids, insulin, selen, transferrin, and were cultivated in a humidified atmosphere at 10 % $CO_2$ at 37°C. After 48 hours of incubation serial dilutions of test compounds or reference compounds (L-T3, L-T4 e.g.) and costimulator if appropriate (final concentration 1 nM) were added to the cell cultures and incubation was continued for the optimal time (e.g. another 4-72 hours). The cells were then lysed by addition of buffer containing Triton X100 and luciferin and the luminescence of luciferase induced by T3 or other compounds was measured in a luminometer. For each concentration of a test compound replicates of 4 were tested. $EC_{50}$ - values for each test compound were calculated by use of the Graph Pad Prism Scientific software.

*Screening Methods*

The disclosure provides assays for screening test compounds which bind to or modulate the activity of a "CVD gene" polypeptide or a "CVD gene" polynucleotide. A test compound preferably binds to a "CVD gene" polypeptide or polynucleotide. More preferably, a test compound decreases or increases "CVD gene" activity by at least about 10, preferably about 50, more preferably about 75, 90, or 100% relative to the absence of the test compound.

*1. Test Compounds*

Test compounds can be pharmacological agents already known in the art or can be compounds previously unknown to have any pharmacological activity. The compounds can be naturally occurring or designed in the laboratory. They can be isolated from microorganisms, animals, or plants, and can be produced recombinant, or synthesised by chemical methods known in the art. If desired, test compounds can be obtained using any of the numerous combinatorial library methods known in the art, including but not limited to, biological libraries, spatially addressable parallel solid phase or solution phase libraries, synthetic library methods requiring deconvolution, the one-bead one-compound library method, and synthetic library methods using affinity chromatography selection. The biological library approach is limited to polypeptide libraries, while the other four approaches are applicable to polypeptide, non-peptide oligomer, or small molecule libraries of compounds. [For review see Lam, (75)].

Methods for the synthesis of molecular libraries are well known in the art [see, for example, DeWitt et al., (76); Erb et al., (77); Zuckermann et al., (78); Cho et al., (79); Carell et al., (80) and Gallop et al.,(81). Libraries of compounds can be presented in solution [see, e.g., Houghten, (82)], or on beads [Lam, (83)], chips [Fodor, (84)], bacteria or spores (Ladner, U.S. Patent 5,223,409), plasmids [Cull et al., (85)], or phage [Scott & Smith, (86); Devlin, (87); Cwirla et al., (88); Felici, (89)].

*High Throughput Screening*

Test compounds can be screened for the ability to bind to "CVD gene" polypeptides or polynucleotides or to affect "CVD gene" activity or "CVD gene" expression using high throughput screening. Using high throughput screening, many discrete compounds can be tested in parallel so that large numbers of test compounds can be quickly screened. The most widely established techniques utilize 96-well microtiter plates. The wells of the microtiter plates typically require assay volumes that range from 50 to 500 μl. In addition to the plates, many instruments, materials, pipettors, robotics, plate washers, and plate readers are commercially available to fit the 96-well format.

Alternatively, free format assays, or assays that have no physical barrier between samples, can be used. For example, an assay using pigment cells (melanocytes) in a simple homogeneous assay for combinatorial peptide libraries is described by Jayawickreme et al.,(90). The cells are placed under agarose in culture dishes, then beads that carry combinatorial compounds are placed on the surface of the agarose. The combinatorial compounds are partially released the compounds from the beads.

Active compounds can be visualised as dark pigment areas because, as the compounds diffuse locally into the gel matrix, the active compounds cause the cells to change colours.

Another example of a free format assay is described by Chelsky, (91), reported at the First Annual Conference of The Society for Biomolecular Screening in Philadelphia, (1995). Chelsky placed a simple homogenous enzyme assay for carbonic anhydrase inside an agarose gel such that the enzyme in the gel would cause a colour change throughout the gel. Thereafter, beads carrying combinatorial compounds via a photo-linker were placed inside the gel and the compounds were partially released by UV light. Compounds that inhibited the enzyme were observed as local zones of inhibition having less colour change.

Yet another example is described by Salmon et al., (92). In this example, combinatorial libraries were screened for compounds that had cytotoxic effects on cancer cells growing in agar.

Another high throughput screening method is described in Beutel et al., U.S. Patent 5,976,813. In this method, test samples are placed in a porous matrix. One or more assay components are then placed within, on top of, or at the bottom of a matrix such as a gel, a plastic sheet, a filter, or other form of easily manipulated solid support. When samples are introduced to the porous matrix they diffuse sufficiently slowly, such that the assays can be performed without the test samples running together.

*Binding Assays*

For binding assays, the test compound is preferably a small molecule which binds to and occupies, for example, the ATP/GTP binding site of the enzyme or the active site of a "CVD gene" polypeptide, such that normal biological activity is prevented. Examples of such small molecules include, but are not limited to, small peptides or peptide-like molecules.

In binding assays, either the test compound or a "CVD gene" polypeptide can comprise a detectable label, such as a fluorescent, radioisotopic, chemiluminescent, or enzymatic label, such as horseradish peroxidase, alkaline phosphatase, or luciferase. Detection of a test compound which is bound to a "CVD gene" polypeptide can then be accomplished, for example, by direct counting of radioemmission, by scintillation counting, or by determining conversion of an appropriate substrate to a detectable product.

Alternatively, binding of a test compound to a "CVD gene" polypeptide can be determined without labeling either of the interactants. For example, a microphysiometer can be used to detect binding of a test compound with a "CVD gene" polypeptide. A microphysiometer (e.g., CytosensorJ) is an analytical instrument that measure the rate at which a cell acidifies its environment using a light-addressable potentiometric sensor (LAPS). Changes in this acidification rate can be used as an indicator of the interaction between a test compound and a "CVD gene" polypeptide [McComell et al., (93)].

Determining the ability of a test compound to bind to a "CVD gene" polypeptide also can be accomplished using a technology such as real-time Bimolecular Interaction Analysis (BIA) [Sjolander & Urbaniczky, (94), and Szabo et al., (95)]. BIA is a technology for studying biospecific interactions in real time, without labeling any of the interactants (e.g., BIAcore™). Changes in the optical phenomenon surface plasmon resonance (SPR) can be used as an indication of real-time reactions between biological molecules.

A "CVD gene" polypeptide can be used as a "bait protein" in a two-hybrid assay or three-hybrid assay (see, e.g., U.S. Patent 5,283,317; Zervos et al., (96); Madura et al., (97); Bartel et al., (98); Iwabuchi et al., (99) and Brent WO 94/10300), to identify other proteins which bind to or interact with the "CVD gene" polypeptide and modulate its activity.

The two-hybrid system is based on the modular nature of most transcription factors, which consist of separable DNA-binding and activation domains. Briefly, the assay utilizes two different DNA constructs. For example, in one construct, polynucleotide encoding a "CVD gene" polypeptide can be fused to a polynucleotide encoding the DNA binding domain of a known transcription factor (e.g., GAL4). In the other construct a DNA sequence that encodes an unidentified protein ("prey" or "sample") can be fused to a polynucleotide that codes for the activation domain of the known transcription factor. If the "bait" and the "prey" proteins are able to interact in vivo to form an protein- dependent complex, the DNA-binding and activation domains of the transcription factor are brought into close proximity. This proximity allows transcription of a reporter gene (e.g., LacZ), which is operably linked to a transcriptional regulatory site responsive to the transcription factor. Expression of the reporter gene can be detected, and cell colonies containing the functional transcription factor can be isolated and used to obtain the DNA sequence encoding' the protein which interacts with the "CVD gene" polypeptide.

It may be desirable to immobilise either a "CVD gene" polypeptide (or polynucleotide) or the test compound to facilitate separation of bound from unbound forms of one or both of the interactants, as well as to accommodate automation of the assay. Thus, either a "CVD gene" polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not limited to, latex, polystyrene, or glass beads). Any method known in the art can be used to attach a "CVD gene" polypeptide (or polynucleotide) or test compound to a solid support, including use of covalent and non-covalent linkages, passive absorption, or pairs of binding moieties attached respectively to the polypeptide (or polynucleotide) or test compound and the solid support. Test compounds are preferably bound to the solid support in an array, so that the location of individual test compounds can be tracked. Binding of a test compound to a "CVD gene" polypeptide (or polynucleotide) can be accomplished in any vessel suitable for containing the reactants. Examples of such vessels include microtiter plates, test tubes, and microcentrifuge tubes.

In one embodiment, a "CVD gene" polypeptide is a fusion protein comprising a domain that allows the "CVD gene" polypeptide to be bound to a solid support. For example, glutathione S-transferase fusion proteins can be adsorbed onto glutathione sepharose beads (Sigma Chemical, St. Louis, Mo.) or glutathione derivatized microtiter plates, which are then combined with the test compound or the test compound and the nonadsorbed "CVD gene" polypeptide; the mixture is then incubated under conditions conducive to complex formation (e.g., at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined. Other techniques for immobilising proteins or polynucleotides on a solid support also can be used in the screening assays of the disclosure. For example, either a "CVD gene" polypeptide (or polynucleotide) or a test compound can be immobilized utilizing conjugation of biotin and streptavidin. Biotinylated "CVD gene" polypeptides (or polynucleotides) or test compounds can be prepared from biotin NHS (N-hydroxysuccinimide) using techniques well known in the art (e.g., biotinylation kit, Pierce Chemicals, Rockford, Ill.) and immobilized in the wells of streptavidin-coated 96 well plates (Pierce Chemical). Alternatively, antibodies which specifically bind to a "CVD gene" polypeptide, polynucleotide, or a test compound, but which do not interfere with a desired binding site, such as the ATP/GTP binding site or the active site of the "CVD gene" polypeptide, can be derivatised to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to a "CVD gene" polypeptide or test compound, enzyme-linked assays which rely on detecting an activity of a "CVD gene" polypeptide, and SDS gel electrophoresis under non-reducing conditions.

Screening for test compounds which bind to a "CVD gene" polypeptide or polynucleotide also can be carried out in an intact cell. Any cell which comprises a "CVD gene" polypeptide or polynucleotide can be used in a cell-based assay system. A "CVD gene" polynucleotide can be naturally occurring in the cell or can be introduced using techniques such as those described above. Binding of the test compound to a "CVD gene" polypeptide or polynucleotide is determined as described above.

*Modulation of Gene Expression*

In another embodiment, test compounds which increase or decrease "CVD gene" expression are identified. A "CVD gene" polynucleotide is contacted with a test compound, and the expression of an RNA or polypeptide product of the "CVD gene" polynucleotide is determined. The level of expression of appropriate mRNA or polypeptide in the presence of the test compound is compared to the level of expression of mRNA or polypeptide in the absence of the test compound. The test compound can then be identified as a modulator of expression based on this comparison. For example, when expression of RNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of "CVD gene" mRNA or polypeptide expression in the cells can be determined by methods well known in the art for detecting mRNA or polypeptide. Either qualitative or quantitative methods can be used. The presence of polypeptide products of a "CVD gene" polynucleotide can be determined, for example, using a variety of techniques known in the art, including immunochemical methods such as radioimmunoassay, Western blotting, and immunohistochemistry. Alternatively, polypeptide synthesis can be determined in vivo, in a cell culture, or in an in vitro translation system by detecting incorporation of labelled amino acids into a "CVD gene" polypeptide.

Such screening can be carried out either in a cell-free assay system or in an intact cell. Any cell which expresses a "CVD gene" polynucleotide can be used in a cell-based assay system. A "CVD gene" polynucleotide can be naturally occurring in the cell or can be introduced using techniques such as those described above. Either a primary culture or an established cell line, such as CHO or human embryonic kidney 293 cells, can be used.

*Therapeutic Indications and Methods*

Therapies for treatment of CVD primarily relied upon effective drugs for lowering cholesterol and high blood pressure. In particular, the statins lower levels of arterio-genic lipoproteins and dramatically decrease clinical events and mortality from arteriosclerosis. Nevertheless, heart disease and stroke remain by far the most common causes of death in westernised societies, and new weapons, particularly agents that block disease at the level of the vessel wall or that raise anti-arteriogenic HDL, are needed. The advent of genomics-driven molecular target identification has opened up the possibility of identifying new cardiovascular disease-specific targets for therapeutic intervention that will provide safer, more effective treatments for CVD patients and arteriosclerosis patients in particular. Thus, newly discovered CVD-associated genes and their products can be tested for their role(s) in disease and used as tools to discover and develop innovative therapies. The identification of the ABC transporter presents exciting new opportunities for treatment of low HDL levels. Preliminary studies in animals suggest that it may be possible not only to block the development of arteriosclerosis but also to achieve significant regression. The most critical clinical aspect of arteriosclerosis is plaque rupture and thrombosis.

Genes playing important roles in any of the physiological processes outlined above can be characterized as cardiovascular disease targets. Genes or gene fragments identified through genomics can readily be expressed in one or more heterologous expression systems to produce functional recombinant proteins. These proteins are characterised in vitro for their biochemical properties and then used as tools in high-throughput molecular screening programs to identify chemical modulators of their biochemical activities. Modulators of target protein activity can be identified in this manner and subsequently tested in cellular and in vivo disease models for therapeutic activity. Optimisation of lead compounds with iterative testing in biological models and detailed pharmacokinetic and toxicological analyses form the basis for drug development and subsequent testing in humans.

The activities of the "CVD genes" provide therapeutic targets for cardiovascular disease and arteriosclerosis in particular.

This disclosure further pertains to the use of novel agents identified by the screening assays described above. Accordingly, the present disclosure uses a test compound identified as described herein in an appropriate animal model.

For example, an agent identified as described herein (e.g., a modulating agent, an antisense nucleic acid molecule, a specific antibody, ribozyme, or a human "CVD gene" polypeptide binding molecule) can be used in an animal model to determine the efficacy, toxicity, or side effects of treatment with such an agent. Alternatively, an agent identified as described herein can be used in an animal model to determine the mechanism of action of such an agent. Furthermore, this disclosure pertains to uses of novel agents identified by the above described screening assays for treatments as described herein.

A reagent which affects human "CVD gene" activity can be administered to a human cell, either in vitro or in vivo, to reduce or increase human "CVD gene" activity. The reagent preferably binds to an expression product of a human "CVD gene". If the expression product is a protein, the reagent is preferably an antibody. For treatment of human cells ex vivo, an antibody can be added to a preparation of stem cells which have been removed from the body. The cells can then be replaced in the same or another human body, with or without clonal propagation, as is known in the art.

In one embodiment, the reagent is delivered using a liposome. Preferably, the liposome is stable in the animal into which it has been administered for at least about 30 minutes, more preferably for at least about 1 hour, and even more preferably for at least about 24 hours. A liposome comprises a lipid composition that is capable of targeting a reagent, particularly a polynucleotide, to a particular site in an animal, such as a human. Preferably, the lipid composition of the liposome is capable of targeting to a specific organ of an animal, such as the lung, liver, spleen, heart brain, lymph nodes, and skin.

A liposome useful in the present disclosure comprises a lipid composition that is capable of fusing with the plasma membrane of the targeted cell to deliver its contents to the cell. Preferably, the transfection efficiency of a liposome is about 0.5 $\mu$g of DNA per 16 nmol of liposome delivered to about $10^6$ cells, more preferably about 1.0 $\mu$g of DNA per 16 nmol of liposome delivered to about $10^6$ cells, and even more preferably about 2.0 $\mu$g of DNA per 16 nmol of liposome delivered to about $10^6$ cells. Preferably, a liposome is between about 100 and 500 nm, more preferably between about 150 and 450 nm, and even more preferably between about 200 and 400 nm in diameter.

Suitable liposomes for use in the present disclosure include those liposomes usually used in, for example, gene delivery methods known to those of skill in the art. More preferred liposomes include liposomes having a polycationic lipid composition and/or liposomes having a cholesterol backbone conjugated to polyethylene glycol. Optionally, a liposome comprises a compound capable of targeting the liposome to a particular cell type, such as a cell-specific ligand exposed on the outer surface of the liposome.

Complexing a liposome with a reagent such as an antisense oligonucleotide or ribozyme can be achieved using methods which are standard in the art (see, for example, U.S. Patent 5,705,151). Preferably, from about 0.1 $\mu$g to about 10 $\mu$g of polynucleotide is combined with about 8 nmol of liposomes, more preferably from about 0.5 $\mu$g to about 5 $\mu$g of polynucleotides are combined with about 8 nmol liposomes, and even more preferably about 1.0 $\mu$g of polynucleotides is combined with about 8 nmol liposomes.

In another embodiment, antibodies can be delivered to specific tissues in vivo using receptor-mediated targeted delivery. Receptor-mediated DNA delivery techniques are taught in, for example, Findeis et al.,(100); Chiou et al., (101); Wu & Wu, (102); Wu et al., (103); Zenke et al., (104); Wu et al., (105).

*Determination of a Therapeutically Effective Dose*

The determination of a therapeutically effective dose is well within the capability of those skilled in the art. A therapeutically effective dose refers to that amount of active ingredient which increases or decreases human "CVD gene" activity relative to the human "CVD gene" activity which occurs in the absence of the therapeutically effective dose.

For any compound, the therapeutically effective dose can be estimated initially either in cell culture assays or in animal models, usually mice, rabbits, dogs, or pigs. The animal model also can be used to determine the appropriate concentration range and route of administration. Such information can then be used to determine useful doses and routes for administration in humans.

Therapeutic efficacy and toxicity, e.g., $ED_{50}$ (the dose therapeutically effective in 50% of the population) and $LD_{50}$ (the dose lethal to 50% of the population), can be determined by standard pharmaceutical procedures in cell cultures or experimental animals. The dose ratio of toxic to therapeutic effects is the therapeutic index, and it can be expressed as the ratio, $LD_{50}/ED_{50}$.

Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the $ED_{50}$ with little or no toxicity. The dosage varies within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active ingredient or to maintain the desired effect. Factors which can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

If the reagent is a single-chain antibody, polynucleotides encoding the antibody can be constructed and introduced into a cell either ex vivo or in vivo using well-established techniques including, but not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, a gene gun, and DEAE- or calcium phosphate-mediated transfection.

Effective in vivo dosages of an antibody are in the range of about 5 μg to about 50 μg/kg, about 50 μg to about 5 mg/kg, about 100 μg to about 500 μg/kg of patient body weight, and about 200 to about 250 μg/kg of patient body weight. For administration of polynucleotides encoding single-chain antibodies, effective in vivo dosages are in the range of about 100 ng to about 200 ng, 500 ng to about 50 mg, about 1 μg to about 2 mg, about 5 μg to about 500 μg, and about 20 μg to about 100 μg of DNA.

If the expression product is mRNA, the reagent is preferably an antisense oligonucleotide or a ribozyme. Polynucleotides which express antisense oligonucleotides or ribozymes can be introduced into cells by a variety of methods, as described above.

Preferably, a reagent reduces expression of a "CVD gene" gene or the activity of a "CVD gene" polypeptide by at least about 10, preferably about 50, more preferably about 75, 90, or 100% relative to the absence of the reagent. The effectiveness of the mechanism chosen to decrease the level of expression of a "CVD gene" gene or the activity of a "CVD gene" polypeptide can be assessed using methods well known in the art, such as hybridization of nucleotide probes to "CVD gene" -specific mRNA, quantitative RT-PCR, immunologic detection of a "CVD gene" polypeptide, or measurement of "CVD gene" activity.

In any of the embodiments described above, any of the pharmaceutical compositions of the disclosure can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects.

Any of the therapeutic methods described above can be applied to any subject in need of such therapy, including, for example, birds and mammals such as dogs, cats, cows, pigs, sheep, goats, horses, rabbits, monkeys, and most preferably, humans.

The above disclosure generally describes the present invention. A more complete understanding can be obtained by reference to the following specific examples which are provided for purposes of illustration only and are not intended to limit the scope of the invention.

*Pharmaceutical Compositions*

The disclosure also provides pharmaceutical compositions which can be administered to a patient to achieve a therapeutic effect. Pharmaceutical compositions of the disclosure can comprise, for example, a "CVD gene" polypeptide, "CVD gene" polynucleotide, ribozymes or antisense oligonucleotides, antibodies which specifically bind to a "CVD gene" polypeptide, or mimetics, agonists, antagonists, or inhibitors of a "CVD gene" polypeptide activity. The compositions can be administered alone or in combination with at least one other agent, such as stabilizing compound, which can be administered in any sterile, biocompatible pharmaceutical carrier, including, but not limited to, saline, buffered saline, dextrose, and water. The compositions can be administered to a patient alone, or in combination with other agents, drugs or hormones.

In addition to the active ingredients, these pharmaceutical compositions can contain suitable pharmaceutically acceptable carriers comprising excipients and auxiliaries which facilitate processing of the active compounds into preparations which can be used pharmaceutically. Pharmaceutical compositions of the disclosure can be administered by any number of routes including, but not limited to, oral, intravenous, intramuscular, intraarterial, intramedullary, intrathecal, intraventricular, transdermal, subcutaneous, intraperitoneal, intranasal, parenteral, topical, sublingual, or rectal means. Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combination of active compounds with solid excipient, optionally grinding a resulting mixture, and processing the mixture of granules, after adding suitable auxiliaries, if desired, to obtain tablets or dragee cores. suitable excipients are carbohydrate or protein fillers, such as sugars, including lactose, sucrose, mannitol, or sorbitol; starch from com, wheat, rice, potato, or other plants; cellulose, such as methyl cellulose, hydroxypropylmethylcellulose, or sodium carboxymethylcellulose; gums including arabic and tragacanth; and proteins such as gelatin and collagen. If desired, disintegrating or solubilizing agents can be added, such as the cross-linked polyvinyl pyrrolidone, agar, alginic acid, or a salt thereof, such as sodium alginate.

Dragee cores can be used in conjunction with suitable coatings, such as concentrated sugar solutions, which also can contain gum arabic, talc, polyvinylpyrrolidone, carbopol gel, polyethylene glycol, and/or titanium dioxide, lacquer solutions, and suitable organic solvents or solvent mixtures. Dyestuffs or pigments can be added to the tablets or dragee coatings for product identification or to characterize the quantity of active compound, i.e., dosage.

Pharmaceutical preparations which can be used orally include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds can be dissolved or suspended in suitable liquids, such as fatty oils, liquid, or liquid polyethylene glycol with or without stabilizers.

Pharmaceutical formulations suitable for parenteral administration can be formulated in aqueous solutions, preferably in physiologically compatible buffers such as Hanks' solution, Ringer's solution, or physiologically buffered saline. Aqueous injection suspensions can contain substances which increase the viscosity of the suspension, such as sodium carboxymethyl cellulose, sorbitol, or dextran. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Non-lipid polycationic amino polymers also can be used for delivery. Optionally, the suspension also can contain suitable stabilizers or agents which increase the solubility of the compounds to allow for the preparation of highly concentrated solutions. For topical or nasal administration, penetrants appropriate to the particular barrier to be permeated are used in the formulation. Such penetrants are generally known in the art.

The pharmaceutical compositions of the present disclosure can be manufactured in a manner that is known in the art, e.g., by means of conventional mixing, dissolving, granulating, dragee making, levigating, emulsifying, encapsulating, entrapping, or lyophilizing processes. The pharmaceutical composition can be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. In other cases, the preferred preparation can be a lyophilized powder which can contain any or all of the following: 150 mM histidine, 0.1%2% sucrose, and 27% mannitol, at a pH range of 4.5 to 5.5, that is combined with buffer prior to use.

Further details on techniques for formulation and administration can be found in the latest edition of REMINGTON'S PHARMACEUTICAL SCIENCES (106). After pharmaceutical compositions have been prepared, they can be placed in an appropriate container and labeled for treatment of an indicated condition. Such labeling would include amount, frequency, and method of administration.

*Material and Methods*

One strategy for identifying genes that are involved in cardiovascular disease is to detect genes that are expressed differentially under conditions associated with the disease versus non-disease conditions. The sub-sections below describe a number of experimental systems which may be used to detect such differentially expressed genes. In general, these experimental systems include at least one experimental condition in which subjects or samples are treated in a manner associated with cardiovascular disease, in addition to at least one experimental control condition lacking such disease associated treatment. Differentially expressed genes are detected, as described below, by comparing the pattern of gene expression between the experimental and control conditions.

Once a particular gene has been identified through the use of one such experiment, its expression pattern may be further characterized by studying its expression in a different experiment and the findings may be validated by an independent technique. Such use of multiple experiments may be useful in distinguishing the roles and relative importance of particular genes in cardiovascular disease. A combined approach, comparing gene expression pattern in cells derived from CVD patients to those of *in vitro* cell culture models can give substantial hints on the pathways involved in development and/or progression of CVD.

Among the experiments which may be utilized for the identification of differentially expressed genes involved in arteriosclerosis, for example, are experiments designed to analyze those genes which are involved in foam cell formation. Such experiments may serve to identify genes involved in the differentiation of this cell type, or their uptake of enzymatic modified LDL.

Within such an experiment, human blood is drawn and peripheral monocytes are isolated by methods routinely practiced in the art. These human monocytes can then be used immediately or cultured *in vitro,* using methods routinely practiced in the art, for 4 to 6 days where they develop more macrophage-like characteristics such as the up-regulation of scavenger receptors. These cells are then treated for various lengths of time with agents thought to be involved in foam cell formation. These agents include but are not limited to enzymatic modified LDL and HDL. Control monocytes that are untreated or directly treated with native HDL are grown in parallel. At a certain time after addition of the test agents, the cells are harvested and analyzed for differential expression as described in detail below in the following section.
In order to identify differentially expressed genes, RNA, either total or mRNA, were isolated from one or more blood samples of the subjects utilized in experiments such as those described earlier. Total RNA samples were obtained from peripheral white blood cells (PWBC) of experimental subjects and from corresponding PWBC of control subjects.
Below are methods described for the identification of genes which are involved in cardiovascular disease, including but not limited to arteriosclerosis, ischemia/reperfusion, hypertension, restenosis, and arterial inflammation. Such genes represent genes which are differentially expressed in cardiovascular disease conditions relative to their expression in normal, or non-cardiovascular disease conditions or upon experimental manipulation based on clinical observations. Such differentially expressed genes represent "target" and/or "marker" genes. Methods for the further characterization of such differentially expressed genes, and for their identification as target and/or marker genes, are presented below.
Thus, a differentially expressed gene may have its expression activated or completely inactivated in normal versus cardiovascular disease conditions (e.g., treated with enzymatic modified LDL versus untreated; mimicking of cholesterol efflux due to HDL treatment), or under control versus experimental conditions. Such a qualitatively regulated gene will exhibit an expression pattern within a given tissue or cell type which is detectable in either control or cardiovascular disease subjects, but is not detectable in both.
Alternatively, a differentially expressed gene may have its expression modulated, i.e., quantitatively increased or decreased, in normal versus cardiovascular disease states, or under control versus experimental conditions. The degree to which expression differs in normal versus cardiovascular disease or control versus experimental states need only be large enough to be visualised via standard characterisation techniques, such as, for example, the differential display technique described below. Other such standard characterisation techniques by which expression differences may be visualised include but are not limited to quantitative RT-PCR and Northern analyses, which are well known to those of skill in the art.

*Physiological and biochemical significance of the results:*

The Tables 1 and 2 show a summary of the genes, identified by the differential expression approach with DNA array technology and TaqMan analysis, which show an excellent correlation of gene expression levels.
All 74 nucleotide sequences were previously described in the literature but have not been previously recognised as being differentially expressed in CVD patients versus non-CVD patients.
Of these 74 nucleotide sequences, several are coding for transporter or channel proteins (e.g., voltage dependent anion channel1 (VDAC1), calmodulin like). These transporters can play an important role in the import and export of cholesterol or triglycerides, one of the key steps in the generation of lipid vessels in macrophages, and if dysregulated, one step in direction of arteriosclerosis. The VDAC protein is thought to form the major pathway for movement of adenine nucleotides through the outer membrane and to be the mitochondrial binding site for hexokinase and glycerol kinase. And may also be involved in the signalling and initiation of an apoptotic cascade in the cell involving the BCL2 protein. The BCL2 family of proteins (see Bfl1), whose members may be anti-apoptotic or pro-apoptotic, regulates cell death by controlling this mitochondrial membrane permeability during apoptosis. Since macrophages transformed by high LDL load into foam cell are driven into apoptosis, within the so called fatty streaks, the upregulation of these genes can function as an indicative marker for arteriosclerosis. Some of the genes identified, belong to a signalling pathway system (e.g., epimorphin, lipoxin or G-CSFR). All represent receptors, mediating cell to cell interactions. Also isomerases and oxidoreductases show a tightly regulated expression pattern upon incubation with eLDL (e.g., IPP isomerase, 5-lipoxygenase, further see Table 4). These enzymes are involved in the degradation of fatty acids or in the synthesis of cholesterol. Some of the genes listed in the Tables 1 and 2 are involved in the phagosomal degradation of fatty acids (e.g., legumain or cathepsin L), which reflects the predispositional changes in the CVD patients analyzed. These genes were not directly affected by a mutation (in case of Tangier's Disease; ABCA1 transporter) but were differentially regulated upon comparison with the same gene in normal individual under the same eLDL conditions. So one can assume that these genes are good candidates for a diagnostic test or therapeutic interaction.

*EXAMPLE 1*

*Probe selection for the differential gene expression analysis*

*Proband and Patient Selection*

For Expression analysis monocytes from healthy donors with Apo E3/E3 and E4/E4 genotype, as well as from Tangier's disease, familial hypercholestermia, Niemann Pick Type C and Lp(a) patients were isolated as described below. Probands and patients were identified and selected due to their clinical appearance and further genetic confirmation of the represented genotypes. For each group two individual were selected and expression profiles generated as described below. In total we have analysed RNA from 9 male and 9 female donors.

*Monocyle Isolation and Cultivation*

Human peripheral blood leukocytes from healthy normolipidemic volunteers were isolated by leukapheresis in a cell separator and subsequent counterflow centrifugation as described by Mueller et al; (107). To guarantee viability of the cells with minimal activation, isolated monocytes were cultured on Ultra Low Attachment Surfaces (Costar) in macrophage serum-free medium (Life Technologies) supplemented with monocyte colony-stimulating factor (M-CSF, 50 ng/ml) for up to 6 days. Cells were detached by rinsing the Costar Ultra Low Attachment Surfaces with PBS. For uptake experiments, 4-day cultured monocytes ($10^6$ cells per milliliter) were incubated with modified LDL for 2 hours at 37°C in 1 ml macrophage media containing 0.5% BSA.

*Preparation of LDL*

Human native LDL (1.006 mg/mL,density,1.063 mg/ml) was isolated from the plasma of healthy blood donors by sequential preparative ultracentrifugation in KBr gradients, followed by extensive dialysis and filter sterilization. Protein concentrations were determined by use of Lowry's method.

*Chemical and Enzymatic Modification of LDL*

Enzyme treatment was conducted with trypsin (6.6 mg/ml, Sigma) and cholesterol esterase (40 mg/ml, Roche Biochemica) for 6 to 8 hours at 37°C. Subsequently, the pH of the solution was adjusted to 5.5 by addition of MES buffer, pH 5.0. Finally, neuraminidase (79 mU/ml, Behring) and magnesium ascorbate solution (30 mg/ml) were added for 14 hours at 37°C. The absence of oxidation products in E-LDL was verified by the determination of thiobarbituric acid-reactive substances to quantify lipid peroxidation product. 12 Modified lipoproteins were stored at 4°C and used within a week. During LDL preparation and subsequent modification, general precautions were taken to avoid LPS contamination. The latter was excluded by *Limulus* endotoxin assay (Kinetic-QCL, BioWhittaker).

*EXAMPLE 2*

*Differential DNA expression profiling*

Expression profiling was carried out using the Affymetrix Array Technology. With minor modifications, the sample preparation protocol followed the Affymetrix GeneChip Expression Analysis Manual (Santa Clara, CA). Total RNA extraction an isolation from PWBC can be performed by using TRIzol (Life Technologies, Rockville, MD) and Oligotex mRNA Midi kit (Qiagen, Hilden, Germany), and an ethanol precipitation step should be carried out to bring the concentration to 1 mg/ml. Using 5-10 mg of mRNA to create double stranded cDNA by the SuperScript system (Life Technologies). First strand cDNA synthesis was primed with a T7-(dT24) oligonucleotide. The cDNA can be extracted with phenol/chloroform and precipitated with ethanol to a final concentration of 1mg /ml. From the generated cDNA, cRNA can be synthesised using Enzo's (Enzo Diagnostics Inc., Farmingdale, NY) *in vitro* Transcription, Kit. Within the same step the cRNA can be labelled with biotin nucleotides Bio-11-CTP and Bio-16-UTP (Enzo Diagnostics Inc., Farmingdale, NY) After labelling and cleanup (Qiagen, Hilden (Germany) the cRNA then should be fragmented in an appropriated fragmentation buffer (e.g., 40 mM Tris-Acetate, pH 8.1, 100 mM KOAc, 30 mM MgOAc, for 35 minutes at 94 °C). As per the Affymetrix protocol, fragmented cRNA should be hybridised on the HG_U95 array set (five chips A-E), comprising app. 13.000 probed transcripts each, for 24 hours at 60 rpm in a 45 °C hybridization oven. After Hybridization step the chip surfaces have to be washed and stained with streptavidin phycoerythrin (SAPE; Molecular Probes, Eugene, OR) in Affymetrix fluidics stations. To amplify staining, a second labeling step can be introduced, which is recommended but not compulsive. Here one should add SAPE solution twice with an antistreptavidin biotinylated antibody.

Hybridization to the probe arrays may be detected by fluorometric scanning (Hewlett Packard Gene Array Scanner; Hewlett Packard Corporation, Palo Alto, CA). After hybridization and scanning, the microarray images can be analyzed for quality control, looking for major chip defects or abnormalities in hybridization signal. Therefor either Affymetrix GeneChip MAS 4.0 Software or other microarray image analysis software can be utilized. Primary data analysis should be carried out by MAS Software.

In case of the genes analyses in one embodiment of this invention the primary data have been analysed by further bioinformatic tools and additional filter criteria. The bioinformatic analysis is described in detail below.

74 genes were identified to be at least 1.5 fold, differentially expressed in patients with cardiovascular disease in comparison to patients without cardiovascular disease. Due to the diversity of the group of cardiovascular disease patients, an inter group comparison was performed, to identify those genes and pathways that are involved in either differentiation and/or expressional reaction of macrophages under lipid stress (high eLDL environment). The differential expression of these 74 genes may only be observed in one group, (e.g. Tangier disease patients), due to the inherited mutation in a specific gene in these patients and the resulting abnormal lipid trafficking. The specific regulation of these genes within one group compared to the others indicates their role in lipid trafficking and development of arteriosclerosis.

To confirm the results obtained by the array analysis with a second independent experimental approach, these 74 genes were analyzed by real-time quantitative PCR (TaqMan), using the PRISM 7700 Sequence Detection System of PE Applied Biosystems (Perkin Elmer, Foster City, CA, USA) with in the same cohort. Within this technique a fluorogenic probe, consisting of an oligonucleotide labelled with both a fluorescent reporter dye and a quencher dye, is included in a typical PCR. Amplification of the probe-specific product causes cleavage of the probe, generating an increase in reporter fluorescence. Primers and probes were selected using the Primer Express software and localized mostly in the 3' region of the coding sequence or in the 3' untranslated region (see Table 3 for primer- and probe- sequences) according to the positions of the probe sequence used for the construction of the Affymetrix HG_U95A-E chip. All primer pairs were checked for specificity by conventional PCR reactions. To standardise the amount of sample RNA, GAPDH was selected as a reference, since it was not differentially regulated in the samples analyzed. TaqMan validation experiments were performed showing that the efficiencies of the target and the control amplifications are approximately equal which is a prerequisite for the relative quantitation of gene expression by the comparative $\Delta\Delta C_T$ method, known to those with skills in the art.

*EXAMPLE 3*

*Data analysis*

According to Affymetrix measurement technique (Affymetrix GeneChip Expression Analysis Manual, Santa Clara, CA) a single gene expression measurement on one chip yields the average difference value and the absolute call. Each chip contains 16-20 oligonucleotide probe pairs per gene or cDNA clone. These probe pairs include perfectly matched sets and mismatched sets, both of which are necessary for the calculation of the average difference, or expression value, a measure of the intensity difference for each probe pair, calculated by subtracting the intensity of the mismatch from the intensity of the perfect match. This takes into consideration variability in hybridization among probe pairs and other hybridization artefacts that could affect the fluorescence intensities. The average difference is a numeric value supposed to represent the expression value of that gene. The absolute call can take the values 'A' (absent), 'M' (marginal), or 'P' (present) and denotes the quality of a single hybridization. We used both the quantitative information given by the average difference and the qualitative information given by the absolute call to identify the genes which are differentially expressed in biological samples from individuals with cardiovascular disease versus biological samples from the normal population. With other algorithms than the Affymetrix one we have obtained different numerical values representing the same expression values and expression differences upon comparison.

The differential expression E in one of the cardiovascular disease groups compared to the normal population is calculated as follows. Given n average difference values $d_1, d_2, ..., d_n$ in the cardiovascular disease population and m average difference values $c_1, c_2, ..., c_m$ in the population of normal individuals, it is computed by the equation:

$$E \equiv \exp\left(\frac{1}{m}\sum_{i=1}^{m}\ln(c_i) - \frac{1}{n}\sum_{i=1}^{n}\ln(d_i)\right)$$

If $d_j<50$ or $c_i<50$ for one or more values of i and j, these particular values $c_i$ and/or $d_j$ are set to an "artificial" expression value of 50. These particular computation of E allows for a correct comparison to TaqMan results.

A gene is called up-regulated in cardiovascular disease versus normal if $E \geq 1.5$ and if the number of absolute calls equal to 'P' in the cardiovascular disease population is greater than n/2.

A gene is called down-regulated in cardiovascular disease versus normal if E≤1.5 and if the number of absolute calls equal to 'P' in the normal population is greater than m/2.

The final list of differentially regulated genes consists of all up-regulated and all down-regulated genes in biological samples from individuals with cardiovascular disease versus biological samples from the normal population. Those genes on this list which are interesting for a pharmaceutical application were finally validated by TaqMan. If a good correlation between the expression values/behavior of a transcript could be observed with both techniques, such a gene is listed in Table 1 or 2.

**TABLE 1**

| **Genes which are up-regulated in diseased vs. Normal individuals** | | | | | | | |
|---|---|---|---|---|---|---|---|
| **DNA Sequence** | **Protein Sequence** | **GB Acc** | **UNIGENE** | **Locus Link** | **Gene_Name** | **Short Description of the Gene** | **Minimal Fold Change** |
| SEQ ID NO. 37 | SEQ ID NO.111 | L32976 | Hs.89449 | 4296 | MLK-3 MLK3 | HUMMLK3A protein kinase (MLK-3) mRNA, complete cds | 7,0 |
| SEQ ID NO. 6 | SEQ ID NO.80 | X13988 | Hs.17384 | 4621 | MYH3 | mRNA for embryonic myosin heavy chain | 5,0 |
| SEQ ID NO. 70 | SEQ ID NO.144 | M98479 | Hs.8265 | 7052 | TGM2 | tissue transglutaminase | 5,0 |
| SEQ ID NO. 60 | Seq_ ID134 | U82812 | Hs.522 | 922 | CD5L | scavenger receptor cysteine rich Sp alpha mRNA, complete cds | 4,0 |
| SEQ ID NO. 72 | SEQ ID NO.146 | X03663 | Hs. 174142 | 1436 | CSF1R | c-fms | 4,0 |
| SEQ ID NO. 65 | SEQ ID NO.139 | AF001383 | Hs. 193163 | 274 | SH3P9 AMPHL | amphiphysin II mRNA, complete cds | 3,5 |
| SEQ ID NO. 9 | SEQ ID NO.83 | AB001325 | Hs. 234642 | 360 | AQP3 | AQP3 gene for aquaporine 3 (water channel), partail cds | 3,0 |
| SEQ ID NO. 11 | SEQ ID NO.85 | U61538 | Hs.8531 | 11261 | CHP | calcium-binding protein chp mRNA, complete cds | 3,0 |
| SEQ ID NO. 19 | SEQ ID NO.93 | Y08374 | Hs.75184 | 1116 | HTG; HTGS_PHAS | gene encoding cartilage GP-39 protein, exon 1 and 2 (and joined CDS) | 3,0 |
| SEQ ID NO. 33 | SEQ ID NO.107 | D17793 | Hs.78183 | 8644 | ets variant gene 6 | mRNA for KIAA0119 gene, complete cds | 3,0 |
| SEQ ID NO. 42 | SEQ ID NO.116 | D14582 | Hs.99865 | 2054 | STX2C STX2B STX2A EPIM | mRNA for epimorphin | 3,0 |

(continued)

| Genes which are up-regulated in diseased vs. Normal individuals | | | | | | | |
|---|---|---|---|---|---|---|---|
| **DNA Sequence** | **Protein Sequence** | **GB Acc** | **UNIGENE** | **Locus Link** | **Gene_Name** | **Short Description of the Gene** | **Minimal Fold Change** |
| SEQ ID NO. 46 | SEQ ID NO.120 | AJ000414 | Hs.73999 | 9322 | TRIP10 | mRNA for Cdc42-interacting protein 4 (CIP4) | 3,0 |
| SEQ ID NO. 48 | SEQ ID NO.122 | U48734 | Hs. 182485 | 81 | ACTN4 | non-muscle alpha-actinin mRNA, | 3,0 |
| SEQ ID NO. 63 | SEQ ID NO.137 | H24861 | Hs. 117167 | | | y142e11.r1 cDNA, 5 end | 3,0 |
| SEQ ID NO.74 | SEQ ID NO.148 | AF057557 | Hs.58831 | 9214 | TOSO | anti-Fas-induced apoptosis mRNA | 3,0 |
| SEQ ID NO. 13 | SEQ ID NO.87 | U22662 | Hs.81336 | 10062 | LXRA LXR-A | nuclear orphan receptor LXR-alpha mRNA, complete | 2,5 |
| SEQ ID NO. 55 | SEQ ID NO.129 | L26232 | Hs.2837 | 5360 | PLTP | phospholipid transfer protein mRNA, complete cds | 2,5 |
| SEQ ID NO. 7 | SEQ ID NO.81 | AB026833 | Hs. 241551 | 9635 | CLCA2 | mRNA for chloride channel protein, complete cds | 2,0 |
| SEQ ID NO. 17 | SEQ ID NO.91 | J04430 | Hs.1211 | 54 | ACP5 | HUMACP5 tartrate-resistant acid phosphatase type 5 | 2,0 |
| SEQ ID NO. 24 | SEQ ID NO.98 | X 17025 | Hs.7638 | 3422 | IDI1 | log of yeast IPP isomerase | 2,0 |
| SEQ ID NO. 26 | SEQ ID NO.100 | J03600 | Hs.89499 | 240 | LOX5 ALOX5 | HUMLOX5 lipoxygenase mRNA, complete cds | 2,0 |
| SEQ ID NO. 35 | SEQ ID NO.109 | AF004709 | Hs. 178695 | 5603 | SAPK4 PRKM13 | stress-activated protein kinase 4 mRNA, | 2,0 |
| SEQ ID NO. 43 | SEQ ID NO.117 | M59818 | Hs.2175 | 1441 | G-CSFR-1 CSF3R | granulocyte colony-stimulating factor receptor (G-CSFR-1) mRNA, complete cds | 2,0 |

(continued)

| Genes which are up-regulated in diseased vs. Normal individuals | | | | | | | |
|---|---|---|---|---|---|---|---|
| **DNA Sequence** | **Protein Sequence** | **GB Acc** | **UNIGENE** | **Locus Link** | **Gene_Name** | **Short Description of the Gene** | **Minimal Fold Change** |
| SEQ ID NO. 59 | SEQ ID NO.133 | AL034562 | Hs. 156114 | 8194 | 1199_at PTPNS1 | dJ684O24.2 (prodynorphin (Beta-Neoendorphin-Dynorphin precursor, Proenkephalin B | 2,0 |
| SEQ ID NO. 64 | SEQ ID NO.138 | AF089750 | Hs. 179986 | 10211 | FLOT1 | flotillin-1 mRNA, complete cds | 2,0 |
| SEQ ID NO. 45 | SEQ ID NO.119 | AB006780 | Hs.621 | 3958 | GALBP MAC2 | mRNA for galectin-3, complete cds | 1,8 |
| SEQ ID NO. 53 | SEQ ID NO.127 | U30930 | Hs.15854 | 7368 | UGT8 | UDP-Galactose ceramide galactosyl transferase (CGT) mRNA | 1,8 |

**TABLE 2**

| Genes which are down-regulated in diseased vs. Normal individuals | | | | | | | |
|---|---|---|---|---|---|---|---|
| **DNA Sequence** | **Protein Sequence** | **GB_Acc** | **UNIGENE** | **Locus Link** | **Gene_Name** | **Short Description of the gene** | **Minimal Fold Change** |
| SEQ ID NO. 50 | SEQ ID NO. 124 | U03644 | Hs.89421 | 9541 | | recepin mRNA, complete cds | - 6,0 |
| SEQ ID NO. 61 | SEQ ID NO.135 | X82460 | Hs.77348 | 3248 | HPGD | hydroxyprostaglandin dehydrogenase 15-(NAD) | - 6,0 |
| SEQ ID NO. 31 | SEQ ID NO.105 | Y13647 | Hs.119597 | 6319 | SCD | mRNA for stearoyl-CoA desaturase | - 5,0 |
| SEQ ID NO. 56 | SEQ ID NO.130 | U41387 | Hs.169531 | 9188 | | Gu protein mRNA, partial cds | - 5,0 |
| SEQ ID NO. 66 | SEQ ID NO.140 | U69274 | Hs.2861 | 27107 | | zinc finger protein mRNA, complete cds | - 5,0 |
| SEQ ID NO. 73 | SEQ ID NO.147 | S70154 | Hs.278544 | 39 | ACAT2 | cytosolic acetoacetyl-coenzyme A thiolase, CT | - 5,0 |
| SEQ ID NO. 28 | SEQ ID NO.102 | U78294 | Hs.111256 | | ALOX15B | 15S-lipoxygenase mRNA, complete cds | - 4,0 |
| SEQ ID NO. 44 | SEQ ID NO.118 | U40572 | Hs.172278 | 6645 | SNT2B2 | beta2-syntrophin (SNT B2) mRNA, complete cds | - 4,0 |
| SEQ ID NO. 47 | SEQ ID NO.121 | Z11793 | Hs.3314 | 6414 | SEPP1 | mRNA for selenoprotein P | - 3,5 |
| SEQ ID NO. 4 | SEQ ID NO.78 | AF046873 | Hs.125878 | 8224 | SYN3 | synapsin IIIa mRNA, complete cds | - 3,0 |
| SEQ ID NO. 22 | SEQ ID NO.96 | U27467 | Hs.227817 | 597 | Bfl-1 U2746 BCL2A1 | HSU27467 Bcl-2 related (Bfl-1) mRNA, complete cds | - 3,0 |
| SEQ ID NO. 25 | SEQ ID NO.99 | AF061741 | Hs.17144 | 9249 | SDRI | retinal short-chain dehydrogenase reductase retSDR1 mRNA, complete cds | - 3,0 |
| SEQ ID NO. 27 | SEQ ID NO.101 | AB016247 | Hs.28831 | 6309 | C5D SC5DL | mRNA for sterol-C5-desaturase, complete cds | - 3,0 |
| SEQ ID NO. 49 | SEQ ID NO.123 | X68733 | Hs.234726 | 12 | ACT AACT | gene for alphal-antichymotrypsin, exon 1 | - 3,0 |

(continued)

| Genes which are down-regulated in diseased vs. Normal individuals | | | | | | | |
|---|---|---|---|---|---|---|---|
| **DNA Sequence** | **Protein Sequence** | **GB_Acc** | **UNIGENE** | **Locus Link** | **Gene_Name** | **Short Description of the gene** | **Minimal Fold Change** |
| SEQ ID NO. 51 | SEQ ID NO.125 | Y09443 | Hs.2258 | 8540 | AGPS | mRNA for alkyl-dihydroxyacetonephosphate synthase precursor | - 3,0 |
| SEQ ID NO. 58 | SEQ ID NO.132 | AB000220 | Hs.171921 | 10512 | IFN-alpha 6 SEMA3C | AB000220 mRNA for semaphorin E, complete cds | - 3,0 |
| SEQ ID NO. 2 | SEQ ID NO.76 | U69108 | Hs.29736 | 7188 | U6910 TRAF5 | HSU69108 TNF receptor associated factor 5 mRNA, partial cds | - 2,5 |
| SEQ ID NO. 3 | SEQ ID NO.77 | L02320 | Hs.25613 | 5962 | RDX | radixin mRNA, complete cds | - 2,5 |
| SEQ ID NO. 12 | SEQ ID NO.86 | AF026166 | Hs.6456 | 10576 | RBP-MS/type 4 CCT2 | chaperonin-containing TCP-1 beta subunit log mRNA, complete cds | - 2,5 |
| SEQ ID NO. 14 | SEQ ID NO.88 | U22431 | Hs.19754 | 3091 | HIF-1 alpha U2243 HIF1A | HSU22431 hypoxia-inducible factor 1 alpha (HIF-1 alpha) mRNA, complete cds | - 2,5 |
| SEQ ID NO. 18 | SEQ ID NO.92 | AB020645 | Hs.239189 | 2744 | KIAA0838 Hs.172839 | mRNA for KIAA0838 protein, complete cds | - 2,5 |
| SEQ ID NO. 34 | Seq_ID108 | Y12735 | Hs.3818 | 8444 | DYRK3 | mRNA for protein kinase, Dyrk3 | - 2,5 |
| SEQ ID NO. 36 | SEQ ID NO.110 | X60188 | Hs.861 | 5595 | ERK1 ERK1 PRKM3 | HSERK1 ERK1 mRNA for protein serine threonine kinase | - 2,5 |
| SEQ ID NO. 54 | SEQ ID NO.128 | Z35102 | Hs.8724 | 11329 | protein kinase C inhibitor NDR | HSPROKINX mRNA for Ndr protein kinase | - 2,5 |
| SEQ ID NO. 69 | SEQ ID NO.143 | M80482 | Hs.17414 | 5046 | PACE4 | subtilisin-like protein (PACE4) | - 2,5 |
| SEQ ID NO. 5 | SEQ ID NO.79 | M59830 | Hs.27442 | 3304 | | MHC class III HSP70-2 gene (HLA), complete cds | - 2,2 |

(continued)

| Genes which are down-regulated in diseased vs. Normal individuals | | | | | | | |
|---|---|---|---|---|---|---|---|
| **DNA Sequence** | **Protein Sequence** | **GB_Acc** | **UNIGENE** | **Locus Link** | **Gene_Name** | **Short Description of the gene** | **Minimal Fold Change** |
| SEQ ID NO. 1 | SEQ ID NO.75 | X58965 | Hs.275163 | 4831 | nm23-H2 | RNA for nm23-H2 gene | - 2,0 |
| SEQ ID NO. 8 | SEQ ID NO.82 | AB008775 | Hs.14624 | 366 | AQP9 | AQP9 mRNA for aquaporin 9, complete cds | - 2,0 |
| SEQ ID NO. 15 | SEQ ID NO.89 | U51903 | Hs.78993 | 10788 | IQGAP2 U5190 | HSU51903 RasGAP-related protein (IQGAP2) mRNA, complete cds | - 2,0 |
| SEQ ID NO. 16 | SEQ ID NO.90 | AF056490 | Hs.78746 | 5151 | rac protein kinase-alpha PDE8A | cAMP-specific phosphodiesterase 8A (PDE8A) mRNA, partial cds | - 2,0 |
| SEQ ID NO. 20 | SEQ ID NO.94 | D55696 | Hs.1869 | 5641 | PRSC1 | mRNA for cysteine protease, complete cds | - 2,0 |
| SEQ ID NO. 21 | SEQ ID NO.95 | X12451 | Hs.7856 | 1514 | CTSL | mRNA for pro-cathepsin L (major excreted protein MEP) | - 2,0 |
| SEQ ID NO. 23 | SEQ ID NO.97 | S81221 | Hs.93199 | 4047 | LSS | lanosterol synthase [, fetal liver, mRNA Partial, 2637 nt] | - 2,0 |
| SEQ ID NO. 29 | SEQ ID NO.103 | AL050118 | Hs.184641 | 9415 | DKFZp586C201 FADSD6 | mRNA; cDNA DKFZp586C201 (from clone DKFZp586C201) | - 2,0 |
| SEQ ID NO. 30 | SEQ ID NO.104 | AF034544 | Hs.1186 | 1717 | DHCR7 | delta7-sterol reductase mRNA, complete cds | - 2,0 |
| SEQ ID NO. 32 | SEQ ID NO.106 | X77094 | Hs.196352 | 4689 | NCF4 | mRNA for p40phox | - 2,0 |
| SEQ ID NO. 38 | SEQ ID NO.112 | X13916 | Hs.89137 | 4035 | LRP1 | mRNA for LDL-receptor related protein | - 2,0 |
| SEQ ID NO. 39 | SEQ ID NO.113 | W60864 | Hs.9963 | 7305 | TYROBP | zd27g05.s1 cDNA,3 end | - 2,0 |
| SEQ ID NO. 40 | SEQ ID NO.114 | X74039 | Hs.179657 | 5329 | PLAUR | mRNA for urokinase plasminogen activator receptor | - 2,0 |

(continued)

| Genes which are down-regulated in diseased vs. Normal individuals | | | | | | | |
|---|---|---|---|---|---|---|---|
| **DNA Sequence** | **Protein Sequence** | **GB_Acc** | **UNIGENE** | **Locus Link** | **Gene_Name** | **Short Description of the gene** | **Minimal Fold Change** |
| SEQ ID NO. 57 | SEQ ID NO.131 | Y08136 | Hs.42945 | 10924 | ASM3A ASML3a | mRNA for ASM-like phosphodiesterase 3a | - 2,0 |
| SEQ ID NO. 67 | SEQ ID NO.141 | U49392 | Hs.76364 | 199 | AIF1 | allograft inflammatory factor-1 (AIF-1) mRNA, complete cds | - 2,0 |
| SEQ ID NO. 68 | -------- | AF035284 | Hs.12214 | 3992 | | clone 23716 mRNA sequence | - 2,0 |
| SEQ ID NO. 41 | SEQ ID NO.115 | M84562 | Hs.99855 | 2358 | FPRL1 | formyl peptide receptor-like receptor (FPRL1) mRNA, complete cds | - 1,8 |
| SEQ ID NO. 52 | SEQ ID NO.126 | M58597 | Hs.2173 | 2526 | FUT4 | ELAM-1 ligand fucosyltransferase (ELFT) mRNA, complete cds | - 1,8 |
| SEQ ID NO. 62 | SEQ ID NO.136 | S60099 | Hs.64797 | 334 | APPH | APPH amyloid precursor protein log [, placenta, mRNA, 3727 nt] | - 1,8 |
| SEQ ID NO. 71 | SEQ ID NO.145 | D38048 | Hs.11865 | 5695 | PSMB/ | proteasomal subunit Z | - 1,8 |
| SEQ ID NO. 10 | SEQ ID NO.84 | L06132 | Hs.149155 | 7416 | VDAC1 | voltage-dependent anion channel isoform 1 (VDAC) mRNA, complete cds | - 1,6 |

**TABLE 3**

| CVD-gene | 5' primer | 3' primer |
|---|---|---|
| L26232 | CTGCGCAGGTTCCGAATCT | GGGCCTGTAATGGGATCAGA |
| AF061741 | ACAGCACCTGGGCACACAC | GTCCTGCTCACCCAGCAGA |
| U41387 | TCCTTCCCTGAAATAAATACCTAAGG | GCAGGTGGCTGAGGAAACC |
| L00352 | TCTGGATCGTTTGACGGGA | TCTCTCCGGACATCAGTGCA |
| AF656490 | CGCCTAATGCACTTCACAGGT | AAATAGAGTAGACTTTTGGAAATTGAATTATAAA |
| U22662 | TCTGTTTTCTTGGCCGGATG | TGCCCTTCTCAGTCTGTTCCA |
| J036600 | AAACACCATAGGGACCCATTCTAC | GATTTGCTGTTGCTGCTTGG |
| Y09443 | ATCCTTGCTAATGGAGGGAGC | CCTTTAGCCATTGCTTCCGTA |
| Y12735 | GCTAACTTAATGTCAGAAACCAATGG | ATACACATATGCATCTCTGGGCA |
| Y08136 | GAATCTAAAGGGAGAGTCCATCTGG | TCCGGCTGCAAATCTTCAAT |
| A026833 | TTATTGACCTGGAAGCTGTAAAAGTAGA | TAGCCTGGCCCTGATCAAAG |
| AF004709 | TGTCGGTTGGGAGAAACTAGCT | CTGCAGGCGATTCTCCAGAT |
| X74039 | CCATGTGGAGATAGAGCCCC | GGCTACATGTCCAAGGTGGC |
| AB016247 | TGATGTTTGAAGTTACAACCTGTAATTTT | GGAGAAGAGGAGGAATAAGATTTTAGAA |
| U03644 | AAGGGAGACAAGGAAACGGG | CTCTATACAAGTCTGTGCCATGGC |
| U78294 | TCCAAGCCTCAAAGTGCCC | CCACGGCTGTAAACGCAAA |
| AB000220 | GTATCTCTGCACCGCTGCC | CATCCCAGGCGCAATAAGG |
| AB000220 | AAAAGCACAAGCGAACCCC | CACAACCCCACGCTGCA |
| D17793 | GCTGGAGGTGCTGGTAGCTG | TGTTGGTGCCTGCCCTTC |
| AL050118 | ATTGCCTTTCAGCTCTAGATCCC | CAGTTCAACACCGTGCACG |
| AF0374544 | GGGCACTGCTGAGGAATGAT | CCGAACAACATCTGGCATTTT |
| AB016247 | ACCAGCAAGGCTGACCTGTC | CTCAAATACATCAAGCACAGCCTTA |
| AF089747 | CAGAAAGAGGAATAAAATGATTAAGTGC | TCTCTGCCTCTGATTCAGGGTT |
| K02268 | GACCCGGAAACAGCGTATCA | ATCCGACCTCTGACCCTGG |
| AF019562 | AGCAACACCTCCCTATTCTGTTATTT | ACACGTCATGAAAAGGTCTAGGATT |

(continued)

| CVD-gene | 5' primer | 3' primer |
|---|---|---|
| U82812 | CCTGCCCTCCTGCCAAAG | GGGCTCAAATGCTGTAGGTTGT |
| AL034562 | GCCACGATCACAATCTGCAG | AATTCCCAGCCCCACTCAG |
| J04430 | GCCGCTGACTTCTTTCACAAG | CCTCCAAGGACAATCCAGCA |
| M58597 | CTCGGTGCTGGGAGGGT | GGTGTCCTTTGTCAAGAGCATG |
| S81221 | CCCTGACTAACAGCCTCAGCA | TTGGCCTCGTCTTCACTTGG |
| AF046873 | GCCTTTAAGTGACTAAGGAACAACATAG | TTGAGAGGCACAATTGAAGTATTCA |
| U51903 | CTGACCCTCGGCCTCTACTTT | TGGTGTGGTCTTCTCTGAGTGAA |
| S60099 | GTCAAAAAGCCCAGAATTCCC | TTCACATTTAATTTCTGCTGTTCTGA |
| AB008775 | CTATGGCCGAGGGTGAAGAC | GTAGGAGGTGGGCACGTAGC |
| AJ000414 | CCCTAATGCCAGTTCCAGCTT | AGCCCCAAATCAGGGACAC |
| M84562 | CAGGATTTCCACTGGACCTTG | ACCCAAATTCCGGTTCCAC |
| AB001325 | AAGAACGCCCTGGAGTCCTAC | TCGGCCTCGCTGATCTTG |
| M59830 | CATAGGAAAATGATCAAACAAGCAA | GGATGAGCGTAAGGCAGTAAGAA |
| H24861 | GACTTTTTTAGACAGATCTTCATGACCTG | ACCCTGCAGACCTTTTGGTG |
| L06132 | AGCCTCATAGCTGAAGTTGCCT | TCAATGGACATGCTCAGGGA |
| A089750 | CACAGCACAACCGGTCCC | CCCTCGCATGGCCCA |
| U48734 | AAACCCCACCCTAGTTTCCCT | CGCGAAGTGACAGCTTTGAC |
| X66188 | TTCACTGAGAGGGTCCCATGA | GAACCACACATTTTTCGGGAG |
| AF001383 | TCCCATGCTTGAGCTTCCA | CTACCTGTCTCCATGGCTTGC |
| AB020645 | GCCATCTTGGCCAGGATTAAA | AAGGAACCTGAGGGACCCC |
| Y13647 | CTGTCTGCTTGGAGTTTACATATCAAA | AACCCATTGGCCAGACAAAA |
| U69274 | CTCTGTTTCGGGAGCGGAG | AGCTCGTCGTCTGTGGTGGT |
| D14582 | CTCAGCCGGGAAGATTTCC | CATTGATGATCCGGTCCCC |
| U61538 | TCATGCAGACCGGGTACAAC | GAAAGGTGGGTAGCCGATGAG |
| Z35102 | GATTTTGGTGGTCCCGAGG | TGCAACACAAATACAATCGGC |

(continued)

| CVD-gene | 5' primer | 3' primer |
|---|---|---|
| U40572 | GCTGAGCTGGAATTTGCCA | CGCAAACCAACTGTTTTCACC |
| L02320 | CCTGTGATCCTTTGATGGCTTT | CATCAGTAAACTCTCCTAGGGAGCTAC |
| AL137751 | CAACTTGAGGAGAAAACCTTTACAATT | GGTGACAATGAAACTCTGTCTCAGA |
| U69108 | CTTCCCCTGGAAGCTGCAC | ACCGTCAGCTCATGGCATC |
| X77094 | CATGCCCTTATGAGACTACTAATGAAATT | GGTTCAGTGCCAAAACTCTCTACA |
| U30930 | TCCGCGCTAAGACTCGAGAC | AATATACATTTTGCATATCTTCTGTCCTG |
| X13988 | ACCCTGCCCTGCTCTGC | GCTAGCCCAGATACCTGTTTGAG |
| Y08374 | CCACAACACACAGATTTGAGCTC | GATGCCTCACTATCCCCACAG |
| M80927 | GGGTCTATTTGCTCCGCTAAAA | GAAAGAAATGGAAGACGCTGAAC |
| AF077200 | TCCCACTGTCAATCAAAGACCTAA | GCTGGGTTATCCTGTAAGTAGCAATC |
| XM016472 | CTCTCTGGATGCCTTCCTGC | TTCTGGCTTCACTGGATCCC |
| L32976 | CAGGACCTTCTTCACAAGATGACTT | CCCACCCTAATTTGTAGTTTTTCAG |
| D55696 | ACAGGCCGAGTGATGCTTG | CAGAAGTCCCCACGAATGGT |
| X58965 | GCGTGTGGACAACATCATCAA | GATCGACAGCACGTGGGAAT |
| AF026166 | GAGGGTCCATAAGCCCATGAC | GCAGATCGCCTGGGAGG |
| M59818 | TTTAATGAGAATAGAAACGTAAACTATGACC | AAGTAATATTGTTTTGAGAGATAAGTAAAGGAAA |
| Z11793 | AGGTGTCCAGTGGCTCCG | TTAGGATGGCAGATCTCTTGCC |
| U49392 | GAATTGGAACATTATTACAGCAGCC | CACTAGATTTGCATCCTTTTACACG |
| U22431 | GACTATCTGCAGTGCGTCCTACAG | AACATTTTGTAGCACTCTGGACGTT |
| U27467 | CCCTCCAACCCACAGCC | AATATGCCTAGAGTAGATGCTGCTGAA |
| AF035284 | TGTGCATTGCAGGATGGTG | TGGCAACATCCGGCATAAC |
| X17025 | GACACTTTGCCAAGGCTCTCC | CATCCTTTCCCGCCTACCTC |
| X13916 | CAAAGACCGGAGAAACCATTG | ATCACCGTCCACCAGCTCA |
| X12451 | TTGCAGAGGCGCTGCTG | TGTACACCTGCTTCAAGATTCCA |
| X82460 | GCTCCCCTGTTTGACGACA | TTAGGGTTAACATTGTACTTGCTTCATT |

(continued)

| CVD-gene | 5' primer | 3' primer |
|---|---|---|
| M80482 | AGCTGGAGAGAACCCAGATGTTGTTTATTGAATC | GCTCCCCTGTTTGACGACA |
| M55153 | TTAGCAGGACTCATGCCCG | ATCCAACTATGCCATGCTTTGA |
| M98479 | AAGGACTTGGAGAGAATCATGCTGTTGCA | TCCAGGGCCCCCCA |
| X82460 | TGCACAGCAGCCGGTTTATTGTGC | TTAGCAGGACTCATGCCCG |
| D38048 | GCCAGCCACCCACTGATGCCA | CCAAACAATGGACACTTCCTGA |
| X03663 | CCTCTGGGAGATCTTCTCACTTGGGCTG | AGAGCGACGTCTGGTCCTATG |
| S70154 | AATAGGACCAATTCCAGCCATAAAGCAAGCT | AGTGGGTGTGGAGCCTTCC |

**TABLE 4**

| GB_Acc | Detailed Description | Enzyme Class | Biological_Function | subcellular |
|---|---|---|---|---|
| X58965 | | Metabolism | | |
| U69108 | Member of a family of proteins that interact with the cytoplasmic domain of oligomerized TNF receptors, binds the lymphotoxin beta receptor (LTBR) | activator of NF-kappa B | | Cytoplasmic |
| L02320 | Radixin, member of a family of proteins that link the cytoskeleton and the plasma membrane, thereby regulating cell adhesion and cortical morphogenesis | Anchor Protein Proteasome | | Cytoskeletal |
| AF046873 | Synapsin III, a synaptic vesicle protein, a member of a family of proteins that bind ATP and may regulate neurotransmitter release | ATPase | Hydrolase | Cytoplasmic |
| M59830 | Member of the heat shock HSP70 family of molecular chaperones that are involved in protein folding, translocation, and assembly into complexes, inducible by heat shock | ATPase | Chaperones | |
| X13988 | Skeletal muscle myosin heavy chain, member of a family of motor proteins that provide the force for muscle contraction, expressed only during embryogenesis | ATPase | Hydrolase | Cytoplasmic |
| AB026833 | Calcium-sensitive chloride channel, contains five transmembrane domains and displays an outward rectifying conductance of anions, expressed in the lung, trachea, and mammary gland, may be involved in the pathogenesis of cystic fibrosis | Channel [passive transporter] | Transporter | Plasma membrane |
| AB008775 | Aquaporin 9, a water and urea channel expressed predominantly in leukocytes | Channel [passive transporter] | Transporter | Plasma membrane |

(continued)

| GB_Acc | Detailed Description | Enzyme Class | Biological_Function | subcellular |
|---|---|---|---|---|
| AB001325 | Aquaporin 3, a water channel, member of the MIP family of proteins involved in transport of water, glycerol and other small molecules | Channel [passive transporter] | Transporter | Plasma membrane |
| L06132 | Voltage-dependent anion channel 1 (mitochondrial porin channel), a voltage-gated pore of the outer mitochondrial membrane, mediates apoptotic signals from Bcl2 and related proteins that lead to release of cytochrome c | Channel [passive transporter] | Transporter | Cytoplasmic |
| U61538 | Calcium-binding protein with similarity to calcineurin B and calmodulin, binds the sodium-potassium exchanger NHE1, inhibits GTPase-stimulation of NHE1 activity when overexpressed | Channel [passive transporter] | Transporter | |
| AF026166 | Beta subunit of the cytosolic chaperonin containing TCP-1 (CCT), assists in the proper folding of tubulin, actin and centractin, may also be required for the proper folding of Cyclin E | Chaperones | | Cytoskeletal |
| U22662 | Member of the nuclear receptor superfamily, forms a heterodimer with the retinoid receptor that makes it responsive to retinoic acid | DNA-binding protein | | Nuclear |
| U22431 | Basic helix-loop-helix transcription factor that contains a PAS domain, heterodimerizes with the Ah receptor nuclear translocator (ARNT) and mediates transcriptional responses to hypoxia and dioxin-signaling | DNA-binding protein | Transcription factor | Nuclear |

(continued)

| GB_Acc | Detailed Description | Enzyme Class | Biological_Function | subcellular |
|---|---|---|---|---|
| U51903 | Protein with GTPase activating domain, multiple calmodulin binding domains, and actin binding domain, inhibits GTPase activity of Cdc42 and Rac1, which are members of the ras family of GTP binding proteins | GTPase activating protein | Inhibitor or repressor | Cytoskeletal |
| AF056490 | cAMP-specific phosphodiesterase, expressed in testis, ovaries, small intestine, and colon | Hydrolase | | |
| J04430 | Tartrate-resistant acid phosphatase (purple acid phosphatase, type-5 acid phosphatase), a binuclear, iron-containing phosphatase expressed in monocytes and induced upon monocyte differentiation | Hydrolase | Other phosphatase | |
| AB020645 | Protein with strong similarity to rat Gls, mitochondrial glutaminase, which contains ankyrin (Ank) repeats that may mediate protein-protein interactions | Hydrolase | | Mitochondrial |
| Y08374 | Cartilage glycoprotein-39, has similarity to chitinases, expressed in rheumatoid arthritis cartilage and synovial cells | Hydrolase | Structural protein | Extracellular matrix (cuticle and basement membrane) |
| D55696 | Legumain, a cysteine endoprotease that hydrolyzes asparaginyl bonds | Hydrolase | Protease ( | |
| X12451 | Cathepsin L, a lysosomal cysteine (thiol) protease that cleaves collagen and elastin and is highly expressed in transformed cells | Hydrolase | Protease | Cytoplasmic |

(continued)

| GB_Acc | Detailed Description | Enzyme Class | Biological_Function | subcellular |
|---|---|---|---|---|
| U27467 | Hemopoietic-specific early-response BCL2-related protein, expression is induced by phorbol ester and inflammatory cytokines, may protect cells during inflammation, required for mitochondrial viability and function | Inhibitor or repressor | | |
| S81221 | Lanosterol synthase ((S)-2,3-epoxysqualene mutase), catalyzes the cyclization of (S)-2,3-oxidosqualene to form lanosterol during sterol biosynthesis | Isomerase | | |
| X17025 | Isopentenyl diphosphate:dimethylallyl diphosphate isomerase (IPP isomerase), catalyses the interconversion of isopentenyl diphosphate and dimethylallyl diphosphate in isoprenoid synthesis | Isomerase | | Cytoplasmic |
| AF061741 | Short-chain dehydrogenase/reductase, reduces all-trans-retinal during bleached visual pigment regeneration, may function in non-photoreceptor retinol metabolism | Oxidoreductase | | Plasma membrane |
| J03600 | 5-lipoxygenase, catalyzes the first two steps in the synthesis of leukotrienes, which are involved in allergic and inflammatory responses | Oxidoreductase | | Cytoplasmic |
| AB016247 | Protein with similarity to S. cerevisiae Erg3p, which is a sterol-C5-desaturase | Oxidoreductase | | |
| U78294 | Arachidonate 15-lipoxygenase, converts arachidonic acid to 15S-hydroperoxyeicosatetraenoic acid, poorly metabolizes linoleic acid | Oxidoreductase | | |

(continued)

| GB_Acc | Detailed Description | Enzyme Class | Biological_Function | subcellular |
|---|---|---|---|---|
| AL050118 | Delta-6 desaturase, desaturates 18:2(n-6) and 18:3(n-3) to form 20:4(n-6) (arachidonic acid) and 22:6(n-3) (docosahexaenoic acid) | Oxidoreductase | | Plasma membrane |
| AF034544 | 7-dehydrocholesterol reductase, removes the C7-8 double bond in 7-dehydrocholesterol; mutations in the corresponding gene cause Smith-Lemli-Opitz syndrome | Oxidoreductase | | Cytoplasmic |
| Y13647 | Stearoyl-coenzyme A desaturase, functions in the synthesis of unsaturated fatty acids; upregulated in esophageal and colonic carcinomas and hepatocellular adenoma | Oxidoreductase | | |
| X77094 | Component of the cytosolic nicotinamide adenine dinucleotide phosphate (NADPH)-oxidase complex, which is required for the oxidative burst, expressed only in hematopoietic cells | Oxidoreductase | | Cytoplasmic |
| D17793 | 3 alpha-hydroxysteroid dehydrogenase, oxidizes xenobiotic alicyclic alcohols and 3alpha- or 17beta-hydroxy-5beta-androstanes, activated on exposure to all-trans-retinoic acid, may function in control of cell growth and differentiation | OxidoreductaseTransformatio n related | Transformation related | |
| Y12735 | Dual-specificity protein kinase | Protein kinase | Transferase | |
| AF004709 | MAP kinase that is activated by stress and proinflammatory cytokines, phosphorylated by MKK6 (PRKMK6) | Protein kinase | Transferaseserine | |
| X60188 | MAP kinase that is activated in response to growth factors | Protein kinase | Transferase | Nuclear |

(continued)

| GB_Acc | Detailed Description | Enzyme Class | Biological_Function | subcellular |
|---|---|---|---|---|
| L32976 | Member of the mixed-lineage kinase family, has SH3 and leucine zipper domains | Protein kinase | Transferase | |
| X13916 | Low density lipoprotein receptor-related protein (alpha-2-macroglobulin receptor), binds to apoE containing lipoproteins and mediates chylomicron remnant clearance from the plasma | Receptor (protein translocation) | | Plasma membrane |
| W60864 | Protein with an immunoreceptor tyrosine-based activation motif (ITAM), associates with membrane glycoproteins of the killer-cell inhibitory receptor (KIR) family and activates NK cells | Receptor (signalling) | | Plasma membrane |
| X74039 | Urokinase-type plasminogen activator receptor, a member of a superfamily that includes CD59, murine Ly-6, and elapid snake venom toxins, functions in pericellular plasminogen activation | Receptor (signalling) | | Plasma membrane |
| M84562 | Lipoxin A4 receptor, a G protein-coupled receptor with similarity to the formyl peptide receptor (FPR1) that binds lipoxins and signals through an inhibitory G-protein to mobilize calcium, stimulates chemotaxis and cell adhesion | Receptor (signalling) | | Plasma membrane |
| D14582 | Epimorphin, a signaling protein, has strong similarity to murine Epim and rat Rn.10623; Epim functions in epithelial-mesenchymal interactions, Rn. 10623 is involved in docking synaptic vesicles with the presynaptic plasma membrane | Receptor (signalling) | | Plasma membrane |

(continued)

| GB_Acc | Detailed Description | Enzyme Class | Biological_Function | subcellular |
|---|---|---|---|---|
| M59818 | Granulocyte colony-stimulating factor receptor; mutation of the corresponding gene causes severe congenital neutropenia and is also associated with acute myeloid leukemia | Receptor (signalling) | | Plasma membrane |
| U40572 | Beta 2-syntrophin, an intracellular membrane-associated protein that binds to dystrophin (DMD), and utrophin/ dystrophin related protein | Small molecule-binding protein | | Basement membrane (extracellular matrix) |
| AB006780 | Galectin 3, a lactose-binding lectin, involved in cell growth regulation | Small molecule-binding protein | | Apical plasma membrane |
| AJ000414 | Protein with an SH3 domain and similarity to the non-kinase domains of FER and Fes/Fps tyrosine kinases, binds to activated Cdc42 and may have a role in regulation of the actin cytoskeleton | Small molecule-binding proteinCIP4 is a target for the small GTPase Cdc42 | | |
| Z11793 | Selenoprotein that contains 10 selenocysteine residues, may function in antioxidant activities | Small molecule-binding protein - Undefmed | | |
| U48734 | Alpha-actinin, a non-muscle cell actin-binding protein; localization to nucleus in cancer cells is correlated with good prognosis for breast cancer patients | Structural protein | | Nuclear |
| X68733 | Alpha-1-antichymotrypsin, a member of the serpin family of serine protease inhibitors; deficiency is associated with lung and liver disease | Structural proteinpre-B cell colony enhancement | pre-B cell colony enhancement | |
| U03644 | CBF1-interacting corepressor, links CBF1 and the histone deacetylase complex, binds to histone deacetylase and to SAP30 | Transcription factor | | |

(continued)

| GB_Acc | Detailed Description | Enzyme Class | Biological_Function | subcellular |
|---|---|---|---|---|
| Y09443 | Alkyl-dihydroxyacetonephosphate synthase, functions in ether phospholipid biosynthesis, may be deficient in peroxisomal biogenesis disorders Zellweger syndrome, rhizomelic chondrodysplasia punctata, and adrenoleukodystrophy | Transferase | | Peroxisome |
| M58597 | Myeloid alpha(1,3)fucosyltransferase (GDP-fucose:[Gal beta 1-4]GlcNAc alpha 1-3-fucosyltransferase), makes the 3-fucosyllactosamine epitope (CD15) on polymorphonuclear cells and monocytes, regulates Lex and Ley antigen expression | Transferase | | Unspecified membrane |
| U30930 | UDP-galactose ceramide galactosyltransferase, member of the UDP-glucuronosyltransferase 8 family of endoplasmic reticulum glycoproteins that is involved in synthesizing glycosphingolipids, cerebrosides and sulfatides, myelin membrane constituents | Transferase | | Endoplasmic reticulum |
| Z35102 | Serine/threonine kinase that localizes to the nucleus and is activated via autophosphorylation, expression is ubiquitous | Transferase | | Nuclear |
| L26232 | Phospholipid transfer protein, has roles in phospholipid transport and conversion of high density lipoproteins into larger and smaller particles | Transporter | | |
| U41387 | | | | |

(continued)

| GB_Acc | Detailed Description | Enzyme Class | Biological_Function | subcellular |
|---|---|---|---|---|
| Y08136 | Protein of unknown function, has low similarity to a region of acid sphingomyelinases | | | |
| AB000220 | Semaphorin E, member of a family of proteins involved in neuronal growth cone guidance and immune system regulation, overexpression is associated with resistance to the anticancer drug cis-diamminedichloroplatinum(II), associated with rheumatoid arthritis | | | |
| AL034562 | Glycosylated receptor-like protein with three immunoglobulin-like domains that probably interacts with phosphotyrosine phosphatases, may have a role in response to growth factors and in cell adhesion | | | Plasma membrane |
| U82812 | Spalpha, a member of the scavenger receptor cysteine-rich family that is expressed in lymphoid tissues and may be involved in the regulation of monocyte activation, function, and survival | | | Extracellular (excluding cell wall) |
| AL034562 | Glycosylated receptor-like protein with three immunoglobulin-like domains that probably interacts with phosphotyrosine phosphatases, may have a role in response to growth factors and in cell adhesion | | | Plasma membrane |
| S60099 | | | | |
| H24861 | | | | |
| AF089750 | Protein with very strong similarity to murine Mm.2931 (flotillin), which is an integral membrane protein of caveolae that is expressed in brain | | | Plasma membrane |

(continued)

| GB_Acc | Detailed Description | Enzyme Class | Biological_Function | subcellular |
|---|---|---|---|---|
| AF001383 | Amphiphysin II, a tumor suppressor that interacts with MYC and colocalizes with ankyrin3 (ANK3), may have a role in endocytosis | | | Cytoplasmic |
| U69274 | | | | |
| U49392 | Allograft inflammatory factor 1, cytokine inducible protein associated with vascular injury | | | Nuclear |
| AF035284 | | | | |

**REFERENCES:**

*Patents cited:*

U.S. Pat. No. 4,843,155 Chomczynski, P.
U.S. Pat. No. 5,262,31 Liang, P., and Pardee, A. B., 1993
U.S. Pat. No. 4,683,202 Mullis, K. B., 1987
U.S. Pat. No. 5,593,839
U.S. Pat. No. 5,578,832
U.S. Pat. No. 5,556,752
U.S. Pat. No. 5,631,734
U.S. Pat. No. 5,599,695
U.S. Pat. No. 4,683,195
U.S: Pat. No. 5,498,531
U.S. Pat. No. 5,714,331
U.S. Pat. No. 5,641,673 Haseloff et al.,
U.S. Pat. No. 5,565,332
U.S. Pat. No. 5,223,409 Lander, E. ,
U.S. Pat. No. 5,705,151
U.S. Pat. No. 5,976,813 Beutel et al.
U.S. Pat. No. 5,283,317
PCT No. WO 97/29212
PCT No. WO 97/27317
PCT No. WO 95/22058
PCT No. WO 99/12826
PCT No. WO 97/02357
PCT No. WO 93/03151
PCT No. WO 94/13804
PCT No. WO 94/10300
EP No. 0 785 280
EP No. 0 799 897
EP No. 0 728 520
EP No. 0 721 016
EP No. 0 321 201
GB2188638B


*Publications cited:*

(1) Ross et al., Nature, 362, 801-809 (1993)
(2) Lusis et al.., Nature, 407, 233-241 (2000)
(3) Sambrook et al., MOLECULAR CLONING: A LABORATORY MANUAL, 2d ed., (1989)
(4) Ausubel et al., CURRENT PROTOCOLS IN MOLECULAR BIOLOGY, John Wiley & Sons, New York, N.Y., (1989).
(5) Tedder, T. F. et al., Proc. Natl. Acad. Sci. U.S.A. 85:208-212, (1988)
(6) Hedrick, S. M. et al., Nature 308:149-153, (1984)
(7) Lee, S. W. et al., Proc. Natl. Acad. Sci. U.S.A. 88:4225, (1984)
(8) Sarkar, PCR Methods Applic. 2, 318-322, (1993)
(9) Triglia et al., Nucleic Acids Res. 16, 81-86, (1988)
(10) Lagerstrom et al., PCR Methods Applic. 1, 111-119, (1991)
(11) Parker et al., Nucleic Acids Res. 19, 3055-3060, (1991)
(12) Copeland & Jenkins, Trends in Genetics 7: 113-118, (1991)
(13) Cohen, et al., Nature 366: 698-701, (1993)
(14) Bonner et al., J. Mol. Biol. 81, 123 (1973)
(15) Bolton and McCarthy, Proc. Natl. Acad. Sci. U.S.A. 48, 1390 (1962)
(16) Plump et al., Cell 71: 343-353, (1992)

(17) Van Heeke & Schuster, J. Biol. Chem. 264, 5503-5509, (1989)
(18) Grant et al., Methods Enzymol. 153, 516-544, (1987)
(19) Takamatsu, EMBO J. 6, 307-311, (1987)
(20) Coruzzi et al., EMBO J. 3, 1671-1680, (1984)
(21) Broglie et al., Science 224, 838-843, (1984)
(22) Winter et al., Results Probl. Cell Differ. 17, 85-105, (1991)
(23) MCGRAW HILL YEARBOOK OF SCIENCE AND TECHNOLOGY, McGraw Hill, New York, N.Y., pp. 191-196, (1992)
(24) Engelhard et al., Proc. Nat. Acad. Sci. 91, 3224-3227, (1994)
(25) Logan & Shenk, Proc. Natl. Acad. Sci. 81, 3655-3659, (1984)
(26) Scharf et al., Results Probl. Cell Differ. 20, 125-162, (1994)
(27) Freshney R.I., ed., ANIMAL CELL CULTURE, (1986)
(28) Wigler et al., Cell 11, 223-232, (1977)
(29) Lowy et al., Cell 22, 817-823, (1980)
(30) Wigler et al., Proc. Natl. Acad. Sci. 77, 3567-3570, (1980)
(31) Colbere-Garapin et al., J. Mol. Biol. 150, 114, (1981)
(32) Hartman & Mulligan, Proc. Natl. Acad. Sci. 85, 8047-8051, (1988)
(33) Rhodes et al., Methods Mol. Biol. 55, 121-131, (1995)
(34) Hampton et al., SEROLOGICAL METHODS: A LABORATORY MANUAL, APS Press, St. Paul, Minn., (1990)
(35) Maddox et al., J. Exp. Med. 158, 1211-1216, (1983)
(36) Porath et al., Prot. Exp. Purif. 3, 263-281, (1992)
(37) Kroll et al., DNA Cell Biol. 12, 441-453, (1993)
(38) Caruthers et al., Nucl. Acids Res. Symp. Ser. 215-223, (1980)
(39) Horn et al. Nucl. Acids Res. Symp. Ser. 225-232, (1980)
(40) Merrifield, J. Am. Chem. Soc. 85,2149-2154, (1963)
(41) Roberge et al., Science 269, 202-204, (1995)
(42) Creighton, PROTEINS: STRUCTURES AND MOLECULAR PRINCIPLES, WH and Co., New York, N.Y., (1983)
(43) Cronin et al., Human Mutation 7:244, (1996)
(44) Landegran et al., Science 241:1077-1080, (1988)
(45) Nakazawa et al., PNAS 91:360-364, (1994)
(46) Abravaya et al., Nuc Acid Res 23:675-682, (1995)
(47) Guatelli, J.C. et al., Proc. Natl. Acad. Sci. USA 87:1874-1878, (1990)
(48) Kwoh, D.Y. et al., Proc. Natl. Acad. Sci. USA 86:1173 -1177, (1989)
(49) Lizardi, P.M. et al., Bio/Technology 6:1197, (1988)
(50) Brown, Meth. Mol. Biol. 20, 18, (1994)
(51) Sonveaux, Meth. Mol. Biol. 26, 1-72, (1994)
(52) Uhlmann et al., Chem. Rev. 90, 543-583, (1990)
(53) Gee et al., in Huber & Carr, MOLECULAR AND IMMUNOLOGIC APPROACHES, Publishing Co., Mt. Kisco, N.Y., (1994)
(54) Agrawal et al., Trends Biotechnol. 10, 152-158, (1992)
(55) Uhlmann et al., Tetrahedron. Lett. 215, 3539-3542, (1987)
(56) Cech, Science 236, 1532-1539, (1987)
(57) Cech, Ann. Rev. Biochem. 59, 543-568, (1990)
(58) Couture & Stinchcomb, Trends Genet. 12, 510-515, (1996)
(59) Haseloff et al. Nature 334, 585-591, (1988)
(60) Kohler et al., Nature 256, 495-497, (1985)
(61) Kozbor et al., J. Immunol. Methods 81, 3142, (1985)
(62) Cote et al., Proc. Natl. Acad. Sci. 80, 2026-2030, (1983)
(63) Cole et al., Mol. Cell Biol. 62, 109-120, (1984)
(64) Morrison et al., Proc. Natl. Acad. Sci. 81, 6851-6855, (1984)
(65) Neuberger et al., Nature 312, 604-608, (1984)
(66) Takeda et al., Nature 314, 452-454, (1985)
(67) Burton, Proc. Natl. Acad. Sci. 88, 11120-11123, (1991)
(68) Thirion et al., Eur. J. Cancer Prev. 5, 507-11, (1996)
(69) Coloma & Morrison, Nat. Biotechnol. 15, 159-63, (1997)
(70) Mallender & Voss, J. Biol. Chem. 269, 199-206, (1994)
(71) Verhaar et al., Int. J. Cancer 61, 497-501, (1995)
(72) Nicholls et al., J. Immunol. Meth. 165, 81-91, (1993)

(73) Orlandi et al., Proc. Natl. Acad. Sci. 86, 3833-3837, (1989)
(74) Winter et al., Nature 349, 293-299, (1991)
(75) Lam, Anticancer Drug Des. 12, 145, (1997)
(76) DeWitt et al., Proc. Natl. Acad. Sci. U.S.A. 90, 6909, (1993)
(77) Erb et al. Proc. Natl. Acad. Sci. U.S.A. 91, 11422, (1994)
(78) Zuckermann et al., J. Med. Chem. 37, 2678, (1994)
(79) Cho et al., Science 261, 1303, (1993)
(80) Carell et al., Angew. Chem. Int. Ed. Engl. 33, 2059 & 2061, (1994)
(81) Gallop et al., J. Med. Chem. 37, 1233, (1994)
(82) Houghten, BioTechniques 13, 412-421, (1992)
(83) Lam, Nature 354, 8284, (1991)
(84) Fodor, Nature 364, 555-556, (1993)
(85) Cull et al., Proc. Natl. Acad. Sci. U.S.A. 89, 1865-1869, (1992)
(86) Scott & Smith, Science 249, 386-390, (1990)
(87) Devlin, Science 249, 404-406, (1990)
(88) Cwirla et al., Proc. Natl. Acad. Sci. 97, 6378-6382, (1990)
(89) Felici, J. Mol. Biol. 222, 301-310, (1991)
(90) Jayawickreme et al., Proc. Natl. Acad. Sci. U.S.A. 19, 1614-1618, (1994)
(91) Chelsky, Strategies for Screening Combinatorial Libraries (1995)
(92) Salmon et al., Molecular Diversity 2, 57-63, (1996)
(93) McConnell et al., Science 257, 1906-1912, (1992)
(94) Sjolander & Urbaniczky, Anal. Chem. 63, 2338-2345, (1991)
(95) Szabo et al., Curr. Opin. Struct. Biol. 5, 699-705, (1995)
(96) Zervos et al., Cell 72, 223-232, (1993)
(97) Madura et al., J. Biol. Chem. 268, 12046-12054, (1993)
(98) Bartel et al., BioTechniques 14, 920-924, (1993)
(99) Iwabuchi et al., Oncogene 8, 1693-1696, (1993)
(100) Findeis et al. Trends in Biotechnol. 11, 202-205, (1993)
(101) Chiou et al., GENE THERAPEUTICS: METHODS AND APPLICATIONS OF DIRECT GENE TRANSFER (J.A. Wolff, ed.), (1994)
(102) Wu & Wu, J. Biol. Chem. 263, 621-24, (1988)
(103) Wu et al., J. Biol. Chem. 269, 542-46, (1994)
(104) Zenke et al., Proc. Natl. Acad. Sci. U.S.A. 87, 3655-59, (1990)
(105) Wu et al., J. Biol. Chem. 266, 338-42, (1991)
(106) REMINGTON'S PHARMACEUTICAL SCIENCES (Maack Publishing Co., Easton, Pa.
(107) Muller et al., Arterioscler Thromb 13:1317-1326 (1993)
(108) Needleman SB, Wunsch, J Mol Biol. 48,443-53,1970
(109) Henikoff S, Henikoff JG, Proc. Natl. Acad. Sci. USA 89,10915-10919,1992
(110) Hille, Excitable Membranes, Sunderland, MA, Sinauer Associates, Inc.

## Claims

1. A method for the prediction, diagnosis or prognosis of a cardiovascular disease by the detection of:

   a) a polynucleotide of the sequences of SEQ ID NO. 1;
   b) a polynucleotide which hybridises under stringent conditions to a polynucleotide specified in (a) and encodes a polypeptide exhibiting the same biological function as specified for the respective sequence in SEQ ID NO. 75;
   c) a polynucleotide the sequence of which deviates from the polynucleotide specified in (a) and (b) due to the degeneration of the genetic code and encodes a polypeptide exhibiting the same biological function as specified for the respective sequence in SEQ ID NO. 75;
   d) a polynucleotide which represents a specific fragment, derivative or allelic variation of a polynucleotide sequence specified in (a) to (c) and encodes a polypeptide exhibiting the same biological function as specified for the respective sequence in SEQ ID NO. 75;

   in a biological sample comprising the following steps:

   hybridising at least one polynucleotide specified in (a) to (d) to a nucleic acid material of a biological sample, thereby forming a hybridization complex; and detecting said hybridization complex, wherein a gene comprising a polynucleotide sequence specified in (a) to (d) is down regulated in cardio-vascular disease.

2. The method of claim 1, wherein before hybridization, the nucleic acid material of the biological sample is amplified.

**Patentansprüche**

1. Verfahren zur Vorhersage, Diagnose oder Prognose einer Herzgefäßkrankheit durch die Detektion:

   a) eines Polynukleotids der Sequenzen von SEQ ID NO. 1;
   b) eines Polynukleotids, welches unter strengen Bedingungen an ein Polynukleotid hybridisiert, das in (a) spezifiziert ist, und ein Polypeptid codiert, das dieselbe biologische Funktion aufweist, wie für die jeweilige Sequenz in SEQ ID NO. 75 spezifiziert;
   c) eines Polynukleotids, dessen Sequenz von dem in (a) und (b) spezifizierten Polynukleotid aufgrund der Degeneration des genetischen Codes abweicht und ein Polypeptid codiert, das dieselbe biologische Funktion aufweist, wie für die jeweilige Sequenz in SEQ ID NO. 75 spezifiziert;
   d) eines Polynukleotids, welches ein spezifisches Fragment, ein Derivat oder eine allelische Variante einer Polynukleotidsequenz darstellt, die in (a) bis (c) spezifiziert ist und ein Polypeptid codiert, das dieselbe biologische Funktion aufweist, wie für die jeweilige Sequenz in SEQ ID NO. 75 spezifiziert;

   in einer biologischen Probe, welches die folgenden Schritte umfasst:

   Hybridisieren wenigstens eines Polynukleotids, das in (a) bis (d) spezifiziert ist, an ein Nukleinsäurematerial einer biologischen Probe, wodurch ein Hybridisierungskomplex gebildet wird; und
   Detektieren des besagten Hybridisierungskomplexes, wobei ein Gen, das eine Polynukleotidsequenz umfasst, die in (a) bis (d) spezifiziert ist, bei Herzgefäßkrankheit downreguliert wird.

2. Verfahren nach Anspruch 1, wobei vor der Hybridisierung das Nukleinsäurematerial der biologischen Probe amplifiziert wird.

**Revendications**

1. Procédé de prédiction de diagnostic ou de pronostic d'une maladie cardiovasculaire par la détection de :

   a) un polynucléotide ayant les séquences de l'identification de séquences numéro 1 ;
   b) un polynucléotide qui s'hybride dans des conditions stringentes en un polynucléotide spécifié en (a) et code un polypeptide ayant la même fonction biologique que spécifiée pour la séquence respective dans l'identification de séquence numéro 75 ;
   c) un polynucléotide dont la séquence s'écarte du polynucléotide spécifié en (a) et (b) en raison de la dégénération du code génétique et code un polypeptide ayant la même fonction biologique que spécifiée pour la séquence respective dans l'identification de séquence numéro 75 ;
   d) un polynucléotide qui représente un fragment spécifique, un dérivé ou une variation allélique d'une séquence de polynucléotide spécifiée en (a) à (c) et code un polypeptide présentant la même fonction biologique que spécifiée pour la séquence respective dans l'identification de séquence numéro 75 ;

   dans un échantillon biologique, comprenant les stades suivantes :

   on hybride au moins un polynucléotide spécifié en (a) à(d) à une matière d'acide nucléique d'un échantillon biologique en formant ainsi un complexe d'hybridation et
   on détecte le complexe d'hybridation, dans lequel un gène comprenant une séquence de polynucléotide spécifiée dans (a) à (d) est régulée négativement dans une maladie cardiovasculaire.

2. Procédé suivant la revendication 1, dans lequel avant hybridation on amplifie la matière d'acide nucléique de l'échantillon biologique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0172774 A
- US PS6087117 A
- US 4843155 A, Chomczynski, P.
- US 5262311 A, Liang, P., and Pardee, A. B.
- US 4683202 A, Mullis, K. B.
- EP 0799897 A
- WO 9729212 A
- WO 9727317 A
- EP 0785280 A
- WO 9702357 A
- US 5593839 A
- US 5578832 A
- EP 0728520 A
- US 5599695 A
- EP 0721016 A
- US 5556752 A
- WO 9522058 A
- US 5631734 A
- US 4683195 A
- US 5498531 A
- US 6203987 B
- US 5714331 A
- WO 9912826 A
- US 5641673 A, Haseloff
- EP 0321201 A, Gerlach
- GB 2188638 B
- US 5565332 A
- WO 9303151 A
- WO 9413804 A
- US 5223409 A, Ladner
- US 5976813 A, Beutel
- US 5283317 A
- WO 9410300 A, Brent
- US 5705151 A
- US 526231 A, Liang, P., and Pardee, A. B.
- US 1993 A
- US 1987 A
- GB 0124145 A


### Non-patent literature cited in the description


- **BARRANS et al.** Construction of a human cardiovascular cDNA microarray: portrait of the failing heart. *BIOCHEMICAL AND BIOPHYSICAL RESEARCH COMMUNICATIONS,* January 2001, vol. 280, 964-969
- **MCCAFFREY et al.** High-level of expression of Egr-1 and Egr-1-inducible genes in mouse and human atherosclerosis. *THE JOURNAL OF CLINICAL INVESTIGATION,* March 2000, vol. 105 (5), 653-662
- **LAWN et al.** The Tangier disease gene product ABC1 controls the cellular apolipoprotein-mediated lipid removal pathway. *THE JOURNAL OF CLINICAL INVESTIGATION,* October 1999, vol. 104 (8), R25R-R31
- **Ross et al.** *Nature,* 1993, vol. 362, 801-809
- **Lusis et al.** *Nature,* 2000, vol. 407, 233-241
- **Sambrook et al.** MOLECULAR CLONING: A LABORATORY MANUAL. 1989
- **Ausubel et al.** CURRENT PROTOCOLS IN MOLECULAR BIOLOGY. John Wiley & Sons, 1989
- **Tedder, T. F. et al.** *Proc. Natl. Acad. Sci. U.S.A.,* 1988, vol. 85, 208-212
- **Hedrick, S. M. et al.** *Nature,* 1984, vol. 308, 149-153
- **Lee, S. W. et al.** *Proc. Natl. Acad. Sci. U.S.A.,* 1984, vol. 88, 4225
- **Sarkar.** *PCR Methods Applic.,* 1993, vol. 2, 318-322
- **Triglia et al.** *Nucleic Acids Res.,* 1988, vol. 16, 81-86
- **Lagerstrom et al.** *PCR Methods Applic.,* 1991, vol. 1, 111-119
- **Parker et al.** *Nucleic Acids Res.,* 1991, vol. 19, 3055-3060
- **Copeland ; Jenkins.** *Trends in Genetics,* 1991, vol. 7, 113-118
- **Cohen et al.** *Nature,* 1993, vol. 366, 698-701
- **Bonner et al.** *J. Mol. Biol.,* 1973, vol. 81, 123
- **Bolton ; McCarthy.** *Proc. Natl. Acad. Sci. U.S.A.,* 1962, vol. 48, 1390
- **Plump et al.** *Cell,* 1992, vol. 71, 343-353
- **Van Heeke ; Schuster.** *J. Biol. Chem.,* 1989, vol. 264, 5503-5509
- **Grant et al.** *Methods Enzymol.,* 1987, vol. 153, 516-544
- **Takamatsu.** *EMBO J.,* 1987, vol. 6, 307-311
- **Coruzzi et al.** *EMBO J.,* 1984, vol. 3, 1671-1680
- **Broglie et al.** *Science,* 1984, vol. 224, 838-843

- **Winter et al.** *Results Probl. Cell Differ.,* 1991, vol. 17, 85-105
- MCGRAW HILL YEARBOOK OF SCIENCE AND TECHNOLOGY. McGraw Hill, 1992, 191-196
- **Engelhard et al.** *Proc. Nat. Acad. Sci.,* 1994, vol. 91, 3224-3227
- **Logan ; Shenk.** *Proc. Natl. Acad. Sci.,* 1984, vol. 81, 3655-3659
- **Scharf et al.** *Results Probl. Cell Differ.,* 1994, vol. 20, 125-162
- ANIMAL CELL CULTURE. 1986
- **Wigler et al.** *Cell,* 1977, vol. 11, 223-232
- **Lowy et al.** *Cell,* 1980, vol. 22, 817-823
- **Wigler et al.** *Proc. Natl. Acad. Sci.,* 1980, vol. 77, 3567-3570
- **Colbere-Garapin et al.** *J. Mol. Biol.,* 1981, vol. 150, 114
- **Hartman ; Mulligan.** *Proc. Natl. Acad. Sci.,* 1988, vol. 85, 8047-8051
- **Rhodes et al.** *Methods Mol. Biol.,* 1995, vol. 55, 121-131
- **Hampton et al.** SEROLOGICAL METHODS: A LABORATORY MANUAL. APS Press, 1990
- **Maddox et al.** *J. Exp. Med.,* 1983, vol. 158, 1211-1216
- **Porath et al.** *Prot. Exp. Purif.,* 1992, vol. 3, 263-281
- **Kroll et al.** *DNA Cell Biol.,* 1993, vol. 12, 441-453
- **Caruthers et al.** *Nucl. Acids Res. Symp. Ser.,* 1980, 215-223
- **Horn et al.** *Nucl. Acids Res. Symp. Ser.,* 1980, 225-232
- **Merrifield.** *J. Am. Chem. Soc.,* 1963, vol. 85, 2149-2154
- **Roberge et al.** *Science,* 1995, vol. 269, 202-204
- **Creighton.** PROTEINS: STRUCTURES AND MOLECULAR PRINCIPLES. WH and Co, 1983
- **Cronin et al.** *Human Mutation,* 1996, vol. 7, 244
- **Landegran et al.** *Science,* 1988, vol. 241, 1077-1080
- **Nakazawa et al.** *PNAS,* 1994, vol. 91, 360-364
- **Abravaya et al.** *Nuc Acid Res,* 1995, vol. 23, 675-682
- **Guatelli, J.C. et al.** *Proc. Natl. Acad. Sci. USA,* 1990, vol. 87, 1874-1878
- **Kwoh, D.Y. et al.** *Proc. Natl. Acad. Sci. USA,* 1989, vol. 86, 1173-1177
- **Lizardi, P.M. et al.** *Bio/Technology,* 1988, vol. 6, 1197
- **Brown.** *Meth. Mol. Biol.,* 1994, vol. 20, 18
- **Sonveaux.** *Meth. Mol. Biol.,* 1994, vol. 26, 1-72
- **Uhlmann et al.** *Chem. Rev.,* 1990, vol. 90, 543-583
- **Gee et al.** MOLECULAR AND IMMUNOLOGIC APPROACHES. Publishing Co, 1994
- **Agrawal et al.** *Trends Biotechnol.,* 1992, vol. 10, 152-158
- **Uhlmann et al.** *Tetrahedron. Lett.,* 1987, vol. 215, 3539-3542
- **Cech.** *Science,* 1987, vol. 236, 1532-1539
- **Cech.** *Ann. Rev. Biochem.,* 1990, vol. 59, 543-568
- **Couture ; Stinchcomb.** *Trends Genet.,* 1996, vol. 12, 510-515
- **Haseloff et al.** *Nature,* 1988, vol. 334, 585-591
- **Kohler et al.** *Nature,* 1985, vol. 256, 495-497
- **Kozbor et al.** *J. Immunol. Methods,* 1985, vol. 81, 3142
- **Cote et al.** *Proc. Natl. Acad. Sci.,* 1983, vol. 80, 2026-2030
- **Cole et al.** *Mol. Cell Biol.,* 1984, vol. 62, 109-120
- **Morrison et al.** *Proc. Natl. Acad. Sci.,* 1984, vol. 81, 6851-6855
- **Neuberger et al.** *Nature,* 1984, vol. 312, 604-608
- **Takeda et al.** *Nature,* 1985, vol. 314, 452-454
- **Burton.** *Proc. Natl. Acad. Sci.,* 1991, vol. 88, 11120-11123
- **Thirion et al.** *Eur. J. Cancer Prev.,* 1996, vol. 5, 507-11
- **Coloma ; Morrison.** *Nat. Biotechnol.,* 1997, vol. 15, 159-63
- **Mallender ; Voss.** *J. Biol. Chem.,* 1994, vol. 269, 199-206
- **Verhaar et al.** *Int. J. Cancer,* 1995, vol. 61, 497-501
- **Nicholls et al.** *J. Immunol. Meth.,* 1993, vol. 165, 81-91
- **Orlandi et al.** *Proc. Natl. Acad. Sci.,* 1989, vol. 86, 3833-3837
- **Winter et al.** *Nature,* 1991, vol. 349, 293-299
- **Lam.** *Anticancer Drug Des.,* 1997, vol. 12, 145
- **DeWitt et al.** *Proc. Natl. Acad. Sci. U.S.A.,* 1993, vol. 90, 6909
- **Erb et al.** *Proc. Natl. Acad. Sci. U.S.A.,* 1994, vol. 91, 11422
- **Zuckermann et al.** *J. Med. Chem.,* 1994, vol. 37, 2678
- **Cho et al.** *Science,* 1993, vol. 261, 1303
- **Carell et al.** *Angew. Chem. Int. Ed. Engl.,* 1994, vol. 33, 2059, 2061
- **Gallop et al.** *J. Med. Chem.,* 1994, vol. 37, 1233
- **Houghten.** *BioTechniques,* 1992, vol. 13, 412-421
- **Lam.** *Nature,* 1991, vol. 354, 8284
- **Fodor.** *Nature,* 1993, vol. 364, 555-556
- **Cull et al.** *Proc. Natl. Acad. Sci. U.S.A.,* 1992, vol. 89, 1865-1869

- **Scott ; Smith.** *Science,* 1990, vol. 249, 386-390
- **Devlin.** *Science,* 1990, vol. 249, 404-406
- **Cwirla et al.** *Proc. Natl. Acad. Sci.,* vol. 97, 6378-6382
- **Felici.** *J. Mol. Biol.,* 1991, vol. 222, 301-310
- **Jayawickreme et al.** *Proc. Natl. Acad. Sci. U.S.A.,* 1994, vol. 19, 1614-1618
- **Chelsky.** Strategies for Screening Combinatorial Libraries. 1995
- **Salmon et al.** *Molecular Diversity,* 1996, vol. 2, 57-63
- **McConnell et al.** *Science,* 1992, vol. 257, 1906-1912
- **Sjolander ; Urbaniczky.** *Anal. Chem.,* 1991, vol. 63, 2338-2345
- **Szabo et al.** *Curr. Opin. Struct. Biol.,* 1995, vol. 5, 699-705
- **Zervos et al.** *Cell,* 1993, vol. 72, 223-232
- **Madura et al.** *J. Biol. Chem.,* 1993, vol. 268, 12046-12054
- **Bartel et al.** *BioTechniques,* 1993, vol. 14, 920-924
- **Iwabuchi et al.** *Oncogene,* 1993, vol. 8, 1693-1696
- **Findeis et al.** *Trends in Biotechnol.,* 1993, vol. 11, 202-205
- **Chiou et al.** GENE THERAPEUTICS: METHODS AND APPLICATIONS OF DIRECT GENE TRANSFER. 1994
- **Wu ; Wu.** *J. Biol. Chem.,* 1988, vol. 263, 621-24
- **Wu et al.** *J. Biol. Chem.,* 1994, vol. 269, 542-46
- **Zenke et al.** *Proc. Natl. Acad. Sci. U.S.A.,* 1990, vol. 87, 3655-59
- **Wu et al.** *J. Biol. Chem.,* 1991, vol. 266, 338-42
- REMINGTON'S PHARMACEUTICAL SCIENCES. Maack Publishing Co,
- **Muller et al.** *Arterioscler Thromb,* 1993, vol. 13, 1317-1326
- **Needleman SB ; Wunsch.** *J Mol Biol.,* 1970, vol. 48, 443-53
- **Henikoff S ; Henikoff JG.** *Proc. Natl. Acad. Sci. USA,* 1992, vol. 89, 10915-10919
- **Hille.** Excitable Membranes. Sinauer Associates, Inc,